# EUROPEAN PATENT APPLICATION

(11) **EP 3 257 442 A2**
(43) Date of publication of application: **20.12.2017**
(21) Application number: 17180578.1
(22) Date of filing: 29.10.2010
(51) Int. Cl.: A61B 5/151

(54) **RELATIVELY SMALL DEVICES APPLIED TO THE SKIN, MODULAR SYSTEMS, AND METHODS OF USE THEREOF**

(30) Priority: 30.10.2009 US 256910 P; 30.10.2009 US 256874 P; 30.10.2009 US 256931 P; 30.10.2009 US 256863 P; 30.10.2009 US 256871 P; 30.10.2009 US 256880 P; 30.10.2009 US 256933 P; 13.01.2010 US 294543 P; 13.05.2010 US 334529 P; 13.05.2010 US 334533 P; 23.06.2010 US 357582 P; 26.07.2010 US 367607 P; 13.08.2010 US 373764 P
(62) Divisional of application: 10776881.4
(71) Applicant: Seventh Sense Biosystems, Inc., Medford, MA 02155 (US)
(72) Inventor: Bernstein, Howard, Cambridge, Massachusetts 02138 (US); Chickering, Donald E., III, Framingham, Massachusetts 01701 (US); Levinson, Douglas A., Sherborn, Massachusetts 01770 (US); Walt, David R., Boston, Massachusetts 02116 (US); Davis, Shawn, Santa Monica, California 90404 (US); Haghgooie, Ramin, Arlington, Massachusetts 02474 (US)
(74) Representative: Potter Clarkson LLP

(57) **Abstract**

The present invention generally relates, in certain aspects, to relatively small devices applied to the skin, modular systems, and methods of use thereof. In some aspects, the device is constructed and arranged to have more than one module. For instance, the device may have a module for delivering to and/or withdrawing fluid from the skin and/or beneath the skin of a subject and a module for transmitting a signal indicative of the fluid delivered to and/or withdrawn from the skin and/or beneath the skin of the subject, a module for analyzing a fluid withdrawn from the skin and/or beneath the skin of the subject, or the like. In some embodiments, the modules are connectable and/or detachable from each other, and in some cases, the connections and/or detachments may be performed while the device is in contact with the subject, e.g., while affixed to the subject. In some embodiments, the device may be repeatedly applicable to the skin of the subject to deliver to and/or withdraw fluid from the skin and/or beneath the skin of a subject, e.g., at the same location, or at different locations on the skin of the subject. In some aspects, the devices may be self-contained and/or have a relatively small size, and in some cases, the device may be sized such that it is wearable and/or able to be carried by a subject. For example, the device may have a mass and/or dimensions that allow the device to be carried or worn by a subject for various periods of time, e.g., at least about an hour, at least about a day, at least about a week, etc., or no more than about an hour, no more than about 10 min, etc.

## Description

### RELATED APPLICATIONS

This application claims the benefit of U.S. Provisional Patent Application Serial No. 61/256,880, filed October 30, 2009, entitled "Systems and Methods for Altering or Masking Perception of Treatment of a Subject," by Chickering, *et al.;* U.S. Provisional Patent Application Serial No. 61/256,874, filed October 30, 2009, entitled "Systems and Methods for Application to Skin and Control of Use Thereof," by Bernstein, *et al.;* U.S. Provisional Patent Application Serial No. 61/256,871, filed October 30, 2009, entitled "Packaging Systems and Methods for Devices Applied to the Skin," by Bernstein, *et al.;* U.S. Provisional Patent Application Serial No. 61/256,863, filed October 30, 2009, entitled "Systems and Methods for Treating or Shielding Blood on the Surface of the Skin," by Bernstein, *et al.;* U.S. Provisional Patent Application Serial No. 61/256,910, filed October 30, 2009, entitled "Systems and Methods for Sanitizing or Treating the Skin or Devices Applied to the Skin," by Bernstein, *et al.;* U.S. Provisional Patent Application Serial No. 61/256,931, filed October 30, 2009, entitled "Modular Systems for Application to the Skin," by Bernstein, *et al.;* U.S. Provisional Patent Application Serial No. 61/256,933, filed October 30, 2009, entitled "Relatively Small Devices Applied to the Skin and Methods of Use Thereof," by Cluckering, *et al.;* U.S. Provisional Patent Application Serial No. 61/294,543, filed January 13, 2010, entitled "Blood Sampling Device and Method," by Chickering, *et al.;* U.S. Provisional Patent Application Serial No. 61/334,533, filed May 13, 2010, entitled "Rapid Delivery and/or Withdrawal of Fluids," by Chickering, *et al.;* U.S. Provisional Patent Application Serial No. 61/334,529, filed May 13, 2010, entitled "Sampling Device Interfaces," by Chickering, *et al.;* U.S. Provisional Patent Application Serial No. 61/357,582, filed June 23, 2010, entitled "Sampling Devices and Methods Involving Relatively Little Pain," by Chickering, *et al.;* U.S. Provisional Patent Application Serial No. 61/367,607, filed July 26, 2010, entitled "Rapid Delivery and/or Withdrawal of Fluids," by Davis, *et al.;* and U.S. Provisional Patent Application Serial No. 61/373,764, filed August 13, 2010, entitled "Clinical and/or Consumer Techniques and Devices," by Chickering, *et al.* Each of the above is incorporated herein by reference.

### FIELD OF INVENTION

The present invention generally relates to systems and methods for delivering to and/or withdrawing fluid from subjects, e.g., to or from the skin and/or beneath the skin.

### BACKGROUND

A variety of techniques and methods exist for sensing and responding to conditions to which a subject is exposed, including sensing of physiological conditions of a mammal and/or a surrounding environment. Other techniques exist for withdrawing a fluid, such as blood, from a mammal. While many such techniques are suitable for various purposes, techniques that have one or more features such as added simplicity and flexibility of use would be advantageous.

### SUMMARY OF THE INVENTION

The present invention generally relates to systems and methods for delivering to and/or withdrawing fluid from subjects, e.g., to or from the skin and/or beneath the skin. The subject matter of the present invention involves, in some cases, interrelated products, alternative solutions to a particular problem, and/or a plurality of different uses of one or more systems and/or articles.

In one aspect, the present invention is generally directed to a device for analysis of an extractable medium of a subject, e.g., from the skin and/or beneath the skin of the subject. In one set of embodiments, the device includes a support structure for application to the skin of the subject, the support structure comprising means for extracting a medium of the subject from or through the subject's skin, and a signal structure for generating a signal indicative of the determination of at least one analyte of the medium. In some cases, the support structure and the signal structure are constructed and arranged to be connectable and/or detachable from each other readily by the subject.

According to another set of embodiments, the device includes a support structure for application to the skin of the subject, and means for extracting a medium of the subject from or through the subject's skin at an extraction area. In some cases, the device comprises at least one of a largest lateral dimension, parallel to the extraction area, of no more than about 5 cm, a largest vertical dimension, extending from the skin of the subject when the device is positioned for extraction of the medium, of no more than about 1 cm, or a mass of no more than about 25 g.

In yet another set of embodiments, the device includes a support structure for application to the skin of the subject, means for extracting a fluid from the skin and/or from beneath the skin of the subject, and a signal structure for generating a signal indicative of the determination of at least one analyte contained within the fluid. In some embodiments, the support structure and the signal structure are constructed and arranged to be readily connectable and/or detachable from each other.

In another aspect, the present invention is generally directed to a device for analysis of an extractable fluid from the skin and/or from beneath the skin of a subject. In one set of embodiments, the device includes a support structure for application to the skin of the subject, a fluid transporter associated with the support structure, and a signal structure for generating a signal indicative of the determination of at least one analyte contained within the fluid. In some embodiments, the support structure and the signal structure are constructed and arranged to be readily connectable and/or detachable from each other.

According to another set of embodiments, the device includes a support structure for application to the skin of the subject, and a fluid transporter associated with the support structure. In some cases, the device comprises at least one of: a largest lateral dimension, when the device is positioned for extraction of the fluid, parallel to the extraction area, of no more than about 5 cm, a largest vertical dimension, extending from the skin of the subject when the device is positioned for extraction of the fluid, of no more than about 1 cm, or a mass of no more than about 25 g, absent the fluid.

In still another set of embodiments, the device includes a support structure for application to the skin of the subject, a fluid transporter associated with the support structure, a sensor for determining at least one condition of a fluid extracted from the skin and/or from beneath the skin of the subject, and a signal and/or display generator for generating a signal and/or display relating to the at least one condition of the fluid determined by the sensor. In some embodiments, the device comprises at least one of a largest lateral dimension, when the device is positioned for extraction of the fluid, parallel to the extraction area, of no more than about 7 cm, a largest vertical dimension, extending from the skin of the subject when the device is positioned for extraction of the fluid, of no more than about 3 cm, or a mass of no more than about 150 g, absent the fluid.

The device, in yet another set of embodiments, includes a support structure for application to the skin of the subject, and a fluid transporter associated with the support structure. The device, according to some embodiments, may be constructed and arranged to be wearable by the subject for a period of at least 15 minutes.

The device, in another set of embodiments, includes a support structure for application to the skin of the subject, and a fluid transporter associated with the support structure. In some embodiments, the device comprises at least one of: a largest lateral dimension, when the device is positioned for extraction of the fluid, parallel to the extraction area, of no more than about 6 cm, a largest vertical dimension, extending from the skin of the subject when the device is positioned for extraction of the fluid, of no more than about 1.5 cm, or a mass of no more than about 25 g, absent the fluid.

The device, in still another set of embodiments, includes a support structure for application to the skin of the subject, a fluid transporter associated with the support structure, and an adhesive for adhesion of the support structure to the skin of the subject.

In yet another set of embodiments, the device includes a support structure for application to the skin of the subject, and means for extracting a fluid from the skin and/or from beneath the skin of the subject at an extraction area. In some embodiments, the device comprises at least one of: a largest lateral dimension, when the device is positioned for extraction of the fluid, parallel to the extraction area, of no more than about 5 cm, a largest vertical dimension, extending from the skin of the subject when the device is positioned for extraction of the fluid, of no more than about 1 cm, or a mass of no more than about 25 g, absent the fluid.

In still another set of embodiments, the device includes a support structure for application to the skin of the subject, means for extracting a fluid from the skin and/or from beneath the skin of the subject at an extraction area, a sensor for determining at least one condition of a fluid extracted from the skin of the subject, and a signal and/or display generator for generating a signal and/or display relating to the at least one condition of the fluid determined by the sensor. In some embodiments, the device comprises at least one of: a largest lateral dimension, when the device is positioned for extraction of the fluid, parallel to the extraction area, of no more than about 7 cm, a largest vertical dimension, extending from the skin of the subject when the device is positioned for extraction of the fluid, of no more than about 3 cm, or a mass of no more than about 150 g, absent the fluid.

The device, according to yet another set of embodiments, includes a support structure for application to the skin of the subject, and means for extracting a fluid from the skin and/or from beneath the skin of the subject at an extraction area. According to some embodiments, the device is constructed and arranged to be wearable by the subject for a period of at least 15 minutes.

The device, in yet another set of embodiments, includes a support structure for application to the skin of the subject, and means for extracting a fluid from the skin and/or from beneath the skin of the subject at an extraction area. In some embodiments, the device comprises at least one of: a largest lateral dimension, when the device is positioned for extraction of the fluid, parallel to the extraction area, of no more than about 6 cm, a largest vertical dimension, extending from the skin of the subject when the device is positioned for extraction of the fluid, of no more than about 1.5 cm, or a mass of no more than about 25 g, absent the fluid.

The device, in still another set of embodiments, includes a support structure for application to the skin of the subject, means for extracting a fluid from the skin and/or from beneath the skin of the subject at an extraction area, and an adhesive for adhesion of the support structure to the skin of the subject.

The present invention, in yet another aspect, is directed to a device for delivering to and/or withdrawing fluid from the skin and/or beneath the skin of a subject. The fluid may be, for example, blood or interstitial fluid, a fluid such as saline carrying a drug or other therapeutic agent, or other suitable fluids such as those described herein.

The device, in one set of embodiments, includes a support structure for application to the skin of the subject, the support structure comprising transport means for delivery to and/or withdrawal of fluid from the skin and/or beneath the skin of the subject, and a signal structure for generating a signal indicative of fluid delivered to and/or withdrawn from the skin of the subject. In some cases, the support structure and the signal structure are constructed and arranged to be connectable and/or detachable from each other.

In yet another set of embodiments, the device includes a support structure for application to the skin of the subject, the support structure comprising transport means for delivery to and/or withdrawal of fluid from the skin and/or beneath the skin of a subject, and a signal structure for generating a signal indicative of fluid delivered to and/or withdrawn from the skin and/or beneath the skin of the subject. In some embodiments, the device is repeatedly applicable to the skin of the subject to deliver to and/or withdraw fluid from the skin and/or beneath the skin of a subject.

In accordance with one set of embodiments, the device includes a support structure for application to the skin of the subject, a fluid transporter associated with the support structure, and a signal structure for generating a signal indicative of the fluid delivered to and/or withdrawn from the skin and/or beneath the skin of the subject. In some embodiments, the support structure and the signal structure are constructed and arranged to be connectable and/or detachable from each other.

The device, in another set of embodiments, includes a support structure for application to the skin of the subject, a fluid transporter associated with the support structure, and a signal structure for generating a signal indicative of the fluid delivered to and/or withdrawn from the skin and/or beneath the skin of the subject. In certain embodiments, the device is repeatedly applicable to the skin of the subject to deliver to and/or withdraw the fluid from the skin and/or beneath the skin of a subject.

The device, in still another set of embodiments, may includes a support structure for application to the skin of the subject, a fluid transporter associated with the support structure, and a stabilizing agent for stabilizing the fluid withdrawn from the skin in fluid communication with the fluid transporter.

In yet another set of embodiments, the device includes a support structure for application to the skin of the subject, a fluid transporter associated with the support structure, and an anticoagulant for stabilizing the fluid withdrawn from the skin in fluid communication with the fluid transporter.

In yet another set of embodiments, the device includes a support structure for application to the skin of the subject, transport means for delivery to and/or withdrawal of a fluid from the skin and/or beneath the skin of the subject, and a signal structure for generating a signal indicative of the fluid delivered to and/or withdrawn from the skin and/or beneath the skin of the subject. In certain embodiments, the support structure and the signal structure are constructed and arranged to be connectable and/or detachable from each other.

The device, in still another set of embodiments, includes a support structure for application to the skin of the subject, transport means for delivery to and/or withdrawal of a fluid from the skin and/or beneath the skin of the subject, and a signal structure for generating a signal indicative of the fluid delivered to and/or withdrawn from the skin and/or beneath the skin of the subject. In some embodiments, the device is repeatedly applicable to the skin of the subject to deliver to and/or withdraw the fluid from the skin and/or beneath the skin of a subject.

The device, in one set of embodiments, includes a support structure for application to the skin of the subject, transport means associated with the support structure, and a stabilizing agent for stabilizing the fluid withdrawn from the skin in fluid communication with the transport means.

In another set of embodiments, the device includes a support structure for application to the skin of the subject, transport means associated with the support structure, and an anticoagulant for stabilizing the fluid withdrawn from the skin in fluid communication with the transport means.

The device, in another set of embodiments, includes a support structure for application to the skin of the subject, a fluid transporter associated with the support structure, and a sensor for determining a condition of the fluid delivered to and/or withdrawn from the skin and/or beneath the skin of the subject. In some cases, the device is constructed and arranged to be wearable by the subject for at least about 15 minutes.

In yet another set of embodiments, the device includes a support structure for application to the skin of the subject, and a fluid transporter associated with the support structure. In some embodiments, the device is constructed and arranged to be wearable by the subject for at least about 15 minutes.

The device, in still another set of embodiments, includes a support structure for application to the skin of the subject, and a fluid transporter associated with the support structure. The device, according to some embodiments, may be constructed and arranged to be wearable by the subject for at least about 15 minutes.

In accordance with yet another set of embodiments, the device includes a support structure for application to the skin of the subject, transport means for delivery to and/or withdrawal of the fluid from the skin and/or beneath the skin of the subject, and a sensor for determining a condition of the fluid delivered to and/or withdrawn from the skin and/or beneath the skin of the subject. In certain embodiments, the device is constructed and arranged to be wearable by the subject for at least about 15 minutes.

The device, in one set of embodiments, includes a support structure for application to the skin of the subject, and transport means for withdrawal of interstitial fluid from the skin and/or from beneath the skin of the subject. In some cases, the device is constructed and arranged to be wearable by the subject for at least about 15 minutes.

In another set of embodiments, the device includes a support structure for application to the skin of the subject, and transport means for withdrawal of the fluid from one or more capillaries in the skin of the subject. In some instances, the device is constructed and arranged to be wearable by the subject for at least about 15 minutes.

In accordance with yet another set of embodiments, the device includes a support structure for application to the skin of the subject, and a fluid transporter associated with the support structure. In certain embodiments, a portion of the device is constructed and arranged to be replaceable without disassembling the device, where the device cannot function to deliver and/or withdraw fluid without the replaceable portion being present within the device, and/or where the replaceable portion does not comprise a power source.

In still another set of embodiments, the device includes a support structure for application to the skin of the subject, a fluid transporter associated with the support structure, and a removable module constructed and arranged to be reversibly removable from the support structure, wherein the removable module comprises one or more of a pressure controller, an actuator for the fluid transporter, a sensor, a fluid assay, a collection chamber, a processor, a memory component, a transmitter, or a display.

In another aspect, the present invention is directed to a device for extraction of a medium from a subject. According to one set of embodiments, the device includes a support structure for application to the skin of the subject, means for extracting a medium of the subject from or through the subject's skin at an extraction area, a sensor for determining at least one condition of a medium extractable from the subject through the at least one perforation, and a signal and/or display generator for generating a signal and/or display relating to the condition of the medium determined by the sensor. In some embodiments, the device comprises at least one of a largest lateral dimension, parallel to the extraction area, of no more than about 7 cm, a largest vertical dimension, extending from the skin of the subject when the device is positioned for extraction of the medium, of no more than about 3 cm, or a mass of no more than about 150 g.

The device, in another set of embodiments, includes a support structure for application to the skin of the subject, and means for extracting a medium of the subject from or through the subject's skin at an extraction area. In some cases, the device is constructed and arranged to be wearable by the subject for a period of at least 15 minutes.

In yet another aspect, the present invention is directed to a device for delivering to and/or withdrawing fluid from the skin and/or beneath the skin of a subject; in some cases, the device may be a self-contained device.

In one set of embodiments, the device may include a support structure for application to the skin of the subject, the support structure comprising transport means for delivery to and/or withdrawal of fluid from the skin and/or beneath the skin of a subject, and a sensor for determining a condition of a fluid delivered to and/or withdrawn from the skin and/or beneath the skin of the subject. In some cases, the device is constructed and arranged to be wearable by the subject for at least about 15 minutes.

The device, according to another set of embodiments, comprises a support structure for application to the skin of the subject, the support structure comprising transport means for delivery to and/or withdrawal of interstitial fluid from the skin and/or beneath the skin of a subject. In some embodiments, the device is constructed and arranged to be wearable by the subject for at least about 15 minutes.

In yet another set of embodiments, the device includes a support structure for application to the skin of the subject, the support structure comprising transport means for delivery to and/or withdrawal of fluid from one or more capillaries in the skin of a subject. In certain embodiments, the device is constructed and arranged to be wearable by the subject for at least about 15 minutes.

In one set of embodiments, the device includes a support structure for application to the skin of the subject, the support structure comprising transport means for delivery to and/or withdrawal of fluid from the skin and/or beneath the skin of a subject. In some cases, the self-contained device, when affixed to the skin, comprises at least one of a largest lateral dimension of no more than about 5 cm, a largest vertical dimension, extending from the skin of the subject when the device is applied to the subject, of no more than about 1 cm, or a mass of no more than about 25 g.

In another set of embodiments, the device includes a support structure for application to the skin of the subject, the support structure comprising transport means for delivery to and/or withdrawal of fluid from the skin and/or beneath the skin of a subject, and a signal structure for generating a signal indicative of fluid delivered to and/or withdrawn from the skin and/or beneath the skin of the subject. In some embodiments, the self-contained device, when affixed to the skin, comprises at least one of a largest lateral dimension of no more than about 7 cm, a largest vertical dimension, extending from the skin of the subject when the device is applied to the subject, of no more than about 3 cm, or a mass of no more than about 150 g.

The device, according to still another set of embodiments, includes a support structure for application to the skin of the subject, the support structure comprising transport means for delivery to and/or withdrawal of fluid from the skin and/or beneath the skin of a subject. In some cases, the device is constructed and arranged to be wearable by the subject for at least about 15 minutes.

In still another aspect, the present invention is generally directed to a self-contained device for delivering to and/or withdrawing a fluid from the skin and/or beneath the skin of a subject. In one set of embodiments, the device includes a support structure for application to the skin of the subject, and a fluid transporter for delivery to and/or withdrawal of a fluid from the skin and/or beneath the skin of the subject. In certain embodiments, the self-contained device, when affixed to the skin of the subject, comprises at least one of: (1) a largest lateral dimension, when the device is positioned for extraction of the fluid, parallel to the extraction area, of no more than about 5 cm, (2) a largest vertical dimension, extending from the skin of the subject when the device is applied to the subject, of no more than about 1 cm, or (3) a mass of no more than about 25 g, absent the fluid.

In another set of embodiments, the device includes a support structure for application to the skin of the subject, a fluid transporter for delivery to and/or withdrawal of a fluid from the skin and/or beneath the skin of the subject, and a signal structure for generating a signal indicative of the fluid delivered to and/or withdrawn from the skin and/or beneath the skin of the subject. In some embodiments, the self-contained device, when affixed to the skin of the subject, comprises at least one of: (1) a largest lateral dimension, when the device is positioned for extraction of the fluid, parallel to the extraction area, of no more than about 7 cm, (2) a largest vertical dimension, extending from the skin of the subject when the device is applied to the subject, of no more than about 3 cm, or (3) a mass of no more than about 150 g, absent the fluid.

The device, in still another set of embodiments, includes a support structure for application to the skin of the subject, and a fluid transporter for delivery to and/or withdrawal of the fluid from the skin and/or beneath the skin of the subject. In some cases, the device is constructed and arranged to be wearable by the subject for at least about 15 minutes.

The device, in yet another set of embodiments, includes a support structure for application to the skin of the subject, and a fluid transporter associated with the support structure. In some embodiments, the self-contained device, when affixed to the skin of the subject, comprises at least one of: (1) a largest lateral dimension, when the device is positioned for extraction of the fluid, parallel to the extraction area, of no more than about 6 cm, (2) a largest vertical dimension, extending from the skin of the subject when the device is applied to the subject, of no more than about 1.5 cm, or (3) a mass of no more than about 25 g, absent the fluid.

In accordance with yet another set of embodiments, the device includes a support structure for application to the skin of the subject, and transport means for delivery to and/or withdrawal of a fluid from the skin and/or beneath the skin of the subject. In some embodiments, the self-contained device, when affixed to the skin of the subject, comprises at least one of: (1) a largest lateral dimension, when the device is positioned for extraction of the fluid, parallel to the extraction area, of no more than about 5 cm, (2) a largest vertical dimension, extending from the skin of the subject when the device is applied to the subject, of no more than about 1 cm, or (3) a mass of no more than about 25 g, absent the fluid.

In still another set of embodiments, the device includes a support structure for application to the skin of the subject, transport means for delivery to and/or withdrawal of a fluid from the skin and/or beneath the skin of the subject, and a signal structure for generating a signal indicative of the fluid delivered to and/or withdrawn from the skin and/or beneath the skin of the subject. In certain embodiments, the self-contained device, when affixed to the skin of the subject, comprises at least one of: (1) a largest lateral dimension, when the device is positioned for extraction of the fluid, parallel to the extraction area, of no more than about 7 cm, (2) a largest vertical dimension, extending from the skin of the subject when the device is applied to the subject, of no more than about 3 cm, or (3) a mass of no more than about 150 g, absent the fluid.

In another set of embodiments, the device includes a support structure for application to the skin of the subject, and transport means for delivery to and/or withdrawal of the fluid from the skin and/or beneath the skin of the subject. In certain instances, the device is constructed and arranged to be wearable by the subject for at least about 15 minutes.

In accordance with yet another set of embodiments, the device includes a support structure for application to the skin of the subject, and transport means for delivery to and/or withdrawal of the fluid from the skin and/or beneath the skin of the subject. In some embodiments, the self-contained device, when affixed to the skin of the subject, comprises at least one of: (1) a largest lateral dimension, when the device is positioned for extraction of the fluid, parallel to the extraction area, of no more than about 6 cm, (2) a largest vertical dimension, extending from the skin of the subject when the device is applied to the subject, of no more than about 1.5 cm, or (3) a mass of no more than about 25 g, absent the fluid.

The invention, in yet another aspect, is generally directed to a method. According to one set of embodiments, the method includes acts of applying a device to the skin of a subject able to deliver to and/or withdraw fluid from the subject, e.g., to or from the skin and/or beneath the skin of the subject, the device comprising a support structure comprising transport means for delivery to and/or withdrawal of fluid from the skin and/or beneath the skin of a subject, and a signal structure for generating a signal indicative of fluid delivered to and/or withdrawn from the skin and/or beneath the skin of the subject, delivering to and/or withdrawing fluid from the subject using the device, e.g., to or from the skin and/or beneath the skin, and removing only one of the support structure and the signal structure from the skin of the subject.

In another set of embodiments, the method includes acts of applying a device to the skin of a subject able to withdraw fluid from the subject, the device comprising a support structure comprising transport means for withdrawal of fluid from the skin of a subject, and transport means associated with the support structure, withdrawing fluid from the subject using the device, and shipping at least a portion of the device containing the withdrawn fluid to a secondary site for analysis.

In one set of embodiments, the method includes acts of applying a device to the skin of a subject, where the device comprises: (1) a support structure for application to the skin of the subject, (2) a fluid transporter associated with the support structure; and (3) a signal structure for generating a signal indicative of the fluid delivered to and/or withdrawn from the skin and/or beneath the skin of the subject, delivering to and/or withdrawing the fluid from the skin and/or beneath the skin of the subject using the device, and removing only one of the support structure and the signal structure from the skin of the subject. The device may be able to deliver to and/or withdraw a fluid from the skin and/or beneath the skin of a subject.

The method, in accordance with yet another set of embodiments, includes acts of applying a device to the skin of a subject, where the device comprises: (1) a support structure for application to the skin of the subject, and (2) a fluid transporter associated with the support structure, withdrawing the fluid from the skin and/or beneath the skin of the subject using the fluid transporter, and transporting at least a portion of the device containing the withdrawn fluid to a secondary site for analysis. In some cases, the device may be able to withdraw a fluid from the skin and/or from beneath the skin of the subject.

The method, in accordance with still another set of embodiments, includes acts of applying a device to the skin of a subject, the device able to deliver to and/or withdraw a fluid from the skin and/or beneath the skin of the subject, the device comprising: (1) a support structure for application to the skin of the subject, (2) transport means for delivery to and/or withdrawal of the fluid from the skin and/or beneath the skin of the subject, and (3) a signal structure for generating a signal indicative of the fluid delivered to and/or withdrawn from the skin and/or beneath the skin of the subject; delivering to and/or withdrawing the fluid from the skin and/or beneath the skin of the subject using the device, and removing only one of the support structure and the signal structure from the skin of the subject.

According to another set of embodiments, the method includes acts of applying a device to the skin of a subject, where the device comprises: (1) a support structure for application to the skin of the subject, and (2) transport means for withdrawal of a fluid from the skin and/or from beneath the skin of a subject associated with the support structure, withdrawing the fluid from the skin and/or beneath the skin of the subject using the transport means, and transporting at least a portion of the device containing the withdrawn fluid to a secondary site for analysis. In some embodiments, the device may be able to withdraw a fluid from the skin and/or from beneath the skin of the subject.

In yet another set of embodiments, the method includes acts of applying a self-contained device to the skin of a subject able to deliver to and/or withdraw a fluid from the skin and/or beneath the skin of the subject, and activating the device. In some embodiments, the self-contained device, when affixed to the skin of the subject, comprises at least one of: (1) a largest lateral dimension, when the device is positioned for extraction of the fluid, parallel to the extraction area, of no more than about 5 cm, (2) a largest vertical dimension, extending from the skin of the subject when the device is applied to the subject, of no more than about 1 cm, or (3) a mass of no more than about 25 g, absent the fluid.

The method, in still another set of embodiments, includes acts of applying a self-contained device to the skin of a subject, and activating the device. In some embodiments, the device comprises: (1) a support structure for application to the skin of the subject; (2) a fluid transporter associated with the support structure; and (3) a signal structure for generating a signal indicative of the fluid delivered to and/or withdrawn from the skin and/or beneath the skin of the subject. In certain instances, the self-contained device, when affixed to the skin of the subject, comprises at least one of: (1) a largest lateral dimension, when the device is positioned for extraction of the fluid, parallel to the extraction area, of no more than about 5 cm, (2) a largest vertical dimension, extending from the skin of the subject when the device is applied to the subject, of no more than about 1 cm, or (3) a mass of no more than about 25 g, absent the fluid.

In yet another set of embodiments, the method includes acts of applying a self-contained device to the skin of a subject able to deliver to and/or withdraw a fluid from the skin and/or beneath the skin of a subject, allowing the subject to wear the device for at least about 15 minutes, and delivering to and/or withdrawing the fluid from the skin and/or beneath the skin of the subject.

The method, in accordance with yet another set of embodiments, includes acts of applying a self-contained device to the skin of a subject able to withdraw a fluid from the skin and/or from beneath the skin of the subject, allowing the subject to wear the device for at least about 15 minutes, withdrawing the fluid from the skin and/or from beneath the skin of the subject using the device, and determining an analyte suspected of being contained within the fluid withdrawn from the skin of the subject.

Another set of embodiments is generally directed to a method including acts of applying a self-contained device to the skin of a subject able to withdraw a fluid from the skin of the subject, allowing the subject to wear the device for at least about 15 minutes, and withdrawing the fluid directly from one or more capillaries in the skin of the subject using the device.

The method, in another set of embodiments, includes acts of applying a self-contained device to the skin of a subject able to deliver to and/or withdraw a fluid from the skin and/or beneath the skin of the subject, and activating the device. In some cases, the self-contained device, when affixed to the skin of the subject, comprises at least one of: (1) a largest lateral dimension, when the device is positioned for extraction of the fluid, parallel to the extraction area, of no more than about 6 cm, (2) a largest vertical dimension, extending from the skin of the subject when the device is applied to the subject, of no more than about 1.5 cm, or (3) a mass of no more than about 25 g, absent the fluid.

The method, in accordance with another set of embodiments, is generally directed to acts of applying a self-contained device to the skin of a subject, and activating the device. In certain embodiments, the device comprises: (1) a support structure for application to the skin of the subject, (2) transport means for delivery to and/or withdrawal of a fluid from the skin and/or beneath the skin of the subject, and (3) a signal structure for generating a signal indicative of the fluid delivered to and/or withdrawn from the skin and/or beneath the skin of the subject. In some cases, the self-contained device, when affixed to the skin of the subject, comprises at least one of: (1) a largest lateral dimension, when the device is positioned for extraction of the fluid, parallel to the extraction area, of no more than about 5 cm, (2) a largest vertical dimension, extending from the skin of the subject when the device is applied to the subject, of no more than about 1 cm, or (3) a mass of no more than about 25 g, absent the fluid.

In another aspect, the invention is directed to a device for withdrawing fluid from the skin of a subject. According to one set of embodiments, the device includes a support structure for application to the skin of the subject, transport means associated with the support structure, and a stabilizing agent for stabilizing the fluid withdrawn from the skin.

In another set of embodiments, the device includes a support structure for application to the skin of the subject, transport means associated with the support structure, and an anticoagulant for stabilizing the fluid withdrawn from the skin.

According to another set of embodiments, the device includes acts of applying a self-contained device to the skin of a subject able to deliver to and/or withdraw fluid from the subject, e.g., to or from the skin and/or beneath the skin, and activating the device. In some embodiments, the self-contained device, when affixed to the skin, comprises at least one of a largest lateral dimension of no more than about 5 cm, a largest vertical dimension, extending from the skin of the subject when the device is applied to the subject, of no more than about 1 cm, or a mass of no more than about 25 g.

In yet another set of embodiments, the method includes acts of applying a self-contained device to the skin, the device comprising a support structure comprising transport means for delivery to and/or withdrawal of fluid from the skin and/or beneath the skin of a subject, and a signal structure for generating a signal indicative of fluid delivered to and/or withdrawn from the skin and/or beneath the skin of the subject, and activating the device. In some cases, the self-contained device, when affixed to the skin, comprises at least one of a largest lateral dimension of no more than about 5 cm, a largest vertical dimension, extending from the skin of the subject when the device is applied to the subject, of no more than about 1 cm, or a mass of no more than about 25 g.

The method, in still another set of embodiments, includes acts of applying a self-contained device to the skin of a subject able to deliver to and/or withdraw fluid from the subject, e.g., to or from the skin and/or beneath the skin, allowing the subject to wear the device for at least about 15 minutes, and delivering to and/or withdrawing fluid from the subject, e.g., to or from the skin and/or beneath the skin of the subject.

In yet another set of embodiments, the method includes acts of applying a self-contained device to the skin of a subject able to deliver to and/or withdraw fluid from the subject, e.g., to or from the skin and/or beneath the skin allowing the subject to wear the device for at least about 15 minutes, withdrawing fluid from the subject using the device, and sensing an analyte suspected of being contained within the fluid withdrawn from the device.

The method, in still another set of embodiments, includes acts of applying a self-contained device to the skin of a subject able to withdraw fluid from the subject, allowing the subject to wear the device for at least about 15 minutes, and withdrawing fluid directly from one or more capillaries in the skin of the subject using the device.

In another aspect, the present invention is directed to a method of making one or more of the embodiments described herein. In another aspect, the present invention is directed to a method of using one or more of the embodiments described herein.

Other advantages and novel features of the present invention will become apparent from the following detailed description of various non-limiting embodiments of the invention when considered in conjunction with the accompanying figures. In cases where the present specification and a document incorporated by reference include conflicting and/or inconsistent disclosure, the present specification shall control. If two or more documents incorporated by reference include conflicting and/or inconsistent disclosure with respect to each other, then the document having the later effective date shall control.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
Fig. 1A-1B illustrate devices according to certain embodiments of the invention;
Figs. 2A-2C illustrate devices according to various embodiments of the invention;
Fig. 2D illustrates a kit containing more than one device, in yet another embodiment of the invention;
Fig. 2E illustrates a device according to still another embodiment of the invention;
Fig. 3 illustrates a device in one embodiment of the invention, having a vacuum chamber;
Fig. 4 illustrates a device in another embodiment of the invention, having a vacuum chamber and a storage chamber;
Fig. 5 illustrates a device in yet another embodiment of the invention, having a flow controller;
Fig. 6 illustrates a device in yet another embodiment of the invention, having an exit port;
Figs. 7A-7C illustrate a device in still another embodiment illustrating a reversibly deformable structure;
Figs. 8A-8C illustrate various devices according to various embodiments of the invention;
Figs. 9A-9B illustrate data collected from certain devices in accordance with certain embodiments of the invention;
Figs. 10A-10C illustrate various modular devices according to certain embodiments of the invention;
Fig. 11 illustrates a device comprising a housing, in yet another embodiment of the invention;
Fig. 12 illustrates another device of the invention, comprising a signal structure;
Fig. 13 illustrates a device of the invention having various dimensions;
Fig. 14 illustrates another device of the invention having various dimensions;
Fig. 15 illustrates a device of the invention; and
Fig. 16 is a schematic diagram used to illustrate modularity in accordance with certain embodiments of the invention.

### DETAILED DESCRIPTION

The present invention generally relates, in certain aspects, to relatively small devices applied to the skin, modular systems, and methods of use thereof. In some aspects, the device is constructed and arranged to have more than one module. For instance, the device may have a module for delivering to and/or withdrawing fluid from the skin and/or beneath the skin of a subject and a module for transmitting a signal indicative of the fluid delivered to and/or withdrawn from the skin and/or beneath the skin of the subject, a module for analyzing a fluid withdrawn from the skin and/or beneath the skin of the subject, or the like. In some embodiments, the modules are connectable and/or detachable from each other, and in some cases, the connections and/or detachments may be performed while the device is in contact with the subject, e.g., while affixed to the subject. In some embodiments, the device may be repeatedly applicable to the skin of the subject to deliver to and/or withdraw fluid from the skin and/or beneath the skin of a subject, e.g., at the same location, or at different locations on the skin of the subject. In some aspects, the devices may be self-contained and/or have a relatively small size, and in some cases, the device may be sized such that it is wearable and/or able to be carried by a subject. For example, the device may have a mass and/or dimensions that allow the device to be carried or worn by a subject for various periods of time, e.g., at least about an hour, at least about a day, at least about a week, etc., or no more than about an hour, no more than about 10 min, etc.

For example, some aspects of the present invention generally relate to modular systems for application to the skin for delivering to and/or withdrawing fluid from subjects, e.g., from the skin and/or beneath the skin. In some aspects, the present invention generally relates to relatively small devices applied to the skin and methods of use thereof for delivering to and/or withdrawing fluid from subjects, e.g., to or from the skin and/or beneath the skin.

Certain aspects of the present invention are directed to devices able to deliver to and/or withdraw fluid from the skin and/or beneath the skin of a subject, or other mucosal surface, as well as methods of use thereof. In some cases, the device may pierce the skin of the subject, and fluid can then be delivered to and/or withdrawn from the skin of the subject. Thus, it should be understood that in the discussions herein, references to withdrawing a fluid "from the skin" includes embodiments in which a fluid is delivered and/or withdrawn through the surface of the skin. For example, a fluid may be delivered into or withdrawn from a layer of skin in one embodiment, while in another embodiment a fluid may be delivered into or withdrawn from a region just below the skin of the subject, e.g., passing through the surface of the skin, as opposed to other routes of administration such as oral delivery. The subject is usually human, although non-human subjects may be used in certain instances, for instance, other mammals such as a dog, a cat, a horse, a rabbit, a cow, a pig, a sheep, a goat, a rat (e.g., *Rattus norvegicus),* a mouse (e.g., *Mus musculus),* a guinea pig, a hamster, a primate (e.g., a monkey, a chimpanzee, a baboon, an ape, a gorilla, etc.), or the like. If a fluid is withdrawn from the skin of the subject, the withdrawn fluid may be any suitable bodily fluid. In one set of embodiments, essentially any body fluid can be used, such as interstitial fluid, other skin-associated material, mucosal material or fluid, whole blood, perspiration and saliva, plasma, or any other bodily fluid.

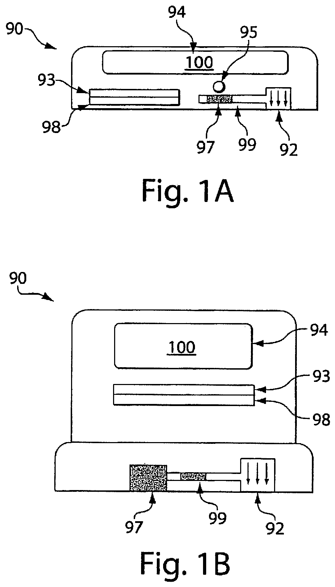

Non-limiting examples of various devices of the invention are shown in Fig. 1. In Fig. 1A, device 90 is used for withdrawing a fluid from a subject when the device is placed on the skin of a subject. Device 90 includes sensor 95 and fluid transporter 92, e.g., one or more needles, microneedles, etc., as discussed herein. In fluidic communication with fluid transporter 92 via fluidic channel 99 is sensing chamber 97. In one embodiment, sensing chamber 97 may contain agents such as particles, enzymes, dyes, etc., for analyzing bodily fluids, such as interstitial fluid or blood. In some cases, fluid may be withdrawn using fluid transporter 92 by a vacuum, for example, a self-contained vacuum contained within device 90. Optionally, device 90 also contains a display 94 and associated electronics 93, batteries or other power supplies, etc., which may be used to display sensor readings obtained via sensor 95. In addition, device 90 may also optionally contain memory 98, transmitters for transmitting a signal indicative of sensor 95 to a receiver, etc.

In the example shown in Fig. 1A, device 90 may contain a vacuum source (not shown) that is self-contained within device 90, although in other embodiments, the vacuum source may be external to device 90. (In still other instances, other systems may be used to deliver to and/or withdraw fluid from the skin and/or beneath the skin, as is discussed herein.) In one embodiment, after being placed on the skin of a subject, the skin may be drawn upward into a recess containing fluid transporter 92, for example, upon exposure to the vacuum source. Access to the vacuum source may be controlled by any suitable method, e.g., by piercing a seal or a septum; by opening a valve or moving a gate, etc. For instance, upon activation of device 90, e.g., by the subject, remotely, automatically, etc., the vacuum source may be put into fluidic communication with the recess such that skin is drawn into the recess containing fluid transporter 92 due to the vacuum. Skin drawn into the recess may come into contact with fluid transporter 92 (e.g., solid or hollow needles or microneedles), which may, in some cases, pierce the skin and allow a fluid to be delivered to and/or withdrawn from the skin and/or beneath the skin. In another embodiment, fluid transporter 92 may be actuated and moved downward to come into contact with the skin, and optionally retracted after use.

Another non-limiting example of a device is shown in Fig. 1B. This figure illustrates a device useful for delivering a fluid to the subject. Device 90 in this figure includes fluid transporter 92, e.g., one or more needles, microneedles, etc., as discussed herein. In fluidic communication with fluid transporter 92 via fluidic channel 99 is chamber 97, which may contain a drug or other agent to be delivered to the subject. In some cases, fluid may be delivered with a pressure controller, and/or withdrawn using fluid transporter 92 by a vacuum, for example, a self-contained vacuum contained within device 90. For instance, upon creating a vacuum, skin may be drawn up towards fluid transporter 92, and fluid transporter 92 may pierce the skin. Fluid from chamber 97 can then be delivered into or through the skin through fluid channel 99 and fluid transporter 92. Optionally, device 90 also contains a display 94 and associated electronics 93, batteries or other power supplies, etc., which may be used control delivery of fluid to or beneath the skin. In addition, device 90 may also optionally contain memory 98, transmitters for transmitting a signal indicative of device 90 or fluid delivery to a receiver, etc.

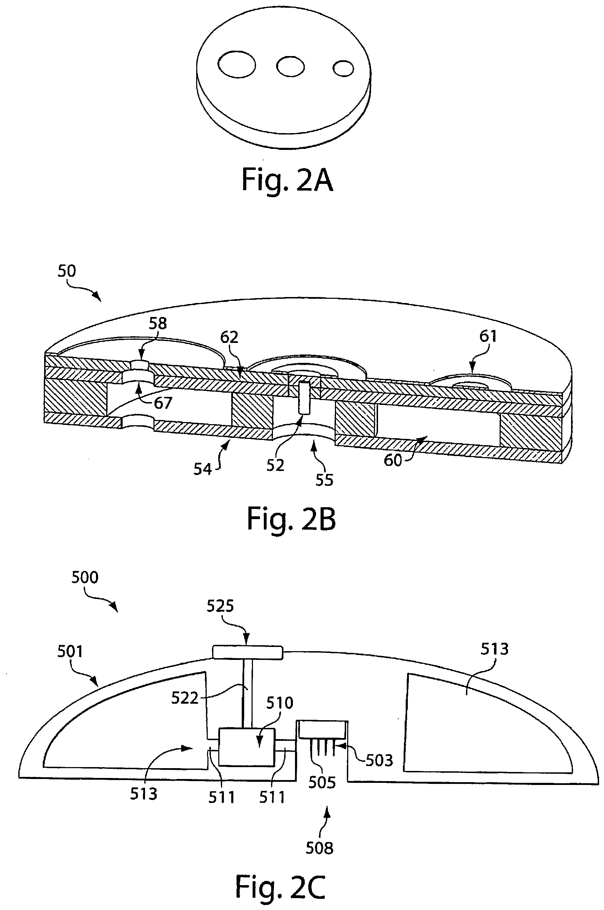

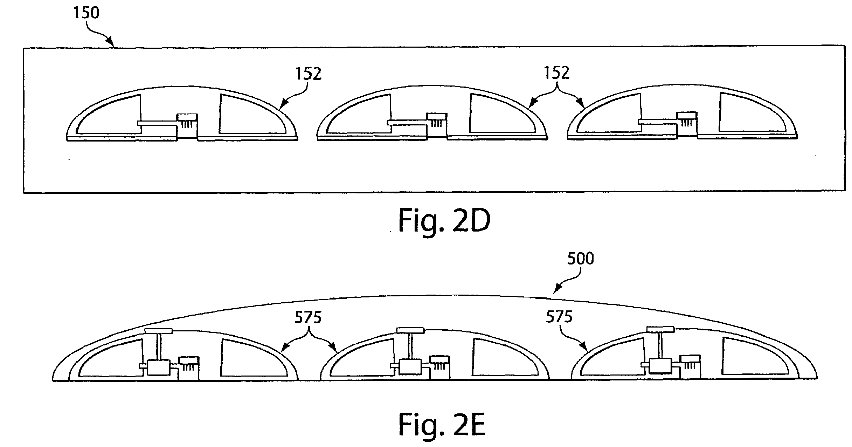

Yet another non-limiting example of a device of the invention is shown in Fig. 2. Fig. 2A illustrates a view of the device (with the cover removed), while Fig. 2B schematically illustrates the device in cross-section. In Fig. 2B, device 50 includes a needle 52 contained within a recess 55. Needle 52 may be solid or hollow, depending on the embodiment, and there may be one or more than one present. Device 50 also includes a self-contained vacuum chamber 60, which wraps around the central portion of the device where needle 52 and recess 55 are located. A channel 62 connects vacuum chamber 60 with recess 55, separated by a foil or a membrane 67. Also shown in device 50 is button 58. When pushed, button 58 breaks foil 67, thereby connecting vacuum chamber 50 with recess 55, creating a vacuum in recess 55. The vacuum may be used, for example, to draw skin into recess 55, preferably such that it contacts needle 52 and pierces the surface of the skin, thereby gaining access to an internal fluid such as blood or interstitial fluid. The fluid may be controlled, for example, by controlling the size of needle 52, and thereby the depth of penetration. For example, the penetration may be limited to the epidermis, e.g., to collect interstitial fluid, or to the dermis, e.g., to collect blood. In some cases, the vacuum may also be used to at least partially secure device 50 on the surface of the skin, and/or to assist in the withdrawal of fluid from the skin. For instance, fluid may flow into channel 62 under action of the vacuum, and optionally to sensor 61, e.g., for detection of an analyte contained within the fluid. For example, sensor 61 may produce a color change if an analyte is present, or otherwise produce a detectable signal.

Other components may be added to the example of the device illustrated in Fig. 2, in some embodiments of the invention. For example, device 50 may contain a cover, displays, ports, transmitters, sensors, chambers such as microfluidic chambers, channels such as microfluidic channels, and/or various electronics, e.g., to control or monitor fluid transport into or out of device 50, to determine an analyte present within a fluid delivered to and/or withdrawn from the skin and/or beneath the skin, to determine the status of the device, to report or transmit information regarding the device and/or analytes, or the like, as is discussed in more detail herein. As another example, device 50 may contain an adhesive, e.g., on surface 54, for adhesion of the device to the skin.

Yet another non-limiting example is illustrated with reference to Fig. 2C. In this example, device 500 includes a support structure 501, and an associated fluid transporter system 503. Fluid transporter system 503 includes one or more needles or microneedles 505, although other fluid transporters as discussed herein may also be used. Also shown in Fig. 2C is sensor 510, connected via channels 511 to recess 508 containing one or more needles or microneedles 505. Chamber 513 may be a self-contained vacuum chamber, and chamber 513 may be in fluidic communication with recess 508 via channel 511, for example, as controlled by a controller or an actuator (not shown). In this figure, device 500 also contains display 525, which is connected to sensor 510 via electrical connection 522. As an example of use of device 500, when fluid is drawn from the skin (e.g., blood, interstitial fluid, etc.), the fluid may flow through channel 511 to be determined by sensor 510, e.g., due to action of the vacuum from vacuum chamber 513. In some cases, the vacuum is used, for example, to draw skin into recess 508, preferably such that it contacts one or more needles or microneedles 505 and pierces the surface of the skin to gain access to a fluid internal of the subject, such as blood or interstitial fluid, etc. Upon determination of the fluid and/or an analyte present or suspected to be present within the fluid, a microprocessor or other controller may display on display 525 a suitable signal. As is discussed below, a display is shown in this figure by way of example only; in other embodiments, no display may be present, or other signals may be used, for example, lights, smell, sound, feel, taste, or the like.

In certain aspects, the device includes a fluid transporter able to deliver to or withdraw fluid from the skin and/or beneath the skin of the subject into the device. As used herein, "fluid transporter" is any component or combination of components that facilitates movement of a fluid from one portion of the device to another, and/or from the device to the skin of the subject or vice versa. For example, at or near the skin, a fluid transporter can be a hollow needle or a solid needle. If a solid needle is used, then if fluid migrates along the needle due to surface forces (e.g., capillary action), then the solid needle can be a fluid transporter. If fluid (e.g. blood or interstitial fluid) partially or fully fills an enclosure surrounding a needle after puncture of skin (whether the needle is or is not withdrawn from the skin after puncture), then the enclosure can define a fluid transporter. Other components including partially or fully enclosed channels, microfluidic channels, tubes, wicking members, vacuum containers, etc. can be fluid transporters.

The fluid may be withdrawn from and/or through the skin of a subject (or other mucosal surface). The fluid transporter may be, for example, one or more needles and/or microneedles, a hygroscopic agent, a cutter or other piercing element, an electrically-assisted system, or the like, as discussed in detail herein. If needles or microneedles are used, they may be solid or hollow, i.e., blood or other fluid may travel in and/or around the needles or microneedles into the device. In some cases, the needles or microneedles may also be removed from the skin of the subject, e.g., after insertion into the skin, for example, to increase the flow of blood or other fluids from the skin of the subject. For example, one or more needles or microneedles may be inserted into the skin and removed, and then a pressure gradient or a vacuum may be applied to the skin to withdraw a fluid, such as blood or interstitial fluid. In one set of embodiments, the fluid transporter includes solid needles that are removed from the skin and a cup or channel may be used to direct the flow of blood or other bodily fluids.

In some cases, more than one fluid transporter system may be present within the device. For instance, the device may be able to be used repeatedly, and/or the device may be able to deliver and/or withdraw fluid at more than one location on a subject, e.g., sequentially and/or simultaneously. As a specific example, in one set of embodiments, the device may include one or more needles, for instance, arranged in an array. In some embodiments, one or more of the needles may be a microneedle. In some cases, the device may be able to simultaneously deliver to and withdraw fluid from a subject. A non-limiting example of a device having more than one fluid transporter system is illustrated with reference to Fig. 2E. In this example, device 500 contains a plurality of structures such as those described herein for delivering to and/or withdrawing fluid from a subject, e.g., to and/or from the skin and/or beneath the skin of the subject. For example, device 500 in this example contains 3 such units, although any number of units are possible in other embodiments. In this example, device 500 contains three such fluid transporter systems 575. Each of these fluid transporter systems may independently have the same or different structures, depending on the particular application, and they may have structures such as those described herein.

Thus, in one aspect, the present invention is generally directed to devices and methods for withdrawing or extracting blood or other bodily fluids from a subject, e.g., from the skin and/or from beneath the skin, using devices containing a fluid transporter (for example, one or more needles or microneedles). The device may also contain, in some embodiments, a storage chamber having an internal pressure less than atmospheric pressure prior to receiving blood or other bodily fluids. In some cases, the fluid transporter may pierce the skin of the subject, and fluid can then be delivered to and/or withdrawn from the skin and/or beneath the skin of the subject.

In some cases, the device can be applied to the skin, and activated to withdraw fluid from the skin of the subject. The device, or a portion thereof, may then be processed to determine the fluid and/or an analyte within the fluid, alone or with an external apparatus. For example, fluid may be withdrawn from the device, and/or the device may contain sensors or agents able to determine the fluid and/or an analyte suspected of being contained in the fluid.

In some embodiments, the device may take the form of a skin "patch." Typically, a skin patch includes one or more layers of material that are adhered to the surface of the skin, and can be applied by the subject or another person. In certain embodiments, layers or portions of the skin patch may be removed, leaving other layers or portions behind on the skin. Often, the skin patch lacks an external power source, although the various layers of the patch may contain various chemicals, such as drugs, therapeutic agents, diagnostic agents, reaction entities, etc. In some cases, the skin patch may also include mechanical elements as well, for example, a cutter such as is discussed herein.

In other embodiments, however, the device may be larger. For example, the device may be an electrical and/or a mechanical device applicable or affixable to the surface of the skin, e.g., using adhesive, or other techniques such as those described herein. As another example, the device may be a handheld device that is applied to the surface of the skin of a subject. In some cases, however, the device may be sufficiently small or portable that the subject can self-administer the device. In certain embodiments, the device may also be powered. In some instances, the device may be applied to the surface of the skin, and is not inserted into the skin. In other embodiments, however, at least a portion of the device may be inserted into the skin, for example, mechanically. For example, in one embodiment, the device may include a cutter, such as a hypodermic needle, a knife blade, a piercing element (e.g., a solid or hollow needle), or the like, as discussed herein.

In some cases, the device may be designed such that portions of the device are separable. For example, a first portion of the device may be removed from the surface of the skin, leaving other portions of the device behind on the skin. In one embodiment, a stop may also be included to prevent or control the depth to which a cutter, fluid transporter, or other device inserts into the skin, e.g., to control penetration to the epidermis, dermis, etc.

Accordingly, as described herein, devices of the invention can be single-stage or multi-stage in some cases. That is, the device can define a single unit that includes one or more components integrally connected to each other which cannot readily be removed from each other by a user, or the device can include one or more components which are designed to be and can readily be removed from each other. As a non-limiting example of the later, a two-stage patch can be provided for application to the skin of a subject. The patch can include a first stage designed to reside proximate the skin of the subject for the duration of the analysis, which might include an analysis region, a reservoir or other material for creating vacuum or otherwise promoting the flow of fluid or other materials relative to the analysis region, a needle, microneedle, or other fluid transporter to access interstitial fluid or blood via a suction blister or without a suction blister or the like.

A second stage or portion of the device can be provided that can initiate operation of the device. For example, a two stage device can be applied to the skin of a subject. A button, switch, or other actuator associated with the second portion of the device can be activated by the subject or other user to cause insertion of one or more microneedles or other fluid transporter to the skin of the subject, or the like. Then, the second stage can be removed, e.g., by the subject or another user, and the first stage can remain on the skin of the subject to facilitate analysis. In another arrangement, a two-stage device can be provided where the first stage includes visualization or other signal-producing components and the second stage includes components necessary to facilitate the analysis, e.g., the second stage can include all components necessary to access bodily fluid, transport the fluid (if necessary) to a site of analysis, and the like, and that stage can be removed, leaving only a visualization stage for the subject or another entity to view or otherwise analyze as described herein.

Any or all of the arrangements described herein can be provided proximate a subject, for example on or proximate the skin of the subject, in various aspects. Activation of the devices can be carried out in a variety of ways, e.g., as described herein. For example, an on-skin device can be in the form of a patch or the like, optionally including multiple layers for activation, sensing, fluid flow, etc. In one embodiment, a patch or a device can be applied to a subject and a region of the patch or device activated (e.g., pushed, pressed, or tapped by a user) to inject a needle or a microneedle, or other fluid transporter, so as to access interstitial fluid or blood. The same or a different activation action, e.g., tapping or pushing action, can activate a vacuum source, open and/or close one or more of a variety of valves, or the like. The device can be a simple one in which it is applied to the skin and operates automatically (where e.g., application to the skin of the device allows access to interstitial fluid or blood, and delivers and/or withdraws fluid) or the patch or other device can be applied to the skin and one tapping or other activation action can cause fluid to flow through administration of a needle or a microneedle (or other fluid transporter), opening of a valve, activation of vacuum, etc., or any combination thereof. Any number of activation protocols can be carried out by a user repeatedly pushing, tapping, etc. a location or selectively, sequentially, and/or periodically activating a variety of switches (e.g., tapping regions of a patch or device).

In another arrangement, activation of one or more needles or microneedles, creation of suction blisters, opening and/or closing of valves, and other techniques to facilitate delivery and/or withdrawal of a fluid can be carried out electronically or in other manners facilitated by the subject or by an outside controlling entity (e.g., another user of the device). For example, a device or patch can be provided proximate the skin of a subject and a radio frequency, electromagnetic, or other signal can be provided by a nearby controller or a distant source to activate any of the needles, fluid transporters, blister devices, valves or other components of the devices described so that delivery and/or withdrawal of a fluid can be carried out as desired.

As discussed, various devices of the invention include various systems and methods for delivering to and/or withdrawing fluid from the skin and/or beneath the skin of the subject, according to certain embodiments. For instance, the device may comprise a needle such as a hypodermic needle, a vacuum source, a hygroscopic agent, or the like. Non-limiting examples of suitable delivery techniques include, but are not limited to, injection (e.g., using needles such as hypodermic needles) or a jet injector, such as those discussed below. For instance, in one embodiment, the fluid is delivered and/or withdrawn manually, e.g., by manipulating a plunger on a syringe. In another embodiment, the fluid can be delivered to and/or withdrawn from the skin and/or beneath the skin mechanically or automatically, e.g., using a piston pump or the like. Fluid may also be withdrawn using vacuums such as those discussed herein. For example, vacuum may be applied to a conduit, such as a needle, in fluidic communication with a bodily fluid in order to draw up at least a portion of the fluid from the skin. In yet another embodiment, fluid is withdrawn using capillary action (e.g., using a microfluidic channel or a hypodermic needle having a suitably narrow inner diameter). In still another embodiment, pressure may be applied to force fluid out of the needle.

In some cases, pooled regions of fluid may be created in the skin for facilitating delivery and/or withdrawal of fluid from the skin. For instance, fluid may be pooled within the skin that is drawn from the surrounding dermal and/or epidermal layers within the skin. The fluid may include, for example, interstitial fluid or blood. In other cases, however, no pooling is necessary for the delivery and/or withdrawal of fluid from the skin.

For instance, fluids withdrawn from the skin of the subject will often contain various analytes within the body that are important for diagnostic purposes, for example, markers for various disease states, such as glucose (e.g., for diabetics); other example analytes include ions such as sodium, potassium, chloride, calcium, magnesium, and/or bicarbonate (e.g., to determine dehydration); gases such as carbon dioxide or oxygen; H⁺ (i.e., pH); metabolites such as urea, blood urea nitrogen or creatinine; hormones such as estradiol, estrone, progesterone, progestin, testosterone, androstenedione, etc. (e.g., to determine pregnancy, illicit drug use, or the like); or cholesterol. Other examples include insulin, or hormone levels. Still other analytes include, but not limited to, high-density lipoprotein ("HDL"), low-density lipoprotein ("LDL"), albumin, alanine transaminase ("ALT"), aspartate transaminase ("AST"), alkaline phosphatase ("ALP"), bilirubin, lactate dehydrogenase, etc. (e.g., for liver function tests); luteinizing hormone or beta-human chorionic gonadotrophin (hCG) (e.g., for fertility tests); prothrombin (e.g., for coagulation tests); troponin, BNT or B-type natriuretic peptide, etc., (e.g., as cardiac markers); infectious disease markers for the flu, respiratory syncytial virus or RSV, etc.; or the like.

As discussed herein, certain embodiments of the present invention are generally directed at methods for withdrawing fluids from the body, and optionally determining one or more analytes within the withdrawn fluid. Thus, in some embodiments, at least a portion of the fluid may be stored, and/or analyzed to determine one or more analytes, e.g., a marker for a disease state, or the like. The fluid withdrawn from the skin may be subjected to such uses, and/or one or more fluids or other materials previously delivered to the skin and/or beneath the skin may be subject to such uses.

In other embodiments, fluid may be delivered to the subject, and such fluids may contain materials useful for delivery, e.g., forming at least a portion of the fluid, dissolved within the fluid, carried by the fluid (e.g., suspended or dispersed), or the like. Examples of suitable materials include, but are not limited to, particles such as microparticles or nanoparticles, a chemical, a drug or a therapeutic agent, a diagnostic agent, a carrier, or the like.

As used herein, the term "fluid" generally refers to a substance that tends to flow and to conform to the outline of its container. Typically, fluids are materials that are unable to withstand a static shear stress, and when a shear stress is applied, the fluid experiences a continuing and permanent distortion. The fluid may have any suitable viscosity that permits at least some flow of the fluid. Non-limiting examples of fluids include liquids and gases, but may also include free-flowing solid particles, viscoelastic fluids, and the like. For example, the fluid may include a flowable matrix or a gel, e.g., formed from biodegradable and/or biocompatible material such as polylactic acid, polyglycolic acid, poly(lactic-co-glycolic acid), etc., or other similar materials.

In some cases, fluids or other materials delivered to the subject may be used for indication of a past, present and/or future condition of the subject. Thus, the condition of the subject to be determined may be one that is currently existing in the subject, and/or one that is not currently existing, but the subject is susceptible or otherwise is at an increased risk to that condition. The condition may be a medical condition, e.g., diabetes or cancer, or other physiological conditions, such as dehydration, pregnancy, illicit drug use, or the like. Additional non-limiting examples are discussed below. In one set of embodiments, the materials may include a diagnostic agent, for example, one which can determine an analyte within the subject, e.g., one that is a marker for a disease state. As a specific non-limiting example, fluid delivered to the skin and/or beneath the skin of a subject may include a particle including an antibody directed at a marker produced by a bacterium.

In other cases, however, fluids or other materials delivered to the subject may be used to determine conditions that are external to the subject. For example, the fluids or other materials may contain reaction entities able to recognize pathogens or other environmental conditions surrounding the subject, for example, an antibody able to recognize an external pathogen (or pathogen marker). As a specific example, the pathogen may be anthrax and the antibody may be an antibody to anthrax spores. As another example, the pathogen may be a *Plasmodia* (some species of which causes malaria) and the antibody may be an antibody that recognizes the *Plasmodia.*

According to one set of embodiments, many devices as discussed herein use various techniques for delivering and/or withdrawing fluid, for example, in connection with fluid transporters, substance transfer components, microinsertion objects, or the like. For example, one or more needles and/or microneedles, a hygroscopic agent, a cutter or other piercing element, an electrically-assisted system, or the like may be used in conjunction with any device described herein. Additional examples of such techniques are described herein and/or in the applications incorporated herein. It is to be understood that, generally, fluids may be delivered and/or withdrawn in a variety of ways, and various systems and methods for delivering to and/or withdrawing fluid from the skin and/or beneath the skin are discussed below and/or in the applications incorporated herein. In one set of embodiments, techniques for piercing or altering the surface of the skin to transport a fluid are discussed, for example, using a needle such as a hypodermic needle or one or more microneedles, chemicals applied to the skin (e.g., penetration enhancers), or jet injectors or other techniques such as those discussed below.

As an example, in one method, a needle such as a hypodermic needle can be used to deliver to and/or withdraw fluid from the skin and/or beneath the skin. Hypodermic needles are well-known to those of ordinary skill in the art, and can be obtained commercially with a range of needle gauges. For example, the needle may be in the 20-30 gauge range, or the needle may be 32 gauge, 33 gauge, 34 gauge, etc.

Those of ordinary skill in the art can arrange needles relative to the skin for these purposes including, in one embodiment, introducing needles into the skin at an angle, relative to the skin's surface, other than 90°, i.e., to introduce a needle or needles into the skin in a slanting fashion so as to limit the depth of penetration. In another embodiment, however, the needles may enter the skin at approximately 90°.

If needles are present, there may be one or more needles, the needles may be of any suitable size and length, and the needles may each be solid or hollow. The needles may have any suitable cross-section (e.g., perpendicular to the direction of penetration), for example, circular, square, oval, elliptical, rectangular, rounded rectangle, triangular, polygonal, hexagonal, irregular, etc. For example, a needle may have a length of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, less than about 10 micrometers, etc. A needle may also have a largest cross-sectional dimension of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, less than about 10 micrometers, etc. For example, in one embodiment, a needle may have a rectangular cross section having dimensions of 175 micrometers by 50 micrometers. In one set of embodiments, the needle may have an aspect ratio of length to largest cross-sectional dimension of at least about 2:1, at least about 3:1, at least about 4:1, at least 5:1, at least about 7:1, at least about 10:1, at least about 15:1, at least about 20:1, at least about 25:1, at least about 30:1, etc.

In one embodiment, the needle is a microneedle. Typically, a microneedle will have an average cross-sectional dimension (e.g., diameter) of less than about a millimeter. It should be understood that references to "needle" or "microneedle" as discussed herein are by way of example and ease of presentation only, and that in other embodiments, more than one needle and/or microneedle may be present in any of the descriptions herein.

As an example, microneedles such as those disclosed in U.S. Patent No. 6,334,856, issued January 1, 2002, entitled "Microneedle Devices and Methods of Manufacture and Use Thereof," by *Allen, et al.,* may be used to deliver to and/or withdraw fluids (or other materials) from a subject. The microneedles may be hollow or solid, and may be formed from any suitable material, e.g., metals, ceramics, semiconductors, organics, polymers, and/or composites. Examples include, but are not limited to, medical grade stainless steel, titanium, nickel, iron, gold, tin, chromium, copper, alloys of these or other metals, silicon, silicon dioxide, and polymers, including polymers of hydroxy acids such as lactic acid and glycolic acid polylactide, polyglycolide, polylactide-co-glycolide, and copolymers with polyethylene glycol, polyanhydrides, polyorthoesters, polyurethanes, polybutyric acid, polyvaleric acid, polylactide-co-caprolactone, polycarbonate, polymethacrylic acid, polyethylenevinyl acetate, polytetrafluorethylene, polymethyl methacrylate, polyacrylic acid, or polyesters.

In some cases, more than one needle or microneedle may be used. For example, arrays of needles or microneedles may be used, and the needles or microneedles may be arranged in the array in any suitable configuration, e.g., periodic, random, etc. In some cases, the array may have 3 or more, 4 or more, 5 or more, 10 or more, 15 or more, 20 or more, 35 or more, 50 or more, 100 or more, or any other suitable number of needles or microneedles. In some embodiments, the device may have at least 3 but no more than 5 needles or microneedles (or other fluid transporters), at least 6 but no more than 10 needles or microneedles, or at least 11 but no more than 20 needles or microneedles.

In some cases, needles (or microneedles) may be present in an array selected such that the density of needles within the array is between about 0.5 needles/mm² and about 10 needles/mm², and in some cases, the density may be between about 0.6 needles/mm² and about 5 needles/mm², between about 0.8 needles/mm² and about 3 needles/mm², between about 1 needles/mm² and about 2.5 needles/mm², or the like. In some cases, the needles may be positioned within the array such that no two needles are closer than about 1 mm, about 0.9 mm, about 0.8 mm, about 0.7 mm, about 0.6 mm, about 0.5 mm, about 0.4 mm, about 0.3 mm, about 0.2 mm, about 0.1 mm, about 0.05 mm, about 0.03 mm, about 0.01 mm, etc.

In another set of embodiments, the needles (or microneedles) may be chosen such that the area of the needles (determined by determining the area of penetration or perforation on the surface of the skin of the subject by the microneedles) allows for adequate flow of fluid to or from the skin of the subject. The needles may be chosen to have smaller or larger areas (or smaller or large diameters), as long as the area of contact for the needles to the skin is sufficient to allow adequate blood flow from the skin of the subject to the device. For example, in certain embodiments, the needles may be selected to have a combined skin-penetration area of at least about 500 nm², at least about 1,000 nm², at least about 3,000 nm², at least about 10,000 nm², at least about 30,000 nm², at least about 100,000 nm², at least about 300,000 nm², at least about 1 microns², at least about 3 microns², at least about 10 microns², at least about 30 microns², at least about 100 microns², at least about 300 microns², at least about 500 microns², at least about 1,000 microns², at least about 2,000 microns², at least about 2,500 microns², at least about 3,000 microns², at least about 5,000 microns², at least about 8,000 microns², at least about 10,000 microns², at least about 35,000 microns², at least about 100,000 microns², at least about 300,000 microns², at least about 500,000 microns², at least about 800,000 microns², at least about 8,000,000 microns², etc., depending on the application.

The needles or microneedles may have any suitable length, and the length may be, in some cases, dependent on the application. For example, needles designed to only penetrate the epidermis may be shorter than needles designed to also penetrate into the dermis, or to extend beneath the dermis or the skin. In certain embodiments, the needles or microneedles may have a maximum penetration into the skin of no more than about 3 mm, no more than about 2 mm, no more than about 1.75 mm, no more than about 1.5 mm, no more than about 1.25 mm, no more than about 1 mm, no more than about 900 microns, no more than about 800 microns, no more than about 750 microns, no more than about 600 microns, no more than about 500 microns, no more than about 400 microns, no more than about 300 microns, no more than about 100 microns, no more than about 175 micrometers, no more than about 150 micrometers, no more than about 125 micrometers, no more than about 100 micrometers, no more than about 75 micrometers, no more than about 50 micrometers, etc. In certain embodiments, the needles or microneedles may be selected so as to have a maximum penetration into the skin of at least about 50 micrometers, at least about 100 micrometers, at least about 300 micrometers, at least about 500 micrometers, at least about 1 mm, at least about 2 mm, at least about 3 mm, etc.

In one set of embodiments, the needles (or microneedles) may be coated. For example, the needles may be coated with a substance that is delivered when the needles are inserted into the skin. For instance, the coating may comprise heparin, an anticoagulant, an anti-inflammatory compound, an analgesic, an anti-histamine compound, etc. to assist with the flow of blood from the skin of the subject, or the coating may comprise a drug or other therapeutic agent such as those described herein. The drug or other therapeutic agent may be one used for localized delivery (e.g., of or proximate the region to which the coated needles or microneedles are applied), and/or the drug or other therapeutic agent may be one intended for systemic delivery within the subject. Examples of such drugs and therapeutic agents include those described herein.

As still another example, pressurized fluids may be used to deliver fluids or other materials into or through the skin, for instance, using a jet injector or a "hypospray." Typically, such devices produce a high-pressure "jet" of liquid or powder (e.g., a biocompatible liquid, such as saline) that drives material into the skin, and the depth of penetration may be controlled, for instance, by controlling the pressure of the jet. The pressure may come from any suitable source, e.g., a standard gas cylinder or a gas cartridge. A non-limiting example of such a device can be seen in U.S. Patent No. 4,103,684, issued August 1, 1978, entitled "Hydraulically Powered Hypodermic Injector with Adapters for Reducing and Increasing Fluid Injection Force," by Ismach. Pressurization of the liquid may be achieved, for example, using compressed air or gas, for instance, from a gas cylinder or a gas cartridge.

In some embodiments, fluid may be withdrawn using a hygroscopic agent applied to the surface of the skin or proximate the skin. For example, a device as described herein may contain a hygroscopic agent. In some cases, pressure may be applied to drive the hygroscopic agent into the skin. Hygroscopic agents typically are able to attract water from the surrounding environment, for instance, through absorption or adsorption. Non-limiting examples of hygroscopic agents include sugar, honey, glycerol, ethanol, methanol, sulfuric acid, methamphetamine, iodine, many chloride and hydroxide salts, and a variety of other substances. Other examples include, but are not limited to, zinc chloride, calcium chloride, potassium hydroxide, or sodium hydroxide. In some cases, a suitable hygroscopic agent may be chosen based on its physical or reactive properties, e.g., inertness or biocompatibility towards the skin of the subject, depending on the application.

In some embodiments, the device may comprise a cutter able to cut or pierce the surface of the skin. The cutter may comprise any mechanism able to create a path through which fluids may be delivered to and/or withdrawn from the skin and/or beneath the skin. For example, the cutter may comprise a hypodermic needle, a blade (e.g., a knife blade, a serrated blade, etc.), a piercing element (e.g., a lancet or a solid or a hollow needle), or the like, which can be applied to the skin to create a suitable conduit for the withdrawal of fluid from the skin. In one embodiment, a cutter is used to create such a pathway and removed, and then fluid may be delivered and/or withdrawn via this pathway. In another embodiment, the cutter remains in place within the skin, and fluid may be delivered and/or withdrawn through a conduit within the cutter.

In some embodiments, fluid may be withdrawn using an electric charge. For example, reverse iontophoresis may be used. Without wishing to be bound by any theory, reverse iontophoresis uses a small electric current to drive charged and highly polar compounds across the skin. Since the skin is negatively charged at physiologic pH, it acts as a permselective membrane to cations, and the passage of counterions across the skin induces an electroosmotic solvent flow that may carry neutral molecules in the anode-to-cathode direction. Components in the solvent flow may be analyzed as described elsewhere herein. In some instances, a reverse iontophoresis apparatus may comprise an anode cell and a cathode cell, each in contact with the skin. The anode cell may be filled, for example, with an aqueous buffer solution (e.g., aqueous Tris buffer) having a pH greater than 4 and an electrolyte (e.g., sodium chloride). The cathode cell can be filled with aqueous buffer. As one example, a first electrode (e.g., an anode) can be inserted into the anode cell and a second electrode (e.g., a cathode) can be inserted in the cathode cell. In some embodiments, the electrodes are not in direct contact with the skin.

A current may be applied to induce reverse iontophoresis, thereby withdrawing a fluid from the skin. The current applied may be, for example, greater than 0.01 mA, greater than 0.3 mA, greater than 0.1 mA, greater than 0.3 mA, greater than 0.5 mA, or greater than 1 mA. It should be understood that currents outside these ranges may be used as well. The current may be applied for a set period of time. For example, the current may be applied for greater than 30 seconds, greater than 1 minute, greater than 5 minutes, greater than 30 minutes, greater than 1 hour, greater than 2 hours, or greater than 5 hours. It should be understood that times outside these ranges may be used as well.

In one set of embodiments, the device may comprise an apparatus for ablating the skin. Without wishing to be bound by any theory, it is believed that ablation comprises removing a microscopic patch of stratum corneum (e.g., ablation forms a micropore), thus allowing access to bodily fluids. In some cases, thermal, radiofrequency, and/or laser energy may be used for ablation. In some instances, thermal ablation may be applied using a heating element. Radiofrequency ablation may be carried out using a frequency and energy capable of heating water and/or tissue. A laser may also be used to irradiate a location on the skin to remove a portion. In some embodiments, the heat may be applied in pulses such that a steep temperature gradient exists essentially perpendicular to the surface of the skin. For example, a temperature of at least 100 °C, at least 200 °C, at least 300 °C, or at least 400 °C may be applied for less than 1 second, less than 0.1 seconds, less than 0.01 seconds, less than 0.005 seconds, or less than 0.001 seconds.

In some embodiments, the device may comprise a mechanism for taking a solid sample of tissue. For example, a solid tissue sample may be acquired by methods such as scraping the skin or cutting out a portion. Scraping may comprise a reciprocating action whereby an instrument is scraped along the surface of the skin in two or more directions. Scraping can also be accomplished by a rotating action, for example parallel to the surface of the skin and in one direction (e.g., with a roller drum) or parallel to the surface of the skin and in a circular manner (e.g., with a drilling instrument). A cutting mechanism may comprise a blade capable of making one or more incisions and a mechanism for removing a portion of tissue (e.g., by suction or mechanically picking up) or may use a pincer mechanism for cutting out a portion of tissue. A cutting mechanism may also function by a coring action. For example, a hollow cylindrical device can be penetrated into the skin such that a cylindrical core of tissue may be removed. A solid sample may be analyzed directly or may be liquefied prior to analysis. Liquefaction can comprise treatment with organic solvents, enzymatic solutions, etc.

The device may also contain, in some aspects, a vacuum source. In some cases, the vacuum source is one that is self-contained within the device, i.e., the device need not be connected to an external vacuum source (e.g., a house vacuum) during use of the device to withdraw blood from the skin. For example, in one set of embodiments, the vacuum source may include a vacuum chamber having a pressure less than atmospheric pressure before blood (or other fluid) is withdrawn into the device, i.e., the vacuum chamber is at a "negative pressure" (that is, negative relative to atmospheric pressure) or a "vacuum pressure" (or just having a "vacuum"). For example, the vacuum in the vacuum chamber may be at least about 50 mmHg, at least about 100 mmHg, at least about 150 mmHg, at least about 200 mmHg, at least about 250 mmHg, at least about 300 mmHg, at least about 350 mmHg, at least about 400 mmHg, at least about 450 mmHg, at least about 500 mmHg, at least 550 mmHg, at least 600 mmHg, at least 650 mmHg, at least about 700 mmHg, or at least about 750 mmHg, i.e., below atmospheric pressure. Thus, the pressure within the vacuum is at a "reduced pressure" relative to atmospheric pressure, e.g., the vacuum chamber is a reduced pressure chamber. However, in other embodiments, it should be understood that other pressures may be used and/or that different methods may be used to produce other pressures (greater than or less than atmospheric pressure). As non-limiting examples, an external vacuum or a mechanical device may be used as the vacuum source; various additional examples are discussed in detail herein.

In some embodiments, fluids may be withdrawn from the skin using vacuum. The vacuum may be an external vacuum source, and/or the vacuum source may be self-contained within the device. For example, vacuums of at least about 50 mmHg, at least about 100 mmHg, at least about 150 mmHg, at least about 200 mmHg, at least about 250 mmHg, at least about 300 mmHg, at least about 350 mmHg, at least about 400 mmHg, at least about 450 mmHg, at least about 500 mmHg, at least 550 mmHg, at least 600 mmHg, at least 650 mmHg, at least about 700 mmHg, or at least about 750 mmHg may be applied to the skin. As used herein, "vacuum" refers to pressures that are below atmospheric pressure.

As mentioned, any source of vacuum may be used. For example, the device may comprise an internal vacuum source, and/or be connectable to a vacuum source is external to the device, such as a vacuum pump or an external (line) vacuum source. In some cases, vacuum may be created manually, e.g., by manipulating a syringe pump, a plunger, or the like, or the low pressure may be created mechanically or automatically, e.g., using a piston pump, a syringe, a bulb, a Venturi tube, manual (mouth) suction, etc., or the like.

As a specific, non-limiting example, in one embodiment, a device may be used to withdraw fluid using vacuum without an external power and/or vacuum source. Examples of such devices that can use vacuum include skin patches, strips, tapes, bandages, or the like. For instance, a skin patch may be contacted with the skin of a subject, and a vacuum created through a change in shape of a portion of the skin patch or other device (e.g., using a shape memory polymer), which may be used to deliver to and/or withdraw fluid from the skin and/or beneath the skin. As another example, a mechanical device may be used to create the vacuum, For example, springs, coils, expanding foam (e.g., from a compressed state), a shape memory polymer, shape memory metal, or the like may be stored in a compressed or wound state upon application to a subject, then released (e.g., unwinding, uncompressing, etc.), to mechanically create the vacuum. In yet another example, a chemical reaction may be used to create a vacuum, e.g., a reaction in which a gas is produced, which can be harnessed to provide the mechanical force to create a vacuum. In some embodiments, the device may be used to create a vacuum automatically, once activated, without any external control by a user.

In one set of embodiments, the device may be able to create a pressure differential (e.g. a vacuum). For example, the device may contain a pressure differential chamber, such as a vacuum chamber or a pressurized chamber, that can be used to create a pressure differential. The pressure differential may also be created by a pressure regulator. As used here, "pressure regulator" is a pressure controller component or system able to create a pressure differential between two or more locations. The pressure differential should be at least sufficient to urge or move fluid or other material in accordance with various embodiments of the invention as discussed herein, and the absolute pressures at the two or more locations are not important so long as their differential is appropriate, and their absolute values are reasonable for the purposes discussed herein. For example, the pressure regulator may produce a pressure higher than atmospheric pressure in one location, relative to a lower pressure at another location (atmospheric pressure or some other pressure), where the differential between the pressures is sufficient to urge or move fluid in accordance with the invention. In another example, the regulator or controller will involve a pressure lower than atmospheric pressure (a vacuum) in one location, and a higher pressure at another location(s) (atmospheric pressure or a different pressure) where the differential between the pressures is sufficient to urge or move fluid in accordance with the invention. Wherever "vacuum" or "pressure" is used herein, it should be understood that the opposite can be implemented as well, as would be understood by those of ordinary skill in the art, i.e., a vacuum chamber can be replaced in many instances with a pressure chamber, for creating a pressure differential suitable for urging the movement of fluid or other material.

The pressure regulator may be an external source of vacuum (e.g. a lab, clinic, hospital, etc., house vacuum line or external vacuum pump), a mechanical device, a vacuum chamber, pre-packaged vacuum chamber, a pressurized chamber, or the like. In some cases, vacuum may be created manually, e.g., by manipulating a syringe pump, a plunger, or the like, or the low pressure may be created mechanically or automatically, e.g., using a piston pump, a syringe, a bulb, a Venturi tube, manual (mouth) suction, etc., or the like. Vacuum chambers can be used in some embodiments, where the device contains, e.g., regions in which a vacuum exits or can be created (e.g. a variable volume chamber, a change in volume of which will affect vacuum or pressure). A vacuum chamber can include pre-evacuated (i.e., pre-packaged) chambers or regions, and/or self-contained actuators.

A "self-contained" vacuum (or pressure) regulator means one that is associated with (e.g., on or within) the device, e.g. one that defines an integral part of the device, or is a separate component constructed and arranged to be specifically connectable to the particular device to form a pressure differential (i.e., not a connection to an external source of vacuum such as a hospital's, clinic's, or lab's house vacuum line, or a vacuum pump suitable for general use). In some embodiments, the self-contained vacuum source may be actuated in some fashion to create a vacuum within the device. For instance, the self-contained vacuum source may include a piston, a syringe, a mechanical device such as a vacuum pump able to create a vacuum within the device, and/or chemicals or other reactants that can react to increase or decrease pressure which, with the assistance of mechanical or other means driven by the reaction, can form a pressure differential associated with a pressure regulator. Chemical reaction can also drive mechanical actuation with or without a change in pressure based on the chemical reaction itself. A self-contained vacuum source can also include an expandable foam, a shape memory material, or the like.

One category of self-contained vacuum or pressure regulators of the invention includes self-contained assisted regulators. These are regulators that, upon actuation (e.g., the push of a button, or automatic actuation upon, e.g., removal from a package or urging a device against the skin), a vacuum or pressure associated with the device is formed where the force that pressurizes or evacuates a chamber is not the same as the actuation force. Examples of self-contained assisted regulators include chambers evacuated by expansion driven by a spring triggered by actuation, release of a shape-memory material or expandable material upon actuation, initiation of a chemical reaction upon actuation, or the like.

Another category of self-contained vacuum or pressure regulators of the invention are devices that are not necessarily pre-packaged with pressure or vacuum, but which can be pressurized or evacuated, e.g. by a subject, health care professional at a hospital or clinic prior to use, e.g. by connecting a chamber of the device to a source of vacuum or pressure. For example, the subject, or another person, may actuate the device to create a pressure or vacuum within the device, for example, immediately prior to use of the device.

The vacuum or pressure regulator may be a "pre-packaged" pressure or vacuum chamber in the device when used (i.e., the device can be provided ready for use by a subject or practitioner with an evacuated region on or in the device, without the need for any actuation to form the initial vacuum). A pre-packaged pressure or vacuum chamber regulator can, e.g., be a region evacuated (relative to atmospheric pressure) upon manufacture and/or at some point prior to the point at which it is used by a subject or practitioner. For example, a chamber is evacuated upon manufacture, or after manufacture but before delivery of the device to the user, e.g. the clinician or subject. For instance, in some embodiments, the device contains a vacuum chamber having a vacuum of at least about 50 mmHg, at least about 100 mmHg, at least about 150 mmHg, at least about 200 mmHg, at least about 250 mmHg, at least about 300 mmHg, at least about 350 mmHg, at least about 400 mmHg, at least about 450 mmHg, at least about 500 mmHg, at least about 550 mmHg, at least about 600 mmHg, at least about 650 mmHg, at least about 700 mmHg, or at least about 750 mmHg below atmospheric pressure. In yet another example, a chemical reaction may be used to create a vacuum, e.g., a reaction in which a gas is produced, which can be harnessed to provide the mechanical force to create a vacuum. In still another example, a component of the device may be able to create a vacuum in the absence of mechanical force. In another example, the device may include a self-contained vacuum actuator, for example, chemical reactants, a deformable structure, a spring, a piston, etc.

In some cases, the device includes an interface that is able to apply vacuum to the skin. The interface may be, for example, a suction cup or a circular bowl that is placed on the surface of the skin, and vacuum applied to the interface to create a vacuum. In one set of embodiments, the interface is part of a support structure, as discussed herein. The interface may be formed from any suitable material, e.g., glass, rubber, polymers such as silicone, polyurethane, nitrile rubber, EPDM rubber, neoprene, or the like. In some cases, the seal between the interface and the skin may be enhanced (e.g., reducing leakage), for instance, using vacuum grease, petroleum jelly, a gel, or the like. In some cases, the interface may be relatively small, for example, having a diameter of less than about 5 cm, less than about 4 cm, less than about 3 cm, less than about 2 cm, less than about 1 cm, less than about 5 mm, less than about 4 mm, less than about 3 mm, less than about 2 mm, or less than about 1 mm. The interface may be circular, although other shapes are also possible, for example, square, star-shaped (having 5, 6, 7, 8, 9, 10, 11, etc. points), tear-drop, oval, rectangular, or the like. In some cases, non-circular shapes may be used since high-energy points, e.g., the points or corners of the shape may enhance or accelerate blister formation.

In some cases, the support structure may be able to draw skin towards the fluid transporter. For example, in one set of embodiments, the support structure may include a vacuum interface. The interface may be connected with a vacuum source (external and/or internal to the device), and when a vacuum is applied, skin may be drawn towards the support structure, e.g., for contact with a fluid transporter, such as with one or more needles or microneedles. The interface may also be selected, in some cases, to keep the size of the contact region below a certain area, e.g., to minimize pain or discomfort to the subject, for aesthetic reasons, or the like. The interface may be constructed out of any suitable material, e.g., glass, plastic, or the like.

In one set of embodiments, a device of the present invention may not have an external power and/or vacuum source. In some cases, the device is "pre-loaded" with a suitable vacuum source; for instance, in one embodiment, the device may be applied to the skin and activated in some fashion to create and/or access the vacuum source. As one example, a device of the present invention may be contacted with the skin of a subject, and a vacuum created through a change in shape of a portion of the device (e.g., using a shape memory polymer), or the device may contain one or more sealed, self-contained vacuum chambers, where a seal is punctured in some manner to create a vacuum. For instance, upon puncturing the seal, a vacuum chamber may be in fluidic communication with one or more needles, which can be used to move the skin towards the device, withdraw fluid from the skin, or the like.

As another example, a shape memory polymer may be shaped to be flat at a first temperature (e.g., room temperature) but curved at a second temperature (e.g., body temperature), and when applied to the skin, the shape memory polymer may alter from a flat shape to a curved shape, thereby creating a vacuum. As yet another example, a mechanical device may be used to create the vacuum, For example, springs, coils, expanding foam (e.g., from a compressed state), a shape memory polymer, shape memory metal, or the like may be stored in a compressed or wound released upon application to a subject, then released (e.g., unwinding, uncompressing, etc.), to mechanically create the vacuum. Non-limiting examples of shape-memory polymers and metals include Nitinol, compositions of oligo(epsilon-caprolactone)diol and crystallizable oligo(rho-dioxanone)diol, or compositions of oligo(epsilon-caprolactone)dimethacrylate and n-butyl acrylate.

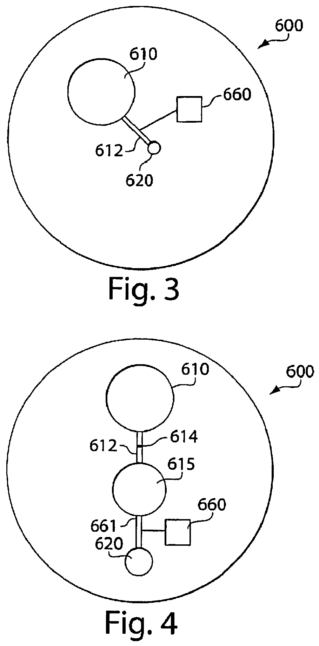

In one set of embodiments, the device contains a vacuum chamber that is also used as a storage chamber to receive blood or other fluid withdrawn from the skin of the subject into the device. For instance, blood withdrawn from a subject through or via the fluid transporter may enter the vacuum chamber due to its negative pressure (i.e., because the chamber has an internal pressure less than atmospheric pressure), and optionally stored in the vacuum chamber for later use. A non-limiting example is illustrated in Fig. 3. In this figure, device 600 contains vacuum chamber 610, which is connected to fluid transporter 620 (which may be, e.g., one or more microneedles). Upon activation of vacuum chamber 610 (e.g., using actuator 660, as discussed below), vacuum chamber 610 may be put into fluidic communication with fluid transporter 620. Fluid transporter 620 may accordingly cause negative pressure to be applied to the skin of the subject, for instance, due to the internal pressure within vacuum chamber 610. Fluid (e.g., blood) withdrawn from the skin via fluid transporter 620 may accordingly be drawn into the device and into vacuum chamber 610, e.g., through conduit 612. The fluid collected by the device can then be analyzed within the device or removed from the device for analysis, storage, etc.

In another set of embodiments, however, the device may include separate vacuum chambers and storage chambers (e.g., chambers to store fluid such as blood from the skin of the subject). The vacuum chamber and storage chambers may be in fluid communication, and may have any suitable arrangement. In some embodiments, the vacuum from the vacuum chamber may be used, at least in part, to withdraw fluid from the skin, which is then directed into a storage chamber, e.g., for later analysis or use, for example, as discussed below. As an example, blood may be withdrawn into the device, flowing towards a vacuum chamber, but the fluid may be prevented from entering the vacuum chamber. For instance, in certain embodiments, a material permeable to gas but not to a liquid such as blood may be used. For example, the material may be a membrane such as a hydrophilic or hydrophobic membrane having a suitable porosity, a porous structure, a porous ceramic frit, a dissolvable interface (e.g., formed from a salt or a polymer, etc.), or the like.

One non-limiting example is illustrated in Fig. 4. In this figure, device 600 contains vacuum chamber 610 and storage chamber 615. Vacuum chamber 610 can be put in fluidic communication with storage chamber 615 via conduit 612, which contains material 614. Material 614 may be any material permeable to gas but not to a liquid in this example, e.g., material 614 may be a membrane such as a hydrophilic membrane or a hydrophobic membrane that has a porosity that allows gas exchange to occur but does not allow the passage of blood from the skin of the subject. When device 600 is actuated using actuator 660, blood (or other fluid) flows through fluid transporter 620 via conduit 661 into collection chamber 615 because of the internal vacuum pressure from vacuum chamber 610, which is not completely impeded by material 614 since it is permeable to gases. However, because of material 614, blood (or other bodily fluid) is prevented from entering vacuum chamber 610, and instead remains in storage chamber 615, e.g., for later analysis or use.

In some embodiments, the flow of blood (or other fluid) into the storage chamber may be controlled using a flow controller. The flow controller may be manually and/or automatically controlled to control the flow of blood. The flow controller may activate or deactivate when a certain amount or volume of fluid has entered the storage chamber in certain cases. For instance, the flow controller may stop blood flow after a predetermined amount or volume of blood has entered the storage chamber, and/or the flow controller may be able to control the internal pressure of the storage chamber, e.g., to a specific level, such as a predetermined level. Examples of suitable flow controllers for the device include, but are not limited to, a membrane, a valve, a dissolvable interface, a gate, or the like.

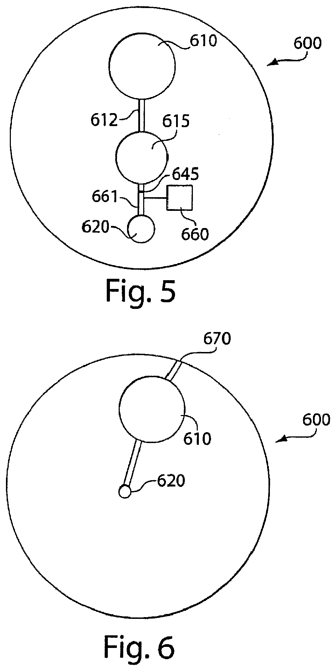

One non-limiting example of a flow controller is now illustrated with reference to Fig. 5. In this example figure, device 600 includes a vacuum chamber 610 and a storage chamber 615. Fluid entering device 600 via fluid transporter 620 is prevented from entering storage chamber 615 due to flow controller 645 present within conduit 611. However, under suitable conditions, flow controller 645 may be opened, thereby allowing at least some fluid to enter storage chamber 615. In some cases, for instance, storage chamber 615 also contains at least a partial vacuum, although this vacuum may be greater or less than the pressure within chamber 610. In other embodiments, flow controller 645 may initially be open, or be externally controllable (e.g., via an actuator), or the like. In some cases, the flow controller may control the flow of fluid into the device such that, after collection, at least some vacuum is still present in the device.

Thus, in some cases, the device may be constructed and arranged to reproducibly obtain from the skin of the subject a controlled amount of fluid, e.g., a controlled amount or volume of blood. The amount of fluid reproducibly obtained from the skin of the subject may be controlled, for example, using flow controllers, materials permeable to gas but not to liquids, membranes, valves, pumps, gates, microfluidic systems, or the like, as discussed herein. In particular, it should be noted that the volume of blood or other fluid obtained from the skin of the subject need not be strictly a function of the initial vacuum pressure or volume within the device. For example, a flow controller may initially be opened (e.g., manually, automatically, electronically, etc.) to allow fluid to begin entering the device; and when a predetermined condition is reached (e.g., when a certain volume or amount of blood has entered the device), the flow controller may be closed at that point, even if some vacuum pressure remains within the device. In some cases, this control of fluid allows the amount of fluid reproducibly obtained from the skin of the subject to be controlled to a great extent. For example, in one set of embodiments, the amount of fluid withdrawn from the skin of the subject may be controlled to be less than about 1 ml, may be less than about 300 microliters, less than about 100 microliters, less than about 30 microliters, less than about 10 microliters, less than about 3 microliters, less than about 1 microliter, etc.

In some embodiments, the device may be connected to an external apparatus for determining at least a portion of the device, a fluid removed from the device, an analyte suspected of being present within the fluid, or the like. For example, the device may be connected to an external analytical apparatus, and fluid removed from the device for later analysis, or the fluid may be analyzed within the *device in situ,* e.g., by adding one or more reaction entities to the device, for instance, to a storage chamber, or to analytical chamber within the device. For example, in one embodiment, the external apparatus may have a port or other suitable surface for mating with a port or other suitable surface on the device, and blood or other fluid can be removed from the device using any suitable technique, e.g., using vacuum or pressure, etc. The blood may be removed by the external apparatus, and optionally, stored and/or analyzed in some fashion. For example, in one set of embodiments, the device may include an exit port for removing a fluid from the device (e.g., blood). In some embodiments, fluid contained within a storage chamber in the device may be removed from the device, and stored for later use or analyzed outside of the device. In some cases, the exit port may be separate from the fluid transporter. An example is shown with exit port 670 and fluid transporter 620 in device 600 in Fig. 6. As shown in this figure, the exit port can be in fluidic communication with vacuum chamber 610.

In some cases, the device may be an electrical and/or a mechanical device applicable or affixable to the surface of the skin, e.g., using adhesive, or other techniques such as those described herein. For example, in one set of embodiments, the device may include a support structure that contains an adhesive that can be used to immobilize the device to the skin. The adhesive may be permanent or temporary, and may be used to affix the device to the surface of the skin. The adhesive may be any suitable adhesive, for example, a pressure sensitive adhesive, a contact adhesive, a permanent adhesive, a cyanoacrylate, glue, gum, hot melts, an epoxy, a hydrogel, a hydrocolloid, or the like. In some cases, the adhesive is chosen to be biocompatible or hypoallergenic.

In another set of embodiments, the device may be mechanically held to the skin. For example, the device may include mechanical elements such as straps, belts, buckles, strings, ties, elastic bands, or the like. For example, a strap may be worn around the device to hold the device in place against the skin of the subject. In yet another set of embodiments, a combination of these and/or other techniques may be used. As one non-limiting example, the device may be affixed to a subject's arm or leg using adhesive and a strap.

In some embodiments, the device may include a support structure for application to the skin of the subject. The support structure may be used, as discussed herein, for applying the fluid transporter to the surface of the skin of the subject, e.g., so that fluid may be delivered to and/or withdrawn from the skin and/or beneath the skin of the subject. In some cases, the support structure may immobilize the fluid transporter such that the fluid transporter cannot move relative to the support structure; in other cases, however, the fluid transporter may be able to move relative to the support structure. In one embodiment, as a non-limiting example, the fluid transporter is immobilized relative to the support structure, and the support structure is positioned within the device such that application of the device to the skin causes at least a portion of the fluid transporter to pierce the skin of the subject.

For instance, in one set of embodiments, the support structure, or a portion of the support structure, may move from a first position to a second position. For example, the first position may be one where the support structure has immobilized relative thereto a fluid transporter does not contact the skin (e.g., the fluid transporter may be contained within a recess), while the second position may be one where the fluid transporter does contact the skin, and in some cases, the fluid transporter may pierce the skin. The support structure may be moved using any suitable technique, e.g., manually, mechanically, electromagnetically, using a servo mechanism, or the like. In one set of embodiments, for example, the support structure may be moved from a first position to a second position by pushing a button on the device, which causes the support structure to move (either directly, or through a mechanism linking the button with the support structure). Other mechanisms (e.g., dials, levers, sliders, etc., as discussed herein) may be used in conjunction of or instead of a button. In another set of embodiments, the support structure may be moved from a first position to a second position automatically, for example, upon activation by a computer, upon remote activation, after a period of time has elapsed, or the like. For example, in one embodiment, a servo connected to the support structure is activated electronically, moving the support structure from the first position to the second position.

In some cases, the support structure may also be moved from the second position to the first position. For example, after fluid has been delivered to and/or withdrawn from the skin and/or beneath the skin, e.g., using a fluid transporter the support structure may be moved, which may move the fluid transporter away from contact with the skin. The support structure may be moved from the second position to the first position using any suitable technique, including those described above, and the technique for moving the support structure from the second position to the first position may be the same or different as that moving the support structure from the first position to the second position.

In some cases, the support structure includes a reversibly deformable structure. In one set of embodiments, the device includes a reversibly deformable structure able to drive a fluid transporter or a substance transfer component into the skin, e.g., so that the fluid transporter can withdraw a fluid from the skin and/or from beneath the skin of a subject, and/or so that the fluid transporter can deliver fluid or other material to a subject, e.g. deliver the fluid or other material to the skin and/or to a location beneath the skin of a subject. The reversibly deformable structure may be a structure that can be deformed using unaided force (e.g., by a human pushing the structure), or other forces (e.g., electrically-applied forces, mechanical interactions or the like), but is able to restore its original shape after the force is removed or at least partially reduced. For example, the structure may restore its original shape spontaneously, or some action (e.g., heating) may be needed to restore the structure to its original shape.

The reversibly deformable structure may be formed out of a suitable elastic material, in some cases. For example, the structure may be formed from a plastic, a polymer, a metal, etc. In one set of embodiments, the structure may have a concave or convex shape. For instance, the edges of the structure may be put under compressive stress such that the structure "bows" out to form a concave or convex shape. A person pushing against the concave or convex shape may deform the structure, but after the person stops pushing on the structure, the structure may be able to return to its original concave or convex shape, e.g., spontaneously or with the aid of other forces as previously discussed. In some cases, the device may be bistable, i.e., having two different positions in which the device is stable.

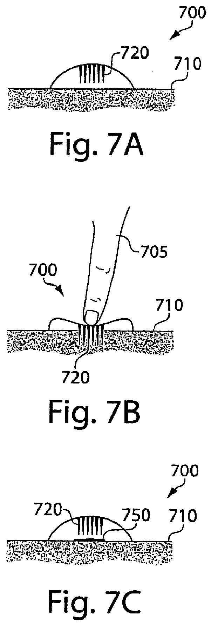

In one set of embodiments, the device may include a flexible concave member or a reversibly deformable structure that is moveable between a first configuration and a second configuration. For instance, the first configuration may have a concave shape, such as a dome shape, and the second configuration may have a different shape, for example, a deformed shape (e.g., a "squashed dome"), a convex shape, an inverted concave shape, or the like. See, for example, Fig. 7B. The flexible concave member (or a reversibly deformable structure) may be moved between the first configuration and the second configuration manually, e.g., by pushing on the flexible concave member using a hand or a finger, and/or the flexible concave member may be moved using an actuator such as is described herein. In some cases, the flexible concave member may be able to spontaneously return from the second configuration back to the first configuration, e.g., as is shown in Fig. 7. In other cases, however, the flexible concave member may not be able to return to the first configuration, for instance, in order to prevent accidental repeated uses of the flexible concave member. The flexible concave member, in some embodiments, may be a reversibly deformable structure, although in other embodiments, it need not be.

The flexible concave member (or a reversibly deformable structure, in some embodiments) may be mechanically coupled to one or more needles (e.g., microneedles), or other fluid transporters such as those discussed herein. The needle may be directly immobilized on the flexible concave member, or the needles can be mechanically coupled to the flexible concave member using bars, rods, levers, plates, springs, or other suitable structures. The needle (or other fluid transporter), in some embodiments, is mechanically coupled to the flexible concave member such that the needle is in a first position when the flexible concave member is in a first configuration and the needle is in a second position when the flexible concave member is in a second configuration.

In some cases, relatively high speeds and/or accelerations may be achieved, and/or insertion of the needle may occur in a relatively short period of time, e.g., as is discussed herein. The first position and the second position, in some cases, may be separated by relatively small distances. For example, the first position and the second position may be separated by a distance of less than about 10 mm, less than about 9 mm, less than about 8 mm, less than about 7 mm, less than about 6 mm, less than about 5 mm, less than about 4 mm, less than about 3 mm, or less than about 2 mm, etc. However, even within such distances, in certain embodiments, high speeds and/or accelerations such as those discussed herein can be achieved.

During use, a device may be placed into contact with the skin of a subject such that a recess or other suitable applicator region is proximate or in contact with the skin. By moving the flexible concave member (or reversibly deformable structure) between a first configuration and a second configuration, because of the mechanical coupling, the flexible concave member is able to cause a needle (or other fluid transporter) to move to a second position within the recess or other applicator region and to contact or penetrate the skin of the subject.

In some embodiments, the device may also include a retraction mechanism able to move the needle (or other fluid transporter) away from the skin after the flexible concave member (or a reversibly deformable structure) reaches a second configuration. Retraction of the flexible concave member may, in some embodiments, be caused by the flexible concave member itself, e.g., spontaneously returning from the second configuration back to the first configuration, and/or the device may include a separate retraction mechanism, for example, a spring, an elastic member, a collapsible foam, or the like.

The needle (or other fluid transporter) may be used for delivering to and/or withdrawing fluids or other materials from a subject, e.g., to or from the skin and/or beneath the skin. For example, in some cases, a vacuum chamber having a reduced pressure or an internal pressure less than atmospheric pressure prior to receiving blood or other bodily fluids (e.g., interstitial fluid) may be used to assist in the withdrawal of the fluid from the skin after the needle (or other fluid transporter) has penetrated the skin. The fluid withdrawn from the skin may be collected in the vacuum chamber and/or in a collection chamber. The collection chamber may be separated from the vacuum chamber using a gas permeable membrane (e.g., one that is substantially impermeable to blood or other bodily fluids), a hydrophilic membrane, a porous structure, a dissolvable interface, or the like, e.g., as is discussed herein.

An example of a reversibly deformable structure is now illustrated with respect to Fig. 7. In Fig. 7A, structure 700 has a generally concave shape, and is positioned on the surface of skin 710. In some cases, structure 700 may be a flexible concave member. Structure 700 also contains a plurality of fluid transporters 720 for insertion into the skin. In Fig. 7B, a person (indicated by finger 705) pushes onto structure 700, deforming at least a portion of the structure and thereby forcing fluid transporters 720 into at least a portion of the skin. In Fig. 7C, after the person releases structure 700, the structure is allowed to return to its original position, e.g., spontaneously, lifting fluid transporters 720 out of the skin. In some cases, e.g., if the fluid transporters are sufficiently large or long, blood or other fluids 750 may come out of the skin through the holes created by the fluid transporters, and optionally the fluid may be collected by the device for later storage and/or use, as discussed herein.

The reversibly deformable structure (or the flexible concave member) may be formed from any suitable material, for example, a metal such as stainless steel (e.g., 301, 301LN, 304, 304L, 304LN, 304H, 305, 312, 321, 321H, 316, 316L, 316LN, 316Ti, 317L, 409, 410, 430, 440A, 440B, 440C, 440F, 904L), carbon steel, spring steel, spring brass, phosphor bronze, beryllium copper, titanium, titanium alloy steels, chrome vanadium, nickel alloy steels (e.g., Monel 400, Monel K 500, Inconel 600, Inconel 718, Inconel x 750, etc.), a polymer (e.g., polyvinylchloride, polypropylene, polycarbonate, etc.), a composite or a laminate (e.g., comprising fiberglass, carbon fiber, bamboo, Kevlar, etc.), or the like.

The reversibly deformable structure may be of any shape and/or size. In one set of embodiments, the reversibly deformable structure is not planar, and has a portion that can be in a first position (a "cocked" or predeployed position) or a second position (a "fired" or deployed position), optionally separated by a relatively high energy configuration. In some cases, both the first position and the second position are stable (i.e., the structure is bistable), although conversion between the first position and the second position requires the structure to proceed through an unstable configuration.

In one embodiment, the reversibly deformable structure is a flexible concave member. The reversibly deformable structure may have, for instance, a generally domed shape (e.g., as in a snap dome), and be circular (no legs), or the reversibly deformable structure may have other shapes, e.g., oblong, triangular (3 legs), square (4 legs), pentagonal (5 legs), hexagonal (6 legs), spider-legged, star-like, clover-shaped (with any number of lobes, e.g., 2, 3, 4, 5, etc.), or the like. The reversibly deformable structure may have, in some embodiments, a hole, dimple, or button in the middle. The reversibly deformable structure may also have a serrated disc or a wave shape. In some cases, a fluid transporter or a substance transfer component may be mounted on the reversibly deformable structure. In other cases, however, the fluid transporter or substance transfer component is mounted on a separate structure which is driven or actuated upon movement of the reversibly deformable structure.

In some embodiments, the device may exhibit a relatively high success rate of withdrawal of fluid from various subjects. For example, in some embodiments, the success rate of withdrawing at least about 5 microliters of blood from a subject may be at least about 95%, at least about 97%, at least about 98%, at least about 99%, or at least about 100%, as compared to prior art devices (e.g., lancet devices) which typically have success rates of less than 95%. In other embodiments, the volume may be at least about 0.1 microliters, at least about 0.3 microliters, at least about 0.5 microliters, at least about 1 microliter, at least about 3 microliters, at least about 5 microliters, or at least about 10 microliters. For instance, a population of subjects may be tested with both a prior art device and a device of the invention such that each subject is tested with both devices in a suitable location (e.g., the forearm) when determining success probabilities, where the population of subjects is randomly chosen. The population may be for example, at least 10, at least 100, at least 1,000, at least 10,000 or more individuals.

In certain aspects, the device may also contain an activator. The activator may be constructed and arranged to cause exposure of the fluid transporter to the skin upon activation of the activator. For example, the activator may cause a chemical to be released to contact the skin, one or more needles or microneedles to be driven into the skin, a vacuum to be applied to the skin, a jet of fluid to be directed to the skin, or the like. The activator may be activated by the subject, and/or by another person (e.g., a health care provider), or the device itself may be self-activating, e.g., upon application to the skin of a subject. The activator may be activated once, or multiple times in some cases.

The device may be activated, for example, by pushing a button, pressing a switch, moving a slider, turning a dial, or the like. The subject, and/or another person, may activate the activator. In some cases, the device may be remotely activated. For example, a health care provider may send an electromagnetic signal which is received by the device in order to activate the device, e.g., a wireless signal, a Bluetooth signal, an Internet signal, a radio signal, etc.

In some aspects, the device may include channels such as microfluidic channels, which may be used to deliver to and/or withdraw fluids and/or other materials from the skin and/or beneath the skin. In some cases, the microfluidic channels are in fluid communication with a fluid transporter that is used to deliver to and/or withdraw fluids from the skin and/or beneath the skin. For example, in one set of embodiments, the device may include a hypodermic needle or other needle (e.g., one or more microneedles) that can be inserted into the skin, and fluid may be delivered into or through the skin via the needle and/or withdrawn from the skin via the needle. The device may also include one or more microfluidic channels to contain fluid for delivery to the needle, e.g., from a source of fluid, and/or to withdraw fluid withdrawn from the skin, e.g., for delivery to an analytical chamber within the device, to a reservoir for later analysis, or the like.

In some cases, more than one chamber may be present within the device, and in some cases, some or all of the chambers may be in fluidic communication, e.g., via channels such as microfluidic channels. In various embodiments, a variety of chambers and/or channels may be present within the device, depending on the application. For example, the device may contain chambers for sensing an analyte, chambers for holding reagents, chambers for controlling temperature, chambers for controlling pH or other conditions, chambers for creating or buffering pressure or vacuum, chambers for controlling or dampening fluid flow, mixing chambers, or the like.

Thus, in one set of embodiments, the device may include a microfluidic channel. As used herein, "microfluidic," "microscopic," "microscale," the "micro-" prefix (for example, as in "microchannel"), and the like generally refers to elements or articles having widths or diameters of less than about 1 mm, and less than about 100 microns (micrometers) in some cases. In some embodiments, larger channels may be used instead of, or in conjunction with, microfluidic channels for any of the embodiments discussed herein. For examples, channels having widths or diameters of less than about 10 mm, less than about 9 mm, less than about 8 mm, less than about 7 mm, less than about 6 mm, less than about 5 mm, less than about 4 mm, less than about 3 mm, or less than about 2 mm may be used in certain instances. In some cases, the element or article includes a channel through which a fluid can flow. In all embodiments, specified widths can be a smallest width (i.e. a width as specified where, at that location, the article can have a larger width in a different dimension), or a largest width (i.e. where, at that location, the article has a width that is no wider than as specified, but can have a length that is greater). Thus, for instance, the microfluidic channel may have an average cross-sectional dimension (e.g., perpendicular to the direction of flow of fluid in the microfluidic channel) of less than about 1 mm, less than about 500 microns, less than about 300 microns, or less than about 100 microns. In some cases, the microfluidic channel may have an average diameter of less than about 60 microns, less than about 50 microns, less than about 40 microns, less than about 30 microns, less than about 25 microns, less than about 10 microns, less than about 5 microns, less than about 3 microns, or less than about 1 micron.

A "channel," as used herein, means a feature on or in an article (e.g., a substrate) that at least partially directs the flow of a fluid. In some cases, the channel may be formed, at least in part, by a single component, e.g. an etched substrate or molded unit. The channel can have any cross-sectional shape, for example, circular, oval, triangular, irregular, square or rectangular (having any aspect ratio), or the like, and can be covered or uncovered (i.e., open to the external environment surrounding the channel). In embodiments where the channel is completely covered, at least one portion of the channel can have a cross-section that is completely enclosed, and/or the entire channel may be completely enclosed along its entire length with the exception of its inlet and outlet.

A channel may have any aspect ratio (length to average cross-sectional dimension), e.g., an aspect ratio of at least about 2:1, more typically at least about 3:1, at least about 5:1, at least about 10:1, etc. As used herein, a "cross-sectional dimension," in reference to a fluidic or microfluidic channel, is measured in a direction generally perpendicular to fluid flow within the channel. A channel generally will include characteristics that facilitate control over fluid transport, e.g., structural characteristics and/or physical or chemical characteristics (hydrophobicity vs. hydrophilicity) and/or other characteristics that can exert a force (e.g., a containing force) on a fluid. The fluid within the channel may partially or completely fill the channel. In some cases the fluid may be held or confined within the channel or a portion of the channel in some fashion, for example, using surface tension (e.g., such that the fluid is held within the channel within a meniscus, such as a concave or convex meniscus). In an article or substrate, some (or all) of the channels may be of a particular size or less, for example, having a largest dimension perpendicular to fluid flow of less than about 5 mm, less than about 2 mm, less than about 1 mm, less than about 500 microns, less than about 200 microns, less than about 100 microns, less than about 60 microns, less than about 50 microns, less than about 40 microns, less than about 30 microns, less than about 25 microns, less than about 10 microns, less than about 3 microns, less than about 1 micron, less than about 300 nm, less than about 100 nm, less than about 30 nm, or less than about 10 nm or less in some cases. In one embodiment, the channel is a capillary.

In some aspects, the device may contain one or more chambers or reservoirs for holding fluid. In some cases, the chambers may be in fluidic communication with one or more fluid transporters and/or one or more microfluidic channels. For instance, the device may contain a chamber for collecting fluid withdrawn from a subject (e.g., for storage and/or later analysis), a chamber for containing a fluid for delivery to the subject (e.g., blood, saline, optionally containing drugs, hormones, vitamins, pharmaceutical agents, or the like), etc.

As mentioned, in some embodiments, blood or other bodily fluids may be stored within the device for later use and/or analysis. For example, the device may be attached to a suitable external apparatus able to analyze a portion of the device (e.g., containing the fluid), and/or the external apparatus may remove at least some of the blood or other fluid from the device for subsequent analysis and/or storage. In some cases, however, at least some analysis may be performed by the device itself, e.g., using one or more sensors, etc., contained within the device.

For example, as discussed in detail below, in some cases, a storage chamber may contain a reagent or a reaction entity able to react with an analyte suspected of being present in the blood (or other fluid) entering the device, and in some cases, the reaction entity may be determined to determine the analyte. In some cases, the determination may be made externally of the device, e.g., by determining a color change or a change in fluorescence, etc. The determination may be made by a person, or by an external apparatus able to analyze at least a portion of the device. In some cases, the determination may be made without removing blood from the device, e.g., from the storage chamber. (In other cases, however, blood or other fluid may first be removed from the device before being analyzed.) For example, the device may include one or more sensors (e.g., ion sensors such as K⁺ sensors, colorimetric sensors, fluorescence sensors, etc.), and/or contain "windows" that allow light to penetrate the device. The windows may be formed of glass, plastic, etc., and may be selected to be at least partially transparent to one or a range of suitable wavelengths, depending on the analyte or condition to be determined. As a specific example, the entire device (or a portion thereof) may be mounted in an external apparatus, and light from the external apparatus may pass through or otherwise interact with at least a portion of the device (e.g., be reflected or refracted via the device) to determine the analyte and/or the reaction entity.

In one aspect, the device may be interfaced with an external apparatus able to determine an analyte contained within a fluid in the device, for example within a storage chamber as discussed herein. For example, the device may be mounted on an external holder, the device may include a port for transporting fluid out of the device, the device may include a window for interrogating a fluid contained within the device, or the like. Examples may be seen in a U.S. Provisional Patent Application Serial No. 61/334,529, filed on May 13, 2010, entitled "Sampling Device Interfaces," incorporated herein by reference in its entirety.

Thus, the device, in certain embodiments, may contain a portion able to determine a fluid removed from the skin. For example, a portion of the device may contain a sensor, or reagents able to interact with an analyte contained or suspected to be present within the withdrawn fluid from the skin of the subject, for example, a marker for a disease state. The sensor may be embedded within or integrally connected to the device, or positioned remotely but with physical, electrical, and/or optical connection with the device so as to be able to sense a chamber within or fluid from the device. For example, the sensor may be in fluidic communication with fluid withdrawn from a subject, directly, via a microfluidic channel, an analytical chamber, etc. The sensor may be able to sense an analyte, e.g., one that is suspected of being in a fluid withdrawn from a subject. For example, a sensor may be free of any physical connection with the device, but may be positioned so as to detect the results of interaction of electromagnetic radiation, such as infrared, ultraviolet, or visible light, which has been directed toward a portion of the device, e.g., a chamber within the device. As another example, a sensor may be positioned on or within the device, and may sense activity in a chamber by being connected optically to the chamber. Sensing communication can also be provided where the chamber is in communication with a sensor fluidly, optically or visually, thermally, pneumatically, electronically, or the like, so as to be able to sense a condition of the chamber. As one example, the sensor may be positioned downstream of a chamber, within a channel such a microfluidic channel, on an external apparatus, or the like.

Thus, the invention provides, in certain embodiments, sensors able to determine an analyte. Such determination may occur within the skin, and/or externally of the subject, e.g., within a device on the surface of the skin, depending on the embodiment. "Determine," in this context, generally refers to the analysis of a species, for example, quantitatively or qualitatively, and/or the detection of the presence or absence of the species. "Determining" may also refer to the analysis of an interaction between two or more species, for example, quantitatively or qualitatively, and/or by detecting the presence or absence of the interaction, e.g. determination of the binding between two species. The species may be, for example, a bodily fluid and/or an analyte suspected of being present in the bodily fluid. "Determining" also means detecting or quantifying interaction between species.

In some embodiments, the device may be connected to an external apparatus for determining at least a portion of the device, a fluid removed from the device, an analyte suspected of being present within the fluid, or the like. For example, the device may be connected to an external analytical apparatus, and fluid removed from the device for later analysis, or the fluid may be analyzed within the *device in situ,* e.g., by adding one or more reaction entities to the device, for instance, to a storage chamber, or to analytical chamber within the device. For example, in one embodiment, the external apparatus may have a port or other suitable surface for mating with a port or other suitable surface on the device, and blood or other fluid can be removed from the device using any suitable technique, e.g., using vacuum or pressure, etc. The blood may be removed by the external apparatus, and optionally, stored and/or analyzed in some fashion. For example, in one set of embodiments, the device may include an exit port for removing a fluid from the device (e.g., blood). In some embodiments, fluid contained within a storage chamber in the device may be removed from the device, and stored for later use or analyzed outside of the device. In some cases, the exit port may be separate from the fluid transporter. For example, an exit port can be in fluidic communication with a vacuum chamber, which can also serve as a fluid reservoir in some cases. Other methods for removing blood or other fluids from the device include, but are not limited to, removal using a vacuum line, a pipette, extraction through a septum instead of an exit port, or the like. In some cases, the device may also be positioned in a centrifuge and subjected to various g forces (e.g., to a centripetal force of at least 50 g), e.g., to cause at separation of cells or other substances within a fluid within the device to occur.

In some aspects, the device may contain an indicator. The indicator may be used for determining a condition of a fluid contained within the device, e.g., within a fluid storage chamber or a fluid reservoir. In some embodiments, the indicator may indicate one or more conditions associated with the introduction of fluid into the storage component and/or one or more conditions associated with storage of fluid in the storage component. For example, the indicator may indicate the condition of blood or interstitial fluid within the device, e.g., as the device is being transported or shipped to a clinical or a laboratory setting. The indicator may indicate the condition of the blood through any suitable technique, e.g., visually (such as with a color change), using a display, by producing a sound, etc. For instance, the indicator may have a display that is green if the fluid has not been exposed to certain temperatures or if there is no adverse chemical reaction present within the fluid (e.g., a change in pH, growth of microorganisms, etc.), but is yellow or red if adverse conditions are or have been present (e.g., exposure to temperatures that are too extreme, growth of microorganisms, etc.). In other embodiments, the display may display a visual message, a sound may be produced by the device, or the like.

In some cases, the indicator may be activated upon the accessing of fluid by the access component and/or introduction of fluid into the storage component. In one set of embodiments, the indicator may be activated upon the introduction of fluid within a fluid storage reservoir, upon activation of the device (e.g., to withdraw fluid from a subject, as discussed below), upon activation by a user (e.g., by the subject, or another person), etc.

In some cases, the indicator may determine the condition of fluid within a fluid storage reservoir within the device using one or more suitable sensors, for example, pH sensors, temperature sensors (e.g., thermocouples), oxygen sensors, or the like. For instance, a sensor may be present within or proximate the fluid storage reservoir for determining the temperature of the fluid within the fluid storage reservoir. In some cases, for example, more than one sensor measurement may be taken, e.g., at multiple points of time or even continuously. In some cases, the indicator may also record the sensor determinations, e.g., for analysis or later study.

In certain embodiments, time information may be determined and/or recorded by the indicator. For example, the time fluid enters a fluid storage reservoir may be recorded, e.g., using a time/date stamp (e.g., absolute time), and/or using the duration of time that fluid has been present within the fluid storage reservoir. The time information may also be recorded in some embodiments.

As discussed, in one set of embodiments, information from sensors and/or time information may be used to determine a condition of the fluid within the fluid storage reservoir. For example, if certain limits are met or exceeded, the indicator may indicate that, as discussed above. As a specific non-limiting example, if the temperature of the device is too low (e.g., reaches 0 °C) or too high (e.g., reaches 100 °C or 37 °C), this may be displayed by a display on the indicator. Thus, fluid exposed to temperature extremes may be identified, e.g., as being problematic or spoiled. As a another non-limiting example, it may be desired to keep the pH of fluid within the device within certain conditions, and if the pH is exceeded (e.g., too acidic or too basic), this may be displayed by a display on the indicator, for example, if the pH is less than 6 or 5, or greater than 8 or 9. In some cases, the time that fluid is present within the device may be kept within certain limits as well, as another condition. For example, the indicator may indicate that fluid has been present within the device for more than about 12 hours, more than about 18 hours, or more than about 24 hours, which may indicate the fluid as being problematic, spoiled, etc.

In one set of embodiments, conditions such as these may also be combined (e.g., time and temperature). Thus, for example, fluid exposed to a first temperature may be allowed to be present within the device for a first time, while fluid exposed to a second temperature may be allowed to be present within the device for a second time, before the indicator displays this.

In some embodiments, the indicator may record and/or transmit sensor or time information. This may be recorded and/or transmitted using any suitable format. For instance, the information may be transmitted using a wireless signal, a radio signal, etc., or recorded on any suitable electronic media, e.g., on a microchip, flash drive, optically, magnetically, etc.

Thus, the invention, in certain aspects, involves the determination of a condition of a subject. Bodily fluids and/or other material associated with the skin may be analyzed, for instance, as an indication of a past, present and/or future condition of the subject, or to determine conditions that are external to the subject. Determination may occur, for instance, visually, tactilely, by odor, via instrumentation, etc. In one aspect, accordingly, the present invention is generally directed to various devices for delivering to and/or withdrawing blood, or other bodily fluids, from the skin and/or beneath the skin of a subject. Accordingly, in the description that follows, the discussion of blood is by way of example only, and in other embodiments, other fluids maybe withdrawn from the skin in addition to and/or instead of blood.

The withdrawn fluid may be any suitable bodily fluid, such as interstitial fluid, other skin-associated material, mucosal material or fluid, whole blood, perspiration, saliva, plasma, tears, lymph, urine, plasma, or any other bodily fluid, or combinations thereof. Substances withdrawn from a subject can include solid or semi-solid material such as skin, cells, or any other substance from the skin of the subject. Substances that can be delivered to a subject in accordance with some embodiments of the invention include diagnostic substances, therapeutic substances such as drugs, and the like. Various embodiments of the invention are described below in the context of delivering or withdrawing a fluid, such as blood, from the skin and/or beneath the skin. It is to be understood that in all embodiments herein, regardless of the specific exemplary language used (e.g., withdrawing blood), the devices and methods of other embodiments of the invention can be used for withdrawing any substance from the skin and/or from beneath the skin of the subject, and/or for delivering any substance to the subject, e.g. to the skin and/or a location beneath the skin of the subject.

In one set of embodiments, the device may include a sensor, for example embedded within or integrally connected to the device, or positioned remotely but with physical, electrical, and/or optical connection with the device so as to be able to sense a chamber within the device. For example, the sensor may be in fluidic communication with fluid withdrawn from a subject, directly, via a microfluidic channel, an analytical chamber, etc. The sensor may be able to sense an analyte, e.g., one that is suspected of being in a fluid withdrawn from a subject. For example, a sensor may be free of any physical connection with the device, but may be positioned so as to detect the results of interaction of electromagnetic radiation, such as infrared, ultraviolet, or visible light, which has been directed toward a portion of the device, e.g., a chamber within the device. As another example, a sensor may be positioned on or within the device, and may sense activity in a chamber by being connected optically to the chamber. Sensing communication can also be provided where the chamber is in communication with a sensor fluidly, optically or visually, thermally, pneumatically, electronically, or the like, so as to be able to sense a condition of the chamber. As one example, the sensor may be positioned downstream of a chamber, within a channel such a microfluidic channel, or the like.

The sensor may be, for example, a pH sensor, an optical sensor, an oxygen sensor, a sensor able to detect the concentration of a substance, or the like. Non-limiting examples of sensors useful in the invention include dye-based detection systems, affinity-based detection systems, microfabricated gravimetric analyzers, CCD cameras, optical detectors, optical microscopy systems, electrical systems, thermocouples and thermistors, pressure sensors, etc. Those of ordinary skill in the art will be able to identify other suitable sensors. The sensor can include a colorimetric detection system in some cases, which may be external to the device, or microfabricated into the device in certain cases. As an example of a colorimetric detection system, if a dye or a fluorescent entity is used (e.g. in a particle), the colorimetric detection system may be able to detect a change or shift in the frequency and/or intensity of the dye or fluorescent entity.

Examples of analytes that the sensor may be used to determine include, but are not limited to, pH or metal ions, proteins, nucleic acids (e.g. DNA, RNA, etc.), drugs, sugars (e.g., glucose), hormones (e.g., estradiol, estrone, progesterone, progestin, testosterone, androstenedione, etc.), carbohydrates, or other analytes of interest. Other conditions that can be determined can include pH changes, which may indicate disease, yeast infection, periodontal disease at a mucosal surface, oxygen or carbon monoxide levels which indicate lung dysfunction, and drug levels, e.g., legal prescription levels of drugs such as coumadin, other drugs such as nicotine, or illegal drugs such as cocaine. Further examples of analytes include those indicative of disease, such as cancer specific markers such as CEA and PSA, viral and bacterial antigens, and autoimmune indicators such as antibodies to double stranded DNA, indicative of Lupus. Still other conditions include exposure to elevated carbon monoxide, which could be from an external source or due to sleep apnea, too much heat (important in the case of babies whose internal temperature controls are not fully self-regulating) or from fever. Still other potentially suitable analytes include various pathogens such as bacteria or viruses, and/or markers produced by such pathogens.

Examples of sensors include, but are not limited to, pH sensors, optical sensors, ion sensors, colorimetric sensors, a sensor able to detect the concentration of a substance, or the like, e.g., as discussed herein. For instance, in one set of embodiments, the device may include an ion selective electrode. The ion selective electrode may be able to determine a specific ion and/or ions such as K⁺, H⁺, Na⁺, Ag⁺, Pb²⁺, Cd²⁺, or the like. Various ion selective electrodes can be obtained commercially. As a non-limiting example, a potassium-selective electrode may include an ion exchange resin membrane, using valinomycin, a potassium channel, as the ion carrier in the membrane to provide potassium specificity.

As additional non-limiting examples, the sensor may contain an antibody able to interact with a marker for a disease state, an enzyme such as glucose oxidase or glucose 1-dehydrogenase able to detect glucose, or the like. The analyte may be determined quantitatively or qualitatively, and/or the presence or absence of the analyte within the withdrawn fluid may be determined in some cases. Those of ordinary skill in the art will be aware of many suitable commercially-available sensors, and the specific sensor used may depend on the particular analyte being sensed. For instance, various non-limiting examples of sensor techniques include pressure or temperature measurements, spectroscopy such as infrared, absorption, fluorescence, UV/visible, FTIR ("Fourier Transform Infrared Spectroscopy"), or Raman; piezoelectric measurements; immunoassays; electrical measurements, electrochemical measurements (e.g., ion-specific electrodes); magnetic measurements, optical measurements such as optical density measurements; circular dichroism; light scattering measurements such as quasielectric light scattering; polarimetry; refractometry; chemical indicators such as dyes; or turbidity measurements, including nephelometry.

Still other potentially suitable analytes include various pathogens such as bacteria or viruses, and/or markers produced by such pathogens. Thus, in certain embodiments of the invention, as discussed herein, one or more analytes within the pooled region of fluid may be determined in some fashion, which may be useful in determining a past, present and/or future condition of the subject.

In one set of embodiments, a sensor in the device may be used to determine a condition of blood present within the device. For example, the sensor may indicate the condition of analytes commonly found within the blood, for example, O₂, K⁺, hemoglobin, Na⁺, glucose, or the like. As a specific non-limiting example, in some embodiments, the sensor may determine the degree of hemolysis within blood contained within the device. Without wishing to be bound by any theory, it is believed that in some cases, hemolysis of red blood cells may cause the release of potassium ions and/or free hemoglobin into the blood. By determining the levels of potassium ions, and/or hemoglobin (e.g., by subjecting the device and/or the blood to separate cells from plasma, then determining hemoglobin in the plasma using a suitable colorimetric assay), the amount of blood lysis or "stress" experienced by the blood contained within the device may be determined. Accordingly, in one set of embodiments, the device may indicate the usability of blood (or other fluid) contained within the device, e.g., by indicating the degree of stress or the amount of blood lysis. Other examples of devices suitable for indicating the usability of blood (or other fluid) contained within the device are also discussed herein (e.g., by indicating the amount of time blood has been contained in the device, the temperature history of the device, etc.).

In some embodiments, an analyte may be determined as an "on/off" or "normal/abnormal" situation. Detection of the analyte, for example, may be indicative that insulin is needed; a trip to the doctor to check cholesterol; ovulation is occurring; kidney dialysis is needed; drug levels are present (e.g., especially in the case of illegal drugs) or too high/too low (e.g., important in care of geriatrics in particular in nursing homes). As another embodiment, however, an analyte may be determined quantitatively.

In one set of embodiments, the sensor may be a test strip, for example, test strips that can be obtained commercially. Examples of test strips include, but are not limited to, glucose test strips, urine test strips, pregnancy test strips, or the like. A test strip will typically include a band, piece, or strip of paper or other material and contain one or more regions able to determine an analyte, e.g., via binding of the analyte to a diagnostic agent or a reaction entity able to interact with and/or associate with the analyte. For example, the test strip may include various enzymes or antibodies, glucose oxidase and/or ferricyanide, or the like. The test strip may be able to determine, for example, glucose, cholesterol, creatinine, ketones, blood, protein, nitrite, pH, urobilinogen, bilirubin, leucocytes, luteinizing hormone, etc., depending on the type of test strip. The test strip may be used in any number of different ways. In some cases, a test strip may be obtained commercially and inserted into the device, e.g., before or after withdrawing blood or other fluids from a subject. At least a portion of the blood or other fluid may be exposed to the test strip to determine an analyte, e.g., in embodiments where the device uses the test strip as a sensor so that the device itself determines the analyte. In some cases, the device may be sold with a test strip pre-loaded, or a user may need to insert a test strip in a device (and optionally, withdraw and replace the test strip between uses). In certain cases, the test strip may form an integral part of the device that is not removable by a user. In some embodiments, after exposure to the blood or other fluid withdrawn from the subject, the test strip may be removed from the device and determined externally, e.g., using other apparatuses able to determine the test strip, for example, commercially-available test strip readers. As described herein, any of a variety of signaling or display methods, associated with analyses, can be provided including signaling visually, by smell, sound, feel, taste, or the like, in one set of embodiments. Signal structures or generators include, but are not limited to, displays (visual, LED, light, etc.), speakers, chemical-releasing chambers (e.g., containing a volatile chemical), mechanical devices, heaters, coolers, or the like. In some cases, the signal structure or generator may be integral with the device (e.g., integrally connected with a support structure for application to the skin of the subject, e.g., containing a fluid transporter such as one or more needles or microneedles), or the signal structure may not be integrally connected with the support structure. As used herein, a "signal structure" or a "signal generator" is any apparatus able to generate a signal that is related to a condition of a medium. For example, the medium may be a bodily fluid, such as blood or interstitial fluid.

In some embodiments, signaling methods such as these may be used to indicate the presence and/or concentration of an analyte determined by the sensor, e.g., to the subject, and/or to another entity, such as those described below. Where a visual signal is provided, it can be provided in the form of change in opaqueness, a change in intensity of color and/or opaqueness, or can be in the form of a message (e.g., numerical signal, or the like), an icon (e.g., signaling by shape or otherwise a particular medical condition), a brand, logo, or the like. For instance, in one embodiment, the device may include a display. A written message such as "take next dose," or "glucose level is high" or a numerical value might be provided, or a message such as "toxin is present." These messages, icons, logos, or the like can be provided as an electronic read-out by a component of a device and/or can be displayed as in inherent arrangement of one or more components of the device.

In some embodiments, a device is provided where the device determines a physical condition of a subject and produces a signal related to the condition that can be readily understood by the subject (e.g., by provision of a visual "OK" signal as described above) or can be designed so as not to be readily understandable by a subject. Where not readily understandable, the signal can take a variety of forms. In one form, the signal might be a series of letters or numbers that mean nothing to the subject (e.g., A1278CDQ) which would have meaning to a medical professional or the like (and/or be decodable by the same, e.g., with reference to a suitable decoder) and can be associated with a particular physiological condition. Alternatively, a signal in the form of bar code can be provided by a device such that, under a particular condition or set of conditions the bar code appears and/or disappears, or changes, and can be read by a bar code reader to communicate information about the subject or analyte. In another embodiment, the device can be designed such that an ultraviolet signal is produced, or a signal that can be read only under ultraviolet light (e.g., a simple spot or patch, or any other signal such as a series of number, letters, bar code, message, or the like that can be readily understandable or not readily understandable by a subject) can be provided. The signal may be invisible to the human eye but, upon application UV light or other excitation energy, may be readable. The signal can be easily readable or understandable by a user via visual observation, or with other sensory activity such as smell, feel, etc. In another set of embodiments equipment as described above may be needed to determine a signal provided by the device, such as equipment in a clinical setting, etc. In some cases, the device is able to transmit a signal indicative of the analyte to a receiver, e.g., as a wireless signal, a Bluetooth signal, an Internet signal, a radio signal, etc.

In some embodiments, quantitative and/or qualitative analyses can be provided by a device. That is, the device in some cases may provide analyses that allow "yes/no" tests or the like, or tests that provide information on the quantity, concentration, or level of a particular analyte or analytes. Display configurations can be provided by the invention that reflect the amount of a particular analyte present in a subject at a particular point in time, or any other variable (presence of analysis over time, type of analyte, etc.) display configurations can take a variety of forms. In one example, a dial can be provided, similar to that of a speedometer with a series of level indications (e.g., numbers around the dial) and a "needle" or other device that indicates a particular level. In other configurations, a particular area of the device (e.g., on a display) can exist that is filled in to a greater or lesser extent depending upon the presence and/or quantity of a particular analyte present, e.g., in the form of a bar graph. In another arrangement a "color wheel" can be provided where the amount of a particular analyte present can control which colors of the wheel are visible. Or, different analytes can cause different colors of a wheel or different bars of a graph to become visible or invisible in a multiple analyte analysis. Multiple-analyte quantitative analyses can be reflected in multiple color wheels, a single color wheel with different colors per analyte where the intensity of each color reflects the amount of the analyte, or, for example, a plurality of bar graphs where each bar graph is reflective of a particular analyte and the level of the bar (and/or degree to which an area is filled in with visible color or other visible feature) is reflective of the amount of the analyte. As with all embodiments here, whatever signal is displayed can be understandable or not understandable to any number of participants. For example, it can be understandable to a subject or not understandable to a subject. Where not understandable it might need to be decoded, read electronically, or the like. Where read electronically, for example, a device may provide a signal that is not understandable to a subject or not even visible or otherwise able to be sensed by a subject, and a reader can be provided adjacent or approximate the device that can provide a visible signal that is understandable or not understandable to the subject, or can transmit a signal to another entity for analysis.

The display may also be used to display other information, in addition or instead of the above. For example, the device may include one or more displays that indicate when the device has been used or has been expired, that indicate that sampling of fluid from a subject is ongoing and/or complete, or that a problem has occurred with sampling (e.g., clogging or insufficient fluid collected), that indicate that analysis of an analyte within the collected sample is ongoing and/or complete, that an adequate amount of a fluid has been delivered to the subject (or that an inadequate amount has been delivered, and/or that fluid delivery is ongoing), that the device can be removed from the skin of the subject (e.g., upon completion of delivery and/or withdrawal of a fluid, and/or upon suitable analysis, transmission, etc.), or the like.

In connection with any signals associated with any analyses described herein, another, potentially related signal or other display (or smell, taste, or the like) can be provided which can assist in interpreting and/or evaluating the signal. In one arrangement, a calibration or control is provided proximate (or otherwise easily comparable with) a signal, e.g., a visual calibration/control or comparator next to or close to a visual signal provided by a device and/or implanted agents, particles, or the like.

A visual control or reference can be used with another sensory signal, such as that of smell, taste, temperature, itch, etc. A reference/control and/or experimental confirmation component can be provided, to be used in connection with an in-skin test or vice versa. References/indicators can also be used to indicate the state of life of a device, changing color or intensity and/or changing in another signaling aspect as the device changes relative to its useful life, so that a user can determine when the device should no longer be relied upon and/or removed. For certain devices, an indicator or control can be effected by adding analyte to the control (e.g., from a source outside of the source to be determine) to confirm operability of the device and/or to provide a reference against which to measure a signal of the device. For example, a device can include a button to be tapped by a user which will allow an analyte from a reservoir to transfer to an indicator region to provide a signal, to demonstrate operability of the device and/or provide a comparator for analysis.

Many of the embodiments described herein involve a quantitative analysis and related signal, i.e., the ability to determine the relative amount or concentration of an analyte in a medium. This can be accomplished in a variety of ways. For example, where an agent (e.g. a binding partner attached to a nanoparticle) is used to capture and analyze an analyte, the agent can be provided in a gradient in concentration across a sensing region of the device. Or a sensing region can include a membrane or other apparatus through which analyte is required to flow or pass prior to capture and identification, and the pathway for analyte travel can vary as a function of position of display region. For example, a membrane can be provided across a sensing region, through which analyte must pass prior to interacting with a layer of binding and/or signaling agent, and the membrane may vary in thickness laterally in a direction related to "bar graph" readout. Where a small amount of analyte is present, it may pass through the thinner portion but not the thicker portion of the membrane, but where a larger amount is present, it may pass across a thicker portion. The boundary (where one exists) between a region through which analyte passes, and one through which it does not completely pass, can define the "line" of the bar graph. Other ways of achieving the same or a similar result can include varying the concentration of a scavenger or transporter of the analyte, or an intermediate reactive species (between analyte and signaling event), across a membrane or other article, gradient in porosity or selectivity of the membrane, ability to absorb or transport sample fluid, or the like. These principles, in combination with other disclosure herein, can be used to facilitate any or all of the quantitative analyses described herein.

In one aspect, a subject having a condition such as a physiological condition to be analyzed (or other user, such as medical personnel) reads and/or otherwise determines a signal from a device. For example, the device may transmit a signal indicative of a condition of the subject and/or the device. Alternatively, or in addition, a signal produced by a device can be acquired in the form of a representation (e.g. a digitized signal, or the like) and transmitted to another entity for analysis and/or action. For example, a signal can be produced by a device, e.g., based on a sensor reading of an analyte, based on fluid delivered to and/or withdrawn from the skin and/or beneath the skin, based on a condition of the device, or the like. The signal may represent any suitable data or image. For example, the signal may represent the presence and/or concentration of an analyte in fluid withdrawn from a subject, the amount of fluid withdrawn from a subject and/or delivered to the subject, the number of times the device has been used, the battery life of the device, the amount of vacuum left in the device, the cleanliness or sterility of the device, the identity of the device (e.g., where multiple devices are given unique identification numbers, to prevent counterfeiting, accidental exchange of equipment to incorrect users, etc.), or the like. For instance, in one set of embodiments, an image of the signal (e.g., a visual image or photograph) can be obtained and transmitted to a different entity (for example, a user can take a cell phone picture of a signal generated by the device and send it, via cell phone, the other entity).

The other entity that the signal is transmitted to can be a human (e.g., a clinician) or a machine. In some cases, the other entity may be able to analyze the signal and take appropriate action. In one arrangement, the other entity is a machine or processor that analyzes the signal and optionally sends a signal back to the device to give direction as to activity (e.g., a cell phone can be used to transmit an image of a signal to a processor which, under one set of conditions, transmits a signal back to the same cell phone giving direction to the user, or takes other action). Other actions can include automatic stimulation of the device or a related device to dispense a medicament or pharmaceutical, or the like. The signal to direct dispensing of a pharmaceutical can take place via the same used to transmit the signal to the entity (e.g., cell phone) or a different vehicle or pathway. Telephone transmission lines, wireless networks, Internet communication, and the like can also facilitate communication of this type.

As one specific example, a device may be a glucose monitor. A signal may be generated by the device and an image of the signal captured by a cell phone camera and then transmitted via cell phone to a clinician. The clinician may then determine that the glucose (or e.g., insulin) level is appropriate or inappropriate and send a message indicating this back to the subject via cell phone.

Information regarding the analysis can also be transmitted to the same or a different entity, or a different location simply by removing the device or a portion of the device from the skin of the subject and transferring it to a different location. For example, a device can be used in connection with a subject to analyze presence and/or amount of a particular analyte. At some point after the onset of use, the device, or a portion of the device carrying a signal or signals indicative of the analysis or analyses, can be removed and, e.g., attached to a record associated with the subject. As a specific example, a patch or other device can be worn by a subject to determine presence and/or amount of one or more analytes qualitatively, quantitatively, and/or over time. The subject can visit a clinician who can remove the patch or a portion of the patch (or other device) and attach it to a medical record associated with the subject.

A variety of materials and methods, according to certain aspects of the invention, can be used to form the device, e.g., microfluidic channels, chambers, etc. For example, various components of the invention can be formed from solid materials, in which the channels can be formed via micromachining, film deposition processes such as spin coating and chemical vapor deposition, laser fabrication, photolithographic techniques, etching methods including wet chemical or plasma processes, and the like. See, for example, Scientific American, 248:44-55, 1983 (Angell, *et al).*

In one set of embodiments, various components of the systems and devices of the invention can be formed of a polymer, for example, an elastomeric polymer such as polydimethylsiloxane ("PDMS"), polytetrafluoroethylene ("PTFE" or Teflon®), or the like. For instance, according to one embodiment, a microfluidic channel may be implemented by fabricating the fluidic system separately using PDMS or other soft lithography techniques (details of soft lithography techniques suitable for this embodiment are discussed in the references entitled "Soft Lithography," by Younan Xia and George M. Whitesides, published in the Annual Review of Material Science, 1998, Vol. 28, pages 153-184, and "Soft Lithography in Biology and Biochemistry," by George M. Whitesides, Emanuele Ostuni, Shuichi Takayama, Xingyu Jiang and Donald E. Ingber, published in the Annual Review of Biomedical Engineering, 2001, Vol. 3, pages 335-373; each of these references is incorporated herein by reference).

Other examples of potentially suitable polymers include, but are not limited to, polyethylene terephthalate ("PET"), polyacrylate, polymethacrylate, polycarbonate, polystyrene, polyethylene, polypropylene, polyvinylchloride, polytetrafluoroethylene, a fluorinated polymer, a silicone such as polydimethylsiloxane, polyvinylidene chloride, cyclic olefin copolymer ("COC"), bis-benzocyclobutene ("BCB"), a polyimide, a polyester, a fluorinated derivative of a polyimide, or the like. Another example is polyethylene terephthalate glycol ("PETG"). In PETG, the ethylene glycol group that is normally part of the PET chain is partially substituted for cyclohexane dimethanol (e.g., approximately 15-35 mol% of the ethylene groups are replaced), which may, in some cases, slow down the crystallization of the polymer when injection molded to allow better processing. Combinations, copolymers, derivatives, or blends involving polymers including those described above are also envisioned. The device may also be formed from composite materials, for example, a composite of a polymer and a semiconductor material.

In some embodiments, various components of the invention are fabricated from polymeric and/or flexible and/or elastomeric materials, and can be conveniently formed of a hardenable fluid, facilitating fabrication via molding (e.g. replica molding, injection molding, cast molding, etc.). The hardenable fluid can be essentially any fluid that can be induced to solidify, or that spontaneously solidifies, into a solid capable of containing and/or transporting fluids contemplated for use in and with the fluidic network. In one embodiment, the hardenable fluid comprises a polymeric liquid or a liquid polymeric precursor (i.e., a "prepolymer"). Suitable polymeric liquids can include, for example, thermoplastic polymers, thermoset polymers, waxes, metals, or mixtures or composites thereof heated above their melting point. As another example, a suitable polymeric liquid may include a solution of one or more polymers in a suitable solvent, which solution forms a solid polymeric material upon removal of the solvent, for example, by evaporation. Such polymeric materials, which can be solidified from, for example, a melt state or by solvent evaporation, are well known to those of ordinary skill in the art. A variety of polymeric materials, many of which are elastomeric, are suitable, and are also suitable for forming molds or mold masters, for embodiments where one or both of the mold masters is composed of an elastomeric material. A non-limiting list of examples of such polymers includes polymers of the general classes of silicone polymers, epoxy polymers, and acrylate polymers. Epoxy polymers are characterized by the presence of a three-membered cyclic ether group commonly referred to as an epoxy group, 1,2-epoxide, or oxirane. For example, diglycidyl ethers of bisphenol A can be used, in addition to compounds based on aromatic amine, triazine, and cycloaliphatic backbones. Another example includes the well-known Novolac polymers. Non-limiting examples of silicone elastomers suitable for use according to the invention include those formed from precursors including the chlorosilanes such as methylchlorosilanes, ethylchlorosilanes, phenylchlorosilanes, etc.

Silicone polymers are used in certain embodiments, for example, the silicone elastomer polydimethylsiloxane. Non-limiting examples of PDMS polymers include those sold under the trademark Sylgard by Dow Chemical Co., Midland, MI, and particularly Sylgard 182, Sylgard 184, and Sylgard 186. Silicone polymers including PDMS have several beneficial properties simplifying fabrication of the microfluidic structures of the invention. For instance, such materials are inexpensive, readily available, and can be solidified from a prepolymeric liquid via curing with heat. For example, PDMSs are typically curable by exposure of the prepolymeric liquid to temperatures of about, for example, about 65 °C to about 75 °C for exposure times of, for example, about an hour. Also, silicone polymers, such as PDMS, can be elastomeric and thus may be useful for forming very small features with relatively high aspect ratios, necessary in certain embodiments of the invention. Flexible (e.g., elastomeric) molds or masters can be advantageous in this regard.

One advantage of forming structures such as microfluidic structures of the invention from silicone polymers, such as PDMS, is the ability of such polymers to be oxidized, for example by exposure to an oxygen-containing plasma such as an air plasma, so that the oxidized structures contain, at their surface, chemical groups capable of cross-linking to other oxidized silicone polymer surfaces or to the oxidized surfaces of a variety of other polymeric and non-polymeric materials. Thus, components can be fabricated and then oxidized and essentially irreversibly sealed to other silicone polymer surfaces, or to the surfaces of other substrates reactive with the oxidized silicone polymer surfaces, without the need for separate adhesives or other sealing means. In most cases, sealing can be completed simply by contacting an oxidized silicone surface to another surface without the need to apply auxiliary pressure to form the seal. That is, the pre-oxidized silicone surface acts as a contact adhesive against suitable mating surfaces. Specifically, in addition to being irreversibly sealable to itself, oxidized silicone such as oxidized PDMS can also be sealed irreversibly to a range of oxidized materials other than itself including, for example, glass, silicon, silicon oxide, quartz, silicon nitride, polyethylene, polystyrene, glassy carbon, and epoxy polymers, which have been oxidized in a similar fashion to the PDMS surface (for example, via exposure to an oxygen-containing plasma). Oxidation and sealing methods useful in the context of the present invention, as well as overall molding techniques, are described in the art, for example, in an article entitled "Rapid Prototyping of Microfluidic Systems and Polydimethylsiloxane," Anal. Chem., 70:474-480, 1998 (Duffy *et al.),* incorporated herein by reference.

Another advantage to forming microfluidic structures of the invention (or interior, fluid-contacting surfaces) from oxidized silicone polymers is that these surfaces can be much more hydrophilic than the surfaces of typical elastomeric polymers (where a hydrophilic interior surface is desired). Such hydrophilic channel surfaces can thus be more easily filled and wetted with aqueous solutions than can structures comprised of typical, unoxidized elastomeric polymers or other hydrophobic materials.

According to various aspects, the device may be used once, or multiple times, depending on the application. For instance, obtaining samples for sensing, according to certain embodiments of the invention, can be done such that sensing can be carried out continuously, discretely, or a combination of these. For example, where a bodily fluid such as blood or interstitial fluid is accessed for determination of an analyte, fluid can be accessed discretely (i.e., as a single dose, once or multiple times), or continuously by creating a continuous flow of fluid which can be analyzed once or any number of times. Additionally, testing can be carried out once, at a single point in time, or at multiple points in time, and/or from multiple samples (e.g., at multiple locations relative to the subject).

Alternatively or in addition, testing can be carried out continuously over any number of points in time involving one or any number of locations relative to the subject or other multiple samples. As an example, one bolus or isolated sample, of fluid such as blood or interstitial fluid can be obtained. From that fluid a test can be carried out to determine whether a particular analyte or other agent exists in the fluid. Alternatively, two or more tests can be carried out involving that quantity of fluid to determine the presence and/or quantity of two or more analytes, and any number of such tests can be carried out. Tests involving that quantity of fluid can be carried out simultaneously or over a period of time. For example, a test for a particular analyte can be carried out at various points in time to determine whether the result changes over time, or different analytes can be determined at different points in time. As another example, a pool of fluid can be formed between layers of skin via, e.g., a suction blister and either within the suction blister or from fluid drawn from the suction blister and placed elsewhere, any of the above and other analysis can be carried out at one or more points in time. Where a suction blister is formed in such a way that interstitial fluid within the blister changes over time (where an equilibrium exists between interstitial fluid within the subject and interstitial fluid in the suction blister itself, i.e., the fluid within the blister is ever changing to reflect the content of the interstitial fluid of the subject in the region of the blister over time). Testing of fluid within or from the suction blister at various points in time can provide useful information.

In another example, one or more needles or microneedles, or other device(s) can be used to access a fluid of a subject such as blood or interstitial fluid (with or without use of a suction blister). Fluid can be drawn to a point of analysis and analyzed in any manner described herein. For example, an analysis can be carried out once, to determine the presence and/or quantity of a single analyte, or a number of tests can be carried out. From a single sample of fluid, a particular test or number of tests can be carried out essentially simultaneously, or analyses can be carried out over time. Moreover, fluid can be drawn continuously from the skin of the subject and one or more tests can be carried out of any number of points in time. A variety of reasons for carrying out one or more tests over the course of time exists, as would be understood by those of ordinary skill in the art. One such reason is to determine whether the quantity or another characteristic of an analyte is constant in a subject, or changes over time. A variety of specific techniques for continuous and/or discrete testing will be described herein.

In some embodiments, the device may include a signal structure and a support structure. The support structure may be used, for example, for applying the fluid transporter to the surface of the skin of the subject, e.g., so that fluid may be delivered to and/or withdrawn from the skin and/or beneath the skin of the subject. The signal structure may be used to indicate a state or condition of the device, e.g., of a condition of the device, and/or a condition of a fluid delivered or removed from the skin and/or beneath the skin of the subject. For instance, the signal structure may indicate analysis of an analyte contained within a fluid removed from the skin and/or beneath the skin of the subject. As discussed, the signal structure may be able to produce a signal visually (e.g., using a display, lights, etc.), by smell, sound, feel, taste, or the like.

In some cases, the signal structure may be integrally connected to the support structure. As used herein, the term "integrally connected," when referring to two or more objects, means objects that do not become separated from each other during the course of normal use, e.g., cannot be separated manually; separation requires at least the use of tools, and/or by causing damage to at least one of the components, for example, by breaking, peeling, etc. (separating components fastened together via adhesives, tools, etc.). For example, the device may be a one-use disposable item, or the device may be used multiple times.

In another set of embodiments, however, the signal structure may not be integrally connected to the support structure. Thus, the signal structure and the support structure may be separated from each other, in various embodiments. Separation may be performed, for example, by a user, or the separation may be automatically driven in some embodiments (e.g., a servo mechanism may cause one of the structures to become ejected or disconnected with the other, for example, similar to how a VCR ejects a tape).

In certain embodiments, the support structure and the signal structure are constructed and arranged to be connectable and/or detachable from each other readily by the subject. Thus, for instance, the subject (or another person) may be able to connect the support structure and the signal structure to assemble a device, and/or disconnect the support structure and the signal structure, without the use of tools such as screwdrivers or tape. In some cases, the connection and/or disconnection can occur while the device is affixed to the skin. Thus, for example, a device may be applied to the subject of the skin, and after use, one of the support structure and the signal structure may be removed from the skin of the subject, leaving the remainder of the device in place on the skin.

As an example, in one embodiment, a device may be fabricated to contain a first module that contains a support structure, and a second module containing the signal structure that is constructed and arranged for repeated connection and disconnection to the first module containing the support structure. The first module containing the support structure may be used to deliver to and/or withdraw fluid from a subject. For instance, as discussed herein, the first module may contain a fluid transporter associated with the support structure for delivering to and/or withdrawing fluid from the skin and/or beneath the skin of the subject. The fluid may optionally be analyzed within the first module, and/or stored for later use, e.g., in a collection chamber. After withdrawal of sufficient fluid, the first module may be removed, leaving the second module in place, and optionally replaced with a new first module for subsequent use (e.g., for subsequent delivery and/or withdrawal of fluid at a later time). In other embodiments, however, the second module may be removed, leaving the first module in place. Depending on the application, the removed module may be reused or disposed of (e.g., thrown in the trash), or the module may be shipped to another location for disposal and/or analysis, for example, to analyze fluid contained within the module, e.g., withdrawn from the skin of the subject. A module may be used once, or multiple times, before being removed from the device, depending on the application.

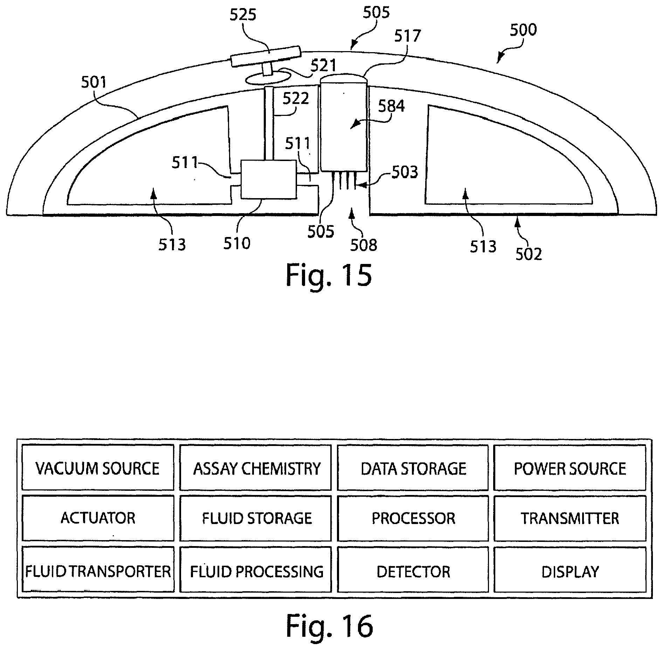

In some aspects of the invention, any of the following components may independently be modular and may be single-use or reusable, or even absent in some cases: a pressure regulator such as a vacuum chamber or other vacuum source, an actuator, an activator, a fluid transporter, a fluid assay, a sensor, a fluid storage (e.g., a collection chamber), a data storage or memory component, a processor, a detector, a power source, a transmitter, a display, or the like. This is shown schematically in Fig. 16 as a specific non-limiting example, where each item in this figure, alone or together with other items, may be single-use, reusable, or absent in some cases. As non-limiting examples, the left half of the figure (fluid transporter, actuator, vacuum source, fluid processing, fluid storage, and assay chemistry) could be a single use module, while the right half (detector, processor, data storage, display, transmitter, and power source) could be a re-useable module. Alternatively, just a single unit (e.g., a fluid transporter, e.g., one or more needles or microneedles) might be single use, and the rest of the device might be re-useable. Other combinations of these are also contemplated, where each item in Fig. 16, alone or together with other items, may be single-use, reusable, or absent. In certain embodiments, the replaceable portion within the device is one that is required for the device to function, for example the device may not be able to function to deliver and/or withdraw fluid without the replaceable portion being present within the device. In one embodiment, the replaceable portion is not a power source (e.g., a battery).

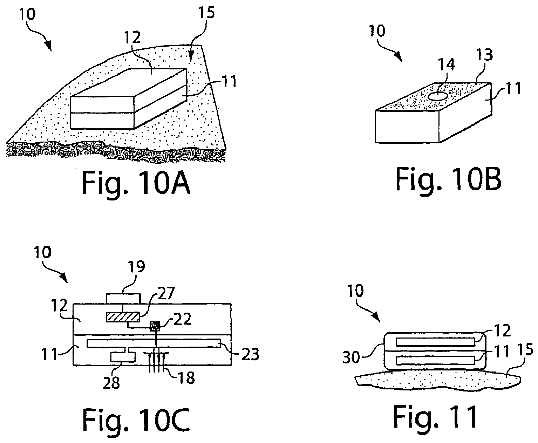

One non-limiting example of such a device is illustrated with reference to Fig. 10. In Fig. 10A, device 10 includes modules 11 and 12. When the device is applied to the skin, module 11 comes in contact with skin 15 while module 12 sits on module 11 and does not come into contact with the skin. In other arrangements, however, the modules may have different configurations; for example, both modules may come into contact with the skin. In Fig. 10B, the underside of module 11 is shown. In this particular example, module 11 contains a surface 13 and a recess 14. Surface 13 may be generally flat, while recess 14 may contain transport means for delivering and/or withdrawing fluid. For example, module 11 may contain one or more needles, microneedles, etc., as discussed herein for delivery and/or withdrawal of fluid. Device 10 may be held onto the surface of the skin, in one set of embodiments, using adhesives, or mechanical elements such as straps, belts, buckles, strings, ties, elastic bands, or the like. For instance, as is shown in Fig. 10B, surface 13 may contain an adhesive that, when pressed against the surface of the skin, forms sufficient adhesion that the device is able to stay on the skin.

In this example, module 11 contains a self-contained vacuum chamber, as is shown in Fig. 10C. In this figure, module 11 contains vacuum chamber 23, and fluid transporter 18, e.g., one or more microneedles. When applied to the skin and activated, the device may cause the fluid transporter to pierce the surface of the skin, and self-contained vacuum chamber 23 may be pierced in some fashion to create a fluidic (vacuum) connection between the vacuum chamber and the fluid transporter. For example, an actuator 22 may be used to move the fluid transporter 18 down and/or up. The force of vacuum may be sufficient to draw a fluid through the fluid transporter into device 11, e.g., into storage chamber 28, which may be in fluid communication with the vacuum chamber and the fluid transporter. After fluid transport, the fluid transporter may optionally be removed from the skin, e.g., withdrawn back into module 11, e.g., by using actuator 22 (for instance, a servo motor).

Module 12 may contain elements such as control elements, sensors, actuators, displays, signaling elements, activators, or the like. For example, as is shown in Fig. 10C, module 12 may contain an activator 19, e.g., a button, switch, lever, slider, dial, etc., in electrical communication with a computer circuit 27, e.g., a semiconductor chip. Upon activation, e.g., by the subject or another person, the computer circuit may cause activation of the fluid transporter in module 11, and/or piercing of vacuum chamber 23. However, after fluid has been withdrawn from the skin into storage chamber 28, modules 11 and 12 may be disconnected from each other. Module 11, in turn, may be stored or shipped for later analysis, e.g., at a secondary site. In some cases, module 11 may be replaced with a new module 11 (which may be the same or different than original module 11), e.g., for subsequent use by the subject.

Module 11 and module 12 may be connected together using any suitable technique. For example, module 11 and module 12 may snap together, or be contained together within a housing. For instance, buttons, interlocks, straps, or other mechanical elements may be used to connect modules 11 and 12.

In the example of Fig. 11, device 10 is shown containing housing 30 containing modules 11 and 12, attached to the surface of the skin 15 of a subject. In this example, either of modules 11 and 12 can be removed from housing 30 without necessarily removing the other module from housing 30. Thus, for example, housing 30 may be applied to the surface of the skin of a subject. Device 10 may be held onto the surface of the skin, in one set of embodiments, using adhesives, mechanical elements such as straps, belts, buckles, strings, ties, elastic bands, or the like, and while affixed on the surface of the skin, one or both modules may be removed and/or replaced for various uses or applications.

In one set of embodiments, the device is reusable. For instance, the device may be used repeatedly (at the same location on the skin of a subject, or at different locations) to deliver to and/or withdraw fluid from the skin and/or beneath the skin of the subject. The device used repeatedly may be a single, integral device, and/or the device may contain one or more modules such as those previously discussed. For example, in some cases, between uses, a module may be removed and/or replaced from the device, e.g., a support module or a signal module, as discussed above.

In another aspect, the device may include an anticoagulant or a stabilizing agent for stabilizing the fluid withdrawn from the skin. The device may be a single, unitary device, or the device may contain one or more modules, e.g., modules 11 and 12 in Fig. 10. The anticoagulant may be located in a vacuum chamber and/or a collection chamber, depending on the application. For example, the fluid may be stored within the device for a certain period of time, and/or the device (or a portion thereof) may be shipped to another location for analysis or later use. For instance, a device may contain anticoagulant or a stabilizing agent in a storage chamber (e.g., storage chamber 28 in Fig. 10C).

As a specific non-limiting example, an anticoagulant may be used for blood withdrawn from the skin. Examples of anticoagulants include, but are not limited to, heparin, citrate, thrombin, oxalate, ethylenediaminetetraacetic acid (EDTA), sodium polyanethol sulfonate, or acid citrate dextrose. Other agents may be used in conjunction or instead of anticoagulants, for example, stabilizing agents such as solvents, diluents, buffers, chelating agents, antioxidants, binding agents, preservatives, antimicrobials, or the like. Examples of preservatives include, for example, benzalkonium chloride, chlorobutanol, parabens, or thimerosal. Non-limiting examples of antioxidants include ascorbic acid, glutathione, lipoic acid, uric acid, carotenes, alpha-tocopherol, ubiquinol, or enzymes such as catalase, superoxide dismutase, or peroxidases. Examples of microbials include, but are not limited to, ethanol or isopropyl alcohol, azides, or the like. Examples of chelating agents include, but are not limited to, ethylene glycol tetraacetic acid or ethylenediaminetetraacetic acid. Examples of buffers include phosphate buffers such as those known to ordinary skill in the art.

Further examples of anticoagulants include, but are not limited to, heparin, citrate, oxalate, or ethylenediaminetetraacetic acid (EDTA). Other agents may be used in conjunction or instead of anticoagulants, for example, stabilizing agents such as solvents, diluents, buffers, chelating agents, antioxidants, binding agents, preservatives, antimicrobials, or the like. Examples of preservatives include, for example, benzalkonium chloride, chlorobutanol, parabens, or thimerosal. Non-limiting examples of antioxidants include ascorbic acid, glutathione, lipoic acid, uric acid, carotenes, alpha-tocopherol, ubiquinol, or enzymes such as catalase, superoxide dismutase, or peroxidases. Examples of microbials include, but are not limited to, ethanol or isopropyl alcohol, azides, or the like. Examples of chelating agents include, but are not limited to, ethylene glycol tetraacetic acid or ethylenediaminetetraacetic acid. Examples of buffers include phosphate buffers such as those known to ordinary skill in the art.

As yet another example, the device may include a therapeutic agent such as an anti-inflammatory compound, an analgesic, or an anti-histamine compound. Examples of anti-inflammatory compounds include, but are not limited to, NSAIDs (non-steroidal anti-inflammatory drugs) such as aspirin, ibuprofen, or naproxen. Examples of analgesics include, but are not limited to, benzocaine, butamben, dibucaine, lidocaine, oxybuprocaine, pramoxine, proparacaine, proxymetacaine, tetracaine, acetaminophen, NSAIDs such as acetylsalicylic acid, salicylic acid, diclofenac, ibuprofen, etc., or opioid drugs such as morphine or opium, etc. Examples of anti-histamine compounds include, but are not limited to, clemastine, diphenhydramine, doxylamine, loratadine, desloratadine, fexofenadine, pheniramine, cetirizine, ebastine, promethazine, chlorpheniramine, levocetirizine, olopatadine, quetiapine, meclizine, dimenhydrinate, embramine, dimethindene, dexchlorpheniramine, vitamin C, cimetidine, famotidine, ranitidine, nizatidine, roxatidine, or lafutidine. Other specific non-limiting examples of therapeutic agents that could be used include, but are not limited to biological agents such as erythropoietin ("EPO"), alpha-interferon, beta-interferon, gamma-interferon, insulin, morphine or other pain medications, antibodies such as monoclonal antibodies, or the like.

In one set of embodiments, a device of the invention as discussed herein may be shipped or transported to another location for analysis. For example, the device may be hand-carried, mailed, etc. In some cases, the device may include an anticoagulant or a stabilizing agent contained within the device, e.g., within a storage chamber for the fluid. Thus, for example, fluid such as blood withdrawn from the skin may be delivered to a chamber (e.g., a storage chamber) within the device, then the device, or a portion of the device (e.g., a module) may be shipped to another location for analysis. Any form of shipping or transport may be used, e.g., via mail or hand-delivery.

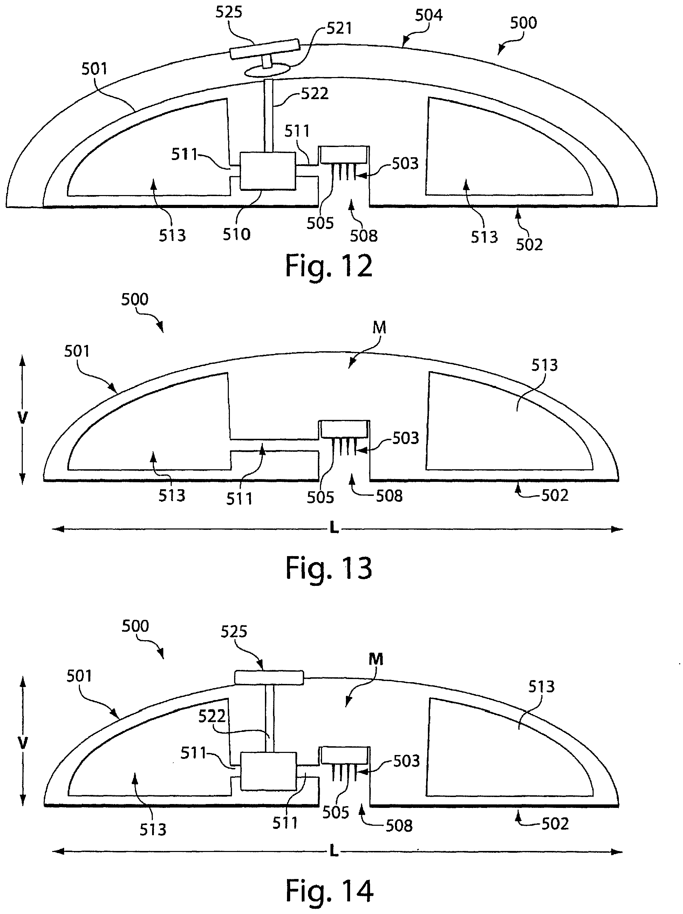

Further examples of various embodiments of the invention are illustrated in Figs. 12-15. In Fig. 12, device 500 is illustrated. In this example, device 500 includes a support structure 501, an adhesive 502 for adhesion of the device to the skin, a signal structure 504, and a fluid transporter system 503. In this figure, fluid transporter system 503 includes a plurality of microneedles 505, although other fluid transporters as discussed herein may also be used. Also shown in Fig. 12 is sensor 510, connected via channels 511 to recess 508 containing microneedles 505. Chamber 513, in this figure, is a self-contained vacuum chamber. Vacuum chamber 513 is in fluidic communication with recess 508 via channel 511, for example, as controlled by a controller or an actuator (not shown). In this figure, device 500 also contains display 525, which is connected to sensor 510 via electrical connection 522 and interface 521 between signal structure 504 and support structure 501. In some cases, signal structure 504 and support structure 510 may be connectable and/or detachable from each other.

In Fig. 13, device 500 includes an adhesive 502 for adhesion of the device to the skin, support structure 501, and a fluid transporter system 503, including a plurality of microneedles 505 contained in recess 508, although other fluid transporters as discussed herein may also be used. Vacuum chamber 513 is in fluidic communication with recess 508 via channel 511, for example, as controlled by a controller or an actuator (not shown). Device 500 in this figure has a largest lateral dimension L, and a largest vertical dimension, extending from the skin of the subject when the device is applied to the subject, V, as well as a mass M. A similar device is shown in Fig. 14, containing additional elements, e.g., display 525, electrical connection 522, and sensor 510, as previously discussed.

Yet another example embodiment is illustrated in Fig. 15. In this figure, device 500 includes support structure 501 and signal structure 505. In some cases, signal structure 505 and support structure 510 may be connectable and/or detachable from each other. Also shown in Fig. 15 is an adhesive 502 for adhesion of the device to the skin, and an extraction activator 584, for example including actuator 517. When actuated, actuator 517 is able to drive component 518 (e.g., a piston, a screw, a mechanical linkage, etc.) downward, moving microneedles 505 down towards the skin when the device is placed on the skin of a subject, and in some cases, actuator 517 may also be able to withdraw the microneedles from the skin after use, e.g., after a fluid is delivered to and/or withdrawn from the skin and/or beneath the skin. Also illustrated in Fig. 15 is sensor 510, connected via channels 511 to recess 508 containing microneedles 505. Device 500 also contains vacuum chamber 513, which may be self-contained, and is in fluidic communication with recess 508 via channel 511, for example, as controlled by a controller or an actuator (not shown). In this figure, device 500 also contains display 525, which is connected to sensor 510 via electrical connection 522 and interface 521 between signal structure 505 and support structure 501.

In one set of embodiments, at least a portion of the device may be colored to indicate the anticoagulant(s) contained within the device. In some cases, the colors used may be identical or equivalent to that commercially used for Vacutainers™, Vacuettes™, or other commercially-available phlebotomy equipment. For example, lavender and/or purple may indicate ethylenediaminetetraacetic acid, light blue may indicate citrate, dark blue may indicate ethylenediaminetetraacetic acid, green may indicate heparin, gray may indicate a fluoride and/or an oxalate, orange may indicate a thrombin, yellow may indicate sodium polyanethol sulfonate and/or acid citrate dextrose, black may indicate citrate, brown may indicate heparin, etc. In other embodiments, however, other coloring systems may be used.

Other coloring systems may be used in other embodiments of the invention, not necessarily indicative of anti-coagulants. For example, in one set of embodiments, the device carries a color indicative of a recommended bodily use site for the device, e.g., a first color indicative of a device suitable for placement on the back, a second color indicative of a device suitable for placement on a leg, a third color indicative of a device suitable for placement on the arm, etc.

After withdrawal of the fluid into the device, the device, or a portion thereof, may be removed from the skin of the subject, e.g., by the subject or by another person. For example, the entire device may be removed, or a portion of the device containing the storage reservoir may be removed from the device, and optionally replaced with another storage reservoir. Thus, for instance, in one embodiment, the device may contain two or more modules, for example, a first module that is able to cause withdrawal of fluid from the skin into a storage reservoir, and a second module containing the storage module. In some cases, the module containing the storage reservoir may be removed from the device. Other examples of modules and modular systems are discussed below; other examples are discussed in U.S. Provisional Patent Application Serial No. 61/256,931, filed October 30, 2009, entitled "Modular Systems for Application to the Skin," incorporated by reference herein in its entirety.

As another example, the device may include at least two modules manually separable from each other, including a first module comprising a vacuum chamber, and a second module comprising other components such as those described herein. In some embodiments, the modules may be separable without the use of tools. For example, the second module may include one or more components such as a fluid transporter (e.g., a needle or microneedle), an applicator region such as a recess, a reversibly deformable structure such as a flexible concave member, a collection chamber, a sensor, a processor, or the like. As a specific example, the first module may be defined entirely or partially by a vacuum chamber, and the first module may be removed and replaced with a fresh vacuum chamber, during or between uses. Thus, for instance, the first module may be inserted into the device when blood or other bodily fluids are desired to be withdrawn from a subject, and optionally, used to cause blood to be withdrawn from the skin of the subject.

In one set of embodiments, the first module may be substantially cylindrical, and in some embodiments, the first module may be a Vacutainer™ tube, a Vacuette™ tube, or other commercially-available vacuum tube, or other vacuum source such as is described herein. In some embodiments, a Vacutainer™ or Vacuette™ tube that is used may have a maximum length of no more than about 75 mm or about 100 mm and a diameter of no more than about 16 mm or about 13 mm. The device, in certain embodiments, may also contain an adaptor able to hold or immobilize such tubes on the device, for example, a clamp. Other examples of adaptors are discussed in detail herein. In some cases, the device may have a shape or geometry that mimics a Vacutainer™ or Vacuette™ tube, e.g., one having the above dimensions. The device, in some embodiments, is substantially cylindrically symmetric.

The withdrawn fluid may then be sent to a clinical and/or laboratory setting, e.g., for analysis. In some embodiments, the entire device may be sent to the clinical and/or laboratory setting; in other embodiments, however, only a portion of the device (e.g., a module containing a storage reservoir containing the fluid) may be sent to the clinical and/or laboratory setting. In some cases, the fluid may be shipped using any suitable technique (e.g., by mail, by hand, etc.). In certain instances, the subject may give the fluid to appropriate personnel at a clinical visit. For instance, a doctor may prescribe a device as discussed above for use by the subject, and at the next doctor visit, the subject may give the doctor the withdrawn fluid, e.g., contained within a device or module.

One aspect of the present invention is directed to an adaptor able to position a device of the invention in apparatuses designed to contain Vacutainer™ tubes or Vacuette™ tubes. In some cases, the Vacutainer™ or Vacuette™ tube sizes have a maximum length of no more than about 75 mm or about 100 mm and a diameter of no more than about 16 mm or about 13 mm. In some cases, the adaptor may be able to immobilize a device of the invention therein, e.g., for subsequent use or processing. In some cases, as previously discussed, devices of the invention may have a largest lateral dimension of no more than about 50 mm, and/or a largest vertical dimension, extending from the skin of the subject when the device is applied to the subject, of no more than about 10 mm. The device may contained within the adaptor using any suitable technique, e.g., using clips, springs, braces, bands, or the application of force to the device present within the adaptor.

According to one aspect of the invention, the device is of a relatively small size. In some embodiments, the device may be sized such that it is wearable and/or able to be carried by a subject. For example, the device may be self-contained, needing no wires, cables, tubes, external structural elements, or other external support. The device may be positioned on any suitable position of the subject, for example, on the arm or leg, on the back, on the abdomen, etc. As mentioned, in some embodiments, the device may be affixed or held onto the surface of the skin using any suitable technique, e.g., using adhesives, mechanical elements such as straps, belts, buckles, strings, ties, elastic bands, or the like. In some cases, the device may be positioned on the subject such that the subject is able to move around (e.g., walking, exercising, typing, writing, drinking or eating, using the bathroom, etc.) while wearing the device. For example, the device may have a mass and/or dimensions such that the subject is able to wear the device for at least about 5 minutes, and in some cases for longer periods of time, e.g., at least about 10 minutes, at least about 15 minutes, at least about 30 minutes, at least about 45 minutes, at least about 1 hour, at least about 3 hours, at least 5 hours, at least about 8 hours, at least about 1 day, at least about 2 days, at least about 4 days, at least about 1 week, at least about 2 weeks, at least about 4 weeks, etc. In other embodiments, however, the device may be used or worn for relatively short periods of time, for example, no more than about 1 hour, no more than about 30 min, no more than about 15 min, no more than about 10 min, or no more than about 5 min.

In some embodiments, the device is relatively lightweight. For example, the device may have a mass of no more than about 1 kg, no more than about 300 g, no more than about 150 g, no more than about 100 g, no more than about 50 g, no more than about 30 g, no more than about 25 g, no more than about 20 g, no more than about 10 g, no more than about 5 g, or no more than about 2 g. For instance, in various embodiments, the device has a mass of between about 2 g and about 25 g, a mass of between about 2 g and about 10 g, a mass of between 10 g and about 50 g, a mass of between about 30 g and about 150 g, etc.

The device, in some cases, may be relatively small. For example, the device may be constructed and arranged to lie relatively close to the skin. Thus, for instance, the device may have a largest vertical dimension, extending from the skin of the subject when the device is positioned on the skin, of no more than about 25 cm, no more than about 10 cm, no more than about 7 cm, no more than about 6 cm, no more than about 5 cm, no more than about 3 cm, no more than about 2.5 cm, no more than about 2 cm, no more than about 1.5 cm, or no more than about 1 cm, no more than about 8 mm, no more than about 5 mm, no more than about 3 mm, no more than about 2 mm, no more than about 1 mm, or no more than about 0.5 mm. In some cases, the device may have a largest vertical dimension of between about 0.5 cm and about 1 cm, between about 2 and about 3 cm, between about 2.5 cm and about 5 cm, between about 2 cm and about 7 cm, between about 0.5 mm and about 7 cm, etc.

In another set of embodiments, the device may have a relatively small size. For example, the device may have a largest lateral dimension (e.g., parallel to the skin) of no more than about 25 cm, no more than about 10 cm, no more than about 7 cm, no more than about 6 cm, no more than about 5.5 cm, no more than about 5 cm, no more than about 4.5 cm, no more than about 4 cm, no more than about 3.5 cm, no more than about 3 cm, no more than about 2 cm, or no more than about 1 cm. In some cases, the device may have a largest lateral dimension of between about 0.5 cm and about 1 cm, between about 2 and about 3 cm, between about 2.5 cm and about 5 cm, between about 2 cm and about 7 cm, etc.

Combinations of these and/or other dimensions are also possible in other embodiments. As non-limiting examples, the device may have a largest lateral dimension of no more than about 5 cm, a largest vertical dimension of no more than about 1 cm, and a mass of no more than about 25 g; or the device may have a largest lateral dimension of no more than about 5 cm, a largest vertical dimension of no more than about 1 cm, and a mass of no more than about 25 g; etc. As additional non-limiting examples, the device may have dimensions of no more than 2.0 cm x 3.1 cm x 5.7 cm (height x width x length), no more than 2.5 cm x 3.5 cm x 6.0 cm, no more than about 1.5 cm x 4.2 cm x 4.7 cm, no more than 2.0 cm x 4.5 cm x 5.0 cm, etc.

In some aspects, one or more materials, such as particles are delivered to or through the skin. Examples of suitable materials include, but are not limited to, particles such as microparticles or nanoparticles, a chemical, a drug or a therapeutic agent, a diagnostic agent, a carrier, or the like. The particles may be, for example, nanoparticles or microparticles, and in some cases, the particles may be anisotropic particles. In some cases, a plurality of particles may be used, and in some cases, some, or substantially all, of the particles maybe the same. For example, at least about 10%, at least about 30%, at least about 40%, at least about 50%, at least about 60%, at least about 70%, at least about 80%, at least about 90%, at least about 95%, or at least about 99% of the particles may have the same shape, and/or may have the same composition.

The particles may be used for a variety of purposes. For instance, the particles may contain a diagnostic agent or a reaction entity able to interact with and/or associate with an analyte, or another reaction entity, or other particles. Such particles may be useful, for example, to determine one or more analytes, such as a marker of a disease state, as discussed below. As another example, the particles may contain a drug or a therapeutic agent, positioned on the surface and/or internally of the particles, which may be released by the particles and delivered to the subject. Specific examples of these and other embodiments are discussed in detail below.

In some cases, materials such as particles may become embedded within the skin, for example, due to physical properties of the materials (e.g., size, hydrophobicity, etc.). Thus, in some cases, a depot of material may be formed within the skin, and the depot may be temporary or permanent. For instance, materials within the depot may eventually degrade (e.g., if the material biodegradable), enter the bloodstream, or be sloughed off to the environment, e.g., as the cells of the dermis differentiate to form new epidermis and accordingly push the material towards the surface of the skin. Thus, the depot of material may be present within the subject on a temporary basis (e.g., on a time scale of days or weeks), in certain instances.

As mentioned, certain aspects of the present invention are generally directed to particles such as anisotropic particles or colloids, which can be used in a wide variety of applications. For instance, the particles may be present within the skin, or externally of the skin, e.g., in a device on the surface of the skin. The particles may include microparticles and/or nanoparticles. As discussed above, a "microparticle" is a particle having an average diameter on the order of micrometers (i.e., between about 1 micrometer and about 1 mm), while a "nanoparticle" is a particle having an average diameter on the order of nanometers (i.e., between about 1 nm and about 1 micrometer. The particles may be spherical or non-spherical, in some cases. For example, the particles may be oblong or elongated, or have other shapes such as those disclosed in U.S. Patent Application Serial No. 11/851,974, filed September 7, 2007, entitled "Engineering Shape of Polymeric Micro- and Nanoparticles," by S. Mitragotri, *et al.;* International Patent Application No. PCT/US2007/077889, filed September 7, 2007, entitled "Engineering Shape of Polymeric Micro- and Nanoparticles," by S. Mitragotri, *et al.,* published as WO 2008/031035 on March 13, 2008; U.S. Patent Application Serial No. 11/272,194, filed November 10, 2005, entitled "Multi-phasic Nanoparticles," by J. Lahann, *et al.,* published as U.S. Patent Application Publication No. 2006/0201390 on September 14, 2006; or U.S. Patent Application Serial No. 11/763,842, filed June 15, 2007, entitled "Multi-Phasic Bioadhesive Nano-Objects as Biofunctional Elements in Drug Delivery Systems," by J. Lahann, published as U.S. Patent Application Publication No. 2007/0237800 on October 11, 2007, each of which is incorporated herein by reference. Other examples of particles can be seen in U.S. Patent Application Serial No. 11/272,194, filed November 10, 2005, entitled "Multi-phasic Nanoparticles," by J. Lahann, *et al.,* published as U.S. Patent Application Publication No. 2006/0201390 on September 14, 2006; U.S. Patent Application Serial No. 11/763,842, filed June 15, 2007, entitled "Multi-Phasic Bioadhesive Nano-Objects as Biofunctional Elements in Drug Delivery Systems," by J. Lahann, published as U.S. Patent Application Publication No. 2007/0237800 on October 11, 2007; or U.S. Provisional Patent Application Serial No. 61/058,796, filed June 4, 2008, entitled "Compositions and Methods for Diagnostics, Therapies, and Other Applications," by D. Levinson, each of which is incorporated herein by reference.

The particles may be formed of any suitable material, depending on the application. For example, the particles may comprise a glass, and/or a polymer such as polyethylene, polystyrene, silicone, polyfluoroethylene, polyacrylic acid, a polyamide (e.g., nylon), polycarbonate, polysulfone, polyurethane, polybutadiene, polybutylene, polyethersulfone, polyetherimide, polyphenylene oxide, polymethylpentene, polyvinylchloride, polyvinylidene chloride, polyphthalamide, polyphenylene sulfide, polyester, polyetheretherketone, polyimide, polymethylmethacylate and/or polypropylene. In some cases, the particles may comprise a ceramic such as tricalcium phosphate, hydroxyapatite, fluorapatite, aluminum oxide, or zirconium oxide. In some cases (for example, in certain biological applications), the particles may be formed from biocompatible and/or biodegradable polymers such as polylactic and/or polyglycolic acids, polyanhydride, polycaprolactone, polyethylene oxide, polyacrylamide, polyacrylic acid, polybutylene terephthalate, starch, cellulose, chitosan, and/or combinations of these. In one set of embodiments, the particles may comprise a hydrogel, such as agarose, collagen, or fibrin. The particles may include a magnetically susceptible material in some cases, e.g., a material displaying paramagnetism or ferromagnetism. For instance, the particles may include iron, iron oxide, magnetite, hematite, or some other compound containing iron, or the like. In another embodiment, the particles can include a conductive material (e.g., a metal such as titanium, copper, platinum, silver, gold, tantalum, palladium, rhodium, etc.), or a semiconductive material (e.g., silicon, germanium, CdSe, CdS, etc.). Other particles potentially useful in the practice of the invention include ZnS, ZnO, TiO₂ AgI, AgBr, HgI₂, PbS, PbSe, ZnTe, CdTe, In₂S₃, In₂Se₃, Cd₃P₂, Cd₃As₂, InAs, or GaAs. The particles may include other species as well, such as cells, biochemical species such as nucleic acids (e.g., RNA, DNA, PNA, etc.), proteins, peptides, enzymes, nanoparticles, quantum dots, fragrances, indicators, dyes, fluorescent species, chemicals, small molecules (e.g., having a molecular weight of less than about 1 kDa), or the like.

The particles may also have any shape or size. For instance, the particles may have an average diameter of less than about 5 mm or 2 mm, or less than about 1 mm, or less than about 500 microns, less than about 200 microns, less than about 100 microns, less than about 60 microns, less than about 50 microns, less than about 40 microns, less than about 30 microns, less than about 25 microns, less than about 10 microns, less than about 3 microns, less than about 1 micron, less than about 300 nm, less than about 100 nm, less than about 30 nm, or less than about 10 nm. As discussed, the particles may be spherical or non-spherical. The average diameter of a non-spherical particle is the diameter of a perfect sphere having the same volume as the non-spherical particle. If the particle is non-spherical, the particle may have a shape of, for instance, an ellipsoid, a cube, a fiber, a tube, a rod, or an irregular shape. In some cases, the particles may be hollow or porous. Other shapes are also possible, for instance, core/shell structures (e.g., having different compositions), rectangular disks, high aspect ratio rectangular disks, high aspect ratio rods, worms, oblate ellipses, prolate ellipses, elliptical disks, UFOs, circular disks, barrels, bullets, pills, pulleys, biconvex lenses, ribbons, ravioli, flat pills, bicones, diamond disks, emarginate disks, elongated hexagonal disks, tacos, wrinkled prolate ellipsoids, wrinkled oblate ellipsoids, porous ellipsoid disks, and the like. See, e.g., International Patent Application No. PCT/US2007/077889, filed September 7, 2007, entitled "Engineering Shape of Polymeric Micro- and Nanoparticles," by S. Mitragotri, *et al.,* published as WO 2008/031035 on March 13, 2008, incorporated herein by reference.

In one aspect of the invention, a particle may include one or more reaction entities present on the surface (or at least a portion of the surface) of the particle. The reaction entity may be any entity able to interact with and/or associate with an analyte, or another reaction entity. For instance, the reaction entity may be a binding partner able to bind an analyte. For example, the reaction entity may be a molecule that can undergo binding with a particular analyte. The reaction entities may be used, for example, to determine pH or metal ions, proteins, nucleic acids (e.g. DNA, RNA, etc.), drugs, sugars (e.g., glucose), hormones (e.g., estradiol, estrone, progesterone, progestin, testosterone, androstenedione, etc.), carbohydrates, or other analytes of interest.

The term "binding partner" refers to a molecule that can undergo binding with a particular molecule, e.g., an analyte. For example, the binding may be highly specific and/or non-covalent. Binding partners which form highly specific, non-covalent, physiochemical interactions with one another are defined herein as "complementary." Biological binding partners are examples. For example, Protein A is a binding partner of the biological molecule IgG, and vice versa. Other non-limiting examples include nucleic acid-nucleic acid binding, nucleic acid-protein binding, protein-protein binding, enzyme-substrate binding, receptor-ligand binding, receptor-hormone binding, antibody-antigen binding, etc. Binding partners include specific, semi-specific, and non-specific binding partners as known to those of ordinary skill in the art. For example, Protein A is usually regarded as a "non-specific" or semi-specific binder. As another example, the particles may contain an enzyme such as glucose oxidase or glucose 1-dehydrogenase, or a lectin such as concanavalin A that is able to bind to glucose.

As additional examples, binding partners may include antibody/antigen pairs, ligand/receptor pairs, enzyme/substrate pairs and complementary nucleic acids or aptamers. Examples of suitable epitopes which may be used for antibody/antigen binding pairs include, but are not limited to, HA, FLAG, c-Myc, glutathione-S-transferase, His₆, GFP, DIG, biotin and avidin. Antibodies may be monoclonal or polyclonal. Suitable antibodies for use as binding partners include antigen-binding fragments, including separate heavy chains, light chains Fab, Fab', F(ab')₂, Fabc, and Fv. Antibodies also include bispecific or bifunctional antibodies. Exemplary binding partners include biotin/avidin, biotin/streptavidin, biotin/neutravidin and glutathione-S-transferase/glutathione.

The term "binding" generally refers to the interaction between a corresponding pair of molecules or surfaces that exhibit mutual affinity or binding capacity, typically due to specific or non-specific binding or interaction, including, but not limited to, biochemical, physiological, and/or chemical interactions. The binding may be between biological molecules, including proteins, nucleic acids, glycoproteins, carbohydrates, hormones, or the like. Specific non-limiting examples include antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, virus/cell surface receptor, etc. As another example, the binding agent may be a chelating agent (e.g., ethylenediaminetetraacetic acid) or an ion selective polymer (e.g., a block copolymer such as poly(carbonate-b-dimethylsiloxane), a crown ether, or the like). As another example, the binding partners may be biotin and streptavidin, or the binding partners may be various antibodies raised against a protein.

The term "specifically binds," when referring to a binding partner (e.g., protein, nucleic acid, antibody, etc.), refers to a reaction that is determinative of the presence and/or identity of one or other member of the binding pair in a mixture of heterogeneous molecules (e.g., proteins and other biologics). Thus, for example, in the case of a receptor/ligand binding pair, the ligand would specifically and/or preferentially select its receptor from a complex mixture of molecules, or vice versa. An enzyme would specifically bind to its substrate, a nucleic acid would specifically bind to its complement, an antibody would specifically bind to its antigen, etc. The binding may be by one or more of a variety of mechanisms including, but not limited to ionic interactions or electrostatic interactions, covalent interactions, hydrophobic interactions, van der Waals interactions, etc.

Thus, the invention provides, in certain embodiments, particles that are able to bind to an analyte, e.g., via a binding partner to the analyte, and such particles can be used to determine the analyte. Such determination may occur within the skin, and/or externally of the subject, e.g., within a device on the surface of the skin, depending on the embodiment. "Determine," in this context, generally refers to the analysis of a species, for example, quantitatively or qualitatively, and/or the detection of the presence or absence of the species. "Determining" may also refer to the analysis of an interaction between two or more species, for example, quantitatively or qualitatively, and/or by detecting the presence or absence of the interaction, e.g. determination of the binding between two species. "Determining" also means detecting or quantifying interaction between species. As an example, an analyte may cause a determinable change in a property of the particles, e.g., a change in a chemical property of the particles, a change in the appearance and/or optical properties of the particles, a change in the temperature of the particles, a change in an electrical property of the particles, etc. In some cases, the change may be one that is determinable by a human, unaided by any equipment that may be directly applied to the human. For instance, the determinable change may be a change in appearance (e.g., color), a change in temperature, the production of an odor, etc., which can be determined by a human without the use of any equipment (e.g., using the eyes). Non-limiting examples include temperature changes, chemical reactions or other interactions (e.g., with capsaicin) that can be sensed, or the like. Examples of capsaicin and capsaicin-like molecules include, but are not limited to, dihydrocapsaicin, nordihydrocapsaicin, homodihydrocapsaicin, homocapsaicin, or nonivamide. Without wishing to be bound by any theory, it is believed that interactions with capsaicin and capsaicin-like molecules can be sensed by a subject, since such molecules may interact with certain nerve endings, which produces a sensation of burning.

In some cases, the particles may contain a diagnostic agent able to determine an analyte. An example of an analyte within a subject is glucose (e.g., for diabetics); other potentially suitable analytes include ions such as sodium, potassium, chloride, calcium, magnesium, and/or bicarbonate (e.g., to determine dehydration); gases such as carbon dioxide or oxygen; pH; metabolites such as urea, blood urea nitrogen or creatinine; hormones such as estradiol, estrone, progesterone, progestin, testosterone, androstenedione, etc. (e.g., to determine pregnancy, illicit drug use, or the like); or cholesterol. Still other potentially suitable analytes include various pathogens such as bacteria or viruses, and/or markers produced by such pathogens. For example, a particle may include an antibody directed at a marker produced by a bacterium. In addition, more than one analyte may be determined in a subject, e.g., through the use of different particle types and/or through the use of particles able to determine more than one analyte, such as those discussed above. For instance, a first set of particles may determine a first analyte and a second set of particles may determine a second analyte. In some cases, such particles may be used to determine a physical condition of a subject. For instance, the particles may exhibit a first color indicating a healthy state and a second color indicating a disease state. In some cases, the appearance of the particles may be used to determine a degree of health. For instance, the particles may exhibit a first color indicating a healthy state, a second color indicating a warning state, and a third color indicating a dangerous state, or the particles may exhibit a range of colors indicating a degree of health of the subject.

Binding partners to these and/or other species are well-known in the art. Non-limiting examples include pH-sensitive entities such as phenol red, bromothymol blue, chlorophenol red, fluorescein, HPTS, 5(6)-carboxy-2',7'-dimethoxyfluorescein SNARF, and phenothalein; entities sensitive to calcium such as Fura-2 and Indo-1; entities sensitive to chloride such as 6-methoxy-N-(3-sulfopropyl)-quinolinim and lucigenin; entities sensitive to nitric oxide such as 4-amino-5-rnethylamino-2',7'-difluorofluorescein; entities sensitive to dissolved oxygen such as tris(4,4'-diphenyl-2,2'-bipyridine) ruthenium (II) chloride pentahydrate; entities sensitive to dissolved CO₂; entities sensitive to fatty acids, such as BODIPY 530-labeled glycerophosphoethanolamine; entities sensitive to proteins such as 4-amino-4'-benzamidostilbene-2-2'-disulfonic acid (sensitive to serum albumin), X-Gal or NBT/BCIP (sensitive to certain enzymes), Tb³⁺ from TbCl₃ (sensitive to certain calcium-binding proteins), BODIPY FL phallacidin (sensitive to actin), or BOCILLIN FL (sensitive to certain penicillin-binding proteins); entities sensitive to concentration of glucose, lactose or other components, or entities sensitive to proteases, lactates or other metabolic byproducts, entities sensitive to proteins, antibodies, or other cellular products.

In some aspects, a pooled region of fluid, such as a suction blister, may be formed in the skin to facilitate delivery to and/or withdrawal of fluid from the skin. Thus, certain aspects of the present invention are generally directed to the creation of suction blisters or other pooled regions of fluid within the skin. In one set of embodiments, a pooled region of fluid can be created between the dermis and epidermis of the skin. Suction blisters or other pooled regions may form in a manner such that the suction blister or other pooled region is not significantly pigmented in some cases, since the basal layer of the epidermis contains melanocytes, which are responsible for producing pigments. Such regions can be created by causing the dermis and the epidermis to at least partially separate, and as will be discussed below, a number of techniques can be used to at least partially separate the dermis from the epidermis.

In one technique, a pool of interstitial fluid is formed between layers of skin of a subject and, after forming the pool, fluid is drawn from the pool by accessing the fluid through a layer of skin, for example, puncturing the outer layer of skin with one or more microneedles. Specifically, for example, a suction blister can be formed and then the suction blister can be punctured and fluid can be drawn from the blister. In another technique, an interstitial region can be accessed and fluid drawn from that region without first forming a pool of fluid via a suction blister or the like. For example, one or more needles or microneedles can be applied to the interstitial region and fluid can be drawn therefrom. Where needles are used, it can be advantageous to select needles or microneedles of length such that interstitial fluid is preferentially obtained and, where not desirable, blood is not accessed (in other embodiments, however, it may be preferred to obtain blood). Those of ordinary skill in the art can arrange needles or microneedles relative to the skin for these purposes including, in one embodiment, introducing needles into the skin at an angle, relative to the skin's surface, other than 90°, i.e., to introduce one or more needles or microneedles into the skin in a slanting fashion so as to access blood or interstitial fluid.

Pooled regions of fluids may be formed on any suitable location within the skin of a subject. Factors such as safety or convenience may be used to select a suitable location, as (in humans) the skin is relatively uniform through the body, with the exception of the hands and feet. As non-limiting examples, the pooled region may be formed on an arm or a leg, on the chest, abdomen, or the back of the subject, or the like. The size of the pooled region of fluid that is formed in the skin and/or the duration that the pooled region lasts within the skin depends on a variety of factors, such as the technique of creating the pooled region, the size of the pooled region, the size of the region of skin to which the technique is applied, the amount of fluid withdrawn from the pooled region (if any), any materials that are delivered into the pooled region, or the like. For example, if vacuum is applied to the skin to create a suction blister, the vacuum applied to the skin, the duration of the vacuum, and/or the area of the skin affected may be controlled to control the size and/or duration of the suction blister. In some embodiments, it may be desirable to keep the pooled regions relatively small, for instance, to prevent an unsightly visual appearance, to allow for greater sampling accuracy (due to a smaller volume of material), or to allow for more controlled placement of particles within the skin. For example, the volume of the pooled region may be kept to less than about 2 ml or less than about 1 ml in certain cases, or the average diameter of the pooled region (i.e., the diameter of a circle having the same area as the pooled region) may be kept to less than about 5 cm, less than about 4 cm, less than about 3 cm, less than about 2 cm, less than about 1 cm, less than about 5 mm, less than about 4 mm, less than about 3 mm, less than about 2 mm, or less than about 1 mm.

A variety of techniques may be used to cause pooled regions of fluid to form within the skin. In one set of embodiments, vacuum is applied to create a suction blister, or otherwise used to collect blood or interstitial fluid from a subject. In other embodiments, however, other methods may be used to create as a pooled region of fluid within the skin besides, or in addition to, the use of vacuum. When vacuum (i.e., the amount of pressure below atmospheric pressure, such that atmospheric pressure has a vacuum of 0 mmHg, i.e., the pressure is gauge pressure rather than absolute pressure) is used to at least partially separate the dermis from the epidermis to cause the pooled region to form, the pooled region of fluid thus formed can be referred to as a suction blister. For example, vacuums of at least about 50 mmHg, at least about 100 mmHg, at least about 150 mmHg, at least about 200 mmHg, at least about 250 mmHg, at least about 300 mmHg, at least about 350 mmHg, at least about 400 mmHg, at least about 450 mmHg, at least about 500 mmHg, at least about 550 mmHg, at least about 600 mmHg, at least about 650 mmHg, at least about 700 mmHg, or at least about 750 mmHg may be applied to the skin, e.g., to cause a suction blister and/or to collect blood or interstitial fluid from a subject (as discussed, these measurements are negative relative to atmospheric pressure). Different amounts of vacuum may be applied to different subjects in some cases, for example, due to differences in the physical characteristics of the skin of the subjects.

The vacuum may be applied to any suitable region of the skin, and the area of the skin to which the vacuum may be controlled in some cases. For instance, the average diameter of the region to which vacuum is applied may be kept to less than about 5 cm, less than about 4 cm, less than about 3 cm, less than about 2 cm, less than about 1 cm, less than about 5 mm, less than about 4 mm, less than about 3 mm, less than about 2 mm, or less than about 1 mm. In addition, such vacuums may be applied for any suitable length of time at least sufficient to cause at least some separation of the dermis from the epidermis to occur. For instance, vacuum may be applied to the skin for at least about 1 min, at least about 3 min, at least about 5 min, at least about 10 min, at least about 15 min, at least about 30 min, at least about 1 hour, at least about 2 hours, at least about 3 hours, at least about 4 hours, etc. Examples of devices suitable for creating such suction blisters are discussed in more detail below. In other cases, however, bodily fluids such as blood or interstitial fluid may be withdrawn from the skin using vacuum without the creation of a suction blister. Other non-limiting examples of fluids include saliva, sweat, tears, mucus, plasma, lymph, or the like.

Other methods besides vacuum may be used to cause such separation to occur. For example, in another set of embodiments, heat may be used. For instance, a portion of the skin may be heated to at least about 40 °C, at least about 50 °C, at least about 55 °C, or at least about 60 °C, using any suitable technique, to cause such separation to occur. The skin may be heated, for instance, using an external heat source (e.g., radiant heat or a heated water bath), a chemical reaction, electromagnetic radiation (e.g., microwave radiation, infrared radiation, etc.), or the like. In some cases, the radiation may be focused on a relatively small region of the skin, e.g., to at least partially spatially contain the amount of heating within the skin that occurs.

In yet another set of embodiments, a separation chemical may be applied to the skin to at least partially cause separation of the dermis and the epidermis to occur. Non-limiting examples of such separation chemicals include proteases such as trypsin, purified human skin tryptase, or compound 48/80. Separation compounds such as these are commercially available from various sources. The separation chemical may be applied directly to the skin, e.g., rubbed into the surface of the skin, or in some cases, the separation chemical can be delivered into the subject, for example, between the epidermis and dermis of the skin. The separation chemical can, for example, be injected in between the dermis and the epidermis.

Another example of a separation chemical is a blistering agent, such as pit viper venom or blister beetle venom. Non-limiting examples of blistering agents include phosgene oxime, Lewisite, sulfur mustards (e.g., mustard gas or 1,5-dichloro-3-thiapentane, 1,2-bis(2-chloroethylthio)ethane, 1,3-bis(2-chloroethylthio)-n-propane, 1,4-bis(2-chloroethylthio)-n-butane, 1,5-bis(2-chloroethylthio)-n-pentane, 2-chloroethylchloromethylsulfide, bis(2-chloroethyl)sulfide, bis(2-chloroethylthio)methane, bis(2-chloroethylthiomethyl)ether, or bis(2-chloroethylthioethyl)ether), or nitrogen mustards (e.g., bis(2-chloroethyl)ethylamine, bis(2-chloroethyl)methylamine, or tris(2-chloroethyl)amine).

In still another set of embodiments, a device may be inserted into the skin and used to mechanically separate the epidermis and the dermis, for example, a wedge or a spike. Fluids may also be used to separate the epidermis and the dermis, in yet another set of embodiments. For example, saline or another relatively inert fluid may be injected into the skin between the epidermis and the dermis to cause them to at least partially separate.

These and/or other techniques may also be combined, in still other embodiments. For example, in one embodiment, vacuum and heat may be applied to the skin of a subject, sequentially and/or simultaneously, to cause such separation to occur. As a specific example, in one embodiment, vacuum is applied while the skin is heated to a temperature of between about 40 °C and about 50 °C.

In another aspect, the present invention is directed to a kit including one or more of the compositions previously discussed, e.g., a kit including a device for the delivery to and/or withdrawal of fluid from the skin and/or beneath the skin, a kit including a device able to create a pooled region of fluid within the skin of a subject, a kit including a device able to determine a fluid, or the like. An example of a kit containing more than one device of the invention is illustrated in Fig. 2D, with kit 150 containing devices 152. A "kit," as used herein, typically defines a package or an assembly including one or more of the compositions or devices of the invention, and/or other compositions or devices associated with the invention, for example, as previously described. For example, in one set of embodiments, the kit may include a device and one or more compositions for use with the device. Each of the compositions of the kit, if present, may be provided in liquid form (e.g., in solution), or in solid form (e.g., a dried powder). In certain cases, some of the compositions may be constitutable or otherwise processable (e.g., to an active form), for example, by the addition of a suitable solvent or other species, which may or may not be provided with the kit. Examples of other compositions or components associated with the invention include, but are not limited to, solvents, surfactants, diluents, salts, buffers, emulsifiers, chelating agents, fillers, antioxidants, binding agents, bulking agents, preservatives, drying agents, antimicrobials, needles, syringes, packaging materials, tubes, bottles, flasks, beakers, dishes, frits, filters, rings, clamps, wraps, patches, containers, tapes, adhesives, and the like, for example, for using, administering, modifying, assembling, storing, packaging, preparing, mixing, diluting, and/or preserving the compositions components for a particular use, for example, to a sample and/or a subject.

A kit of the invention may, in some cases, include instructions in any form that are provided in connection with the compositions of the invention in such a manner that one of ordinary skill in the art would recognize that the instructions are to be associated with the compositions of the invention. For instance, the instructions may include instructions for the use, modification, mixing, diluting, preserving, administering, assembly, storage, packaging, and/or preparation of the compositions and/or other compositions associated with the kit. In some cases, the instructions may also include instructions for the delivery and/or administration of the compositions, for example, for a particular use, e.g., to a sample and/or a subject. The instructions may be provided in any form recognizable by one of ordinary skill in the art as a suitable vehicle for containing such instructions, for example, written or published, verbal, audible (e.g., telephonic), digital, optical, visual (e.g., videotape, DVD, etc.) or electronic communications (including Internet or web-based communications), provided in any manner.

In some embodiments, the present invention is directed to methods of promoting one or more embodiments of the invention as discussed herein. As used herein, "promoted" includes all methods of doing business including, but not limited to, methods of selling, advertising, assigning, licensing, contracting, instructing, educating, researching, importing, exporting, negotiating, financing, loaning, trading, vending, reselling, distributing, repairing, replacing, insuring, suing, patenting, or the like that are associated with the systems, devices, apparatuses, articles, methods, compositions, kits, etc. of the invention as discussed herein. Methods of promotion can be performed by any party including, but not limited to, personal parties, businesses (public or private), partnerships, corporations, trusts, contractual or sub-contractual agencies, educational institutions such as colleges and universities, research institutions, hospitals or other clinical institutions, governmental agencies, etc. Promotional activities may include communications of any form (e.g., written, oral, and/or electronic communications, such as, but not limited to, e-mail, telephonic, Internet, Web-based, etc.) that are clearly associated with the invention.

In one set of embodiments, the method of promotion may involve one or more instructions. As used herein, "instructions" can define a component of instructional utility (e.g., directions, guides, warnings, labels, notes, FAQs or "frequently asked questions," etc.), and typically involve written instructions on or associated with the invention and/or with the packaging of the invention. Instructions can also include instructional communications in any form (e.g., oral, electronic, audible, digital, optical, visual, etc.), provided in any manner such that a user will clearly recognize that the instructions are to be associated with the invention, e.g., as discussed herein.

The following documents are incorporated herein by reference: U.S. Provisional Patent Application Serial No. 61/058,796, filed June 4, 2008, entitled "Compositions and Methods for Diagnostics, Therapies, and Other Applications"; U.S. Provisional Patent Application Serial No. 61/163,791, filed March 26, 2009, entitled "Composition and Methods for Rapid One-Step Diagnosis"; U.S. Provisional Patent Application Serial No. 61/163,793, filed March 26, 2009, entitled "Compositions and Methods for Diagnostics, Therapies, and Other Applications"; U.S. Patent Application Serial No. 12/478,756, filed June 4, 2009, entitled "Compositions and Methods for Diagnostics, Therapies, and Other Applications"; International Patent Application No. PCT/US09/046333, filed June 4, 2009, entitled "Compositions and Methods for Diagnostics, Therapies, and Other Applications"; U.S. Provisional Patent Application Serial No. 61/163,710, filed March 26, 2009, entitled "Systems and Methods for Creating and Using Suction Blisters or Other Pooled Regions of Fluid within the Skin"; U.S. Provisional Patent Application Serial No. 61/163,733, filed March 26, 2009, entitled "Determination of Tracers within Subjects"; U.S. Provisional Patent Application Serial No. 61/163,750, filed March 26, 2009, entitled "Monitoring of Implants and Other Devices"; U.S. Provisional Patent Application Serial No. 61/154,632, filed March 2, 2009, entitled "Oxygen Sensor"; and U.S. Provisional Patent Application Serial No. 61/269,436, filed June 24, 2009, entitled "Devices and Techniques associated with Diagnostics, Therapies, and Other Applications, Including Skin-Associated Applications."

Also incorporated herein by reference are the following applications: U.S. Provisional Patent Application Serial No. 61/256,933, filed October 30, 2009, entitled "Relatively Small Devices Applied to the Skin and Methods of Use Thereof," by *Chickering, et al.;* and U.S. Provisional Patent Application Serial No. 61/256,931, filed October 30, 2009, entitled "Modular Systems for Application to the Skin," by Bernstein, *et al.* In addition, the following are incorporated by reference herein: U.S. Provisional Patent Application Serial No. 61/256,880, filed October 30, 2009, entitled "Systems and Methods for Altering or Masking Perception of Treatment of a Subject," by Chickering, *et al.;* U.S. Provisional Patent Application Serial No. 61/256,874, filed October 30, 2009, entitled "Systems and Methods for Application to Skin and Control of Use Thereof," by Bernstein, *et al.;* U.S. Provisional Patent Application Serial No. 61/256,871, filed October 30, 2009, entitled "Packaging Systems and Methods for Devices Applied to the Skin," by Bernstein, *et al.;* U.S. Provisional Patent Application Serial No. 61/256,863, filed October 30, 2009, entitled "Systems and Methods for Treating or Shielding Blood on the Surface of the Skin," by Bernstein, *et al.;* U.S. Provisional Patent Application Serial No. 61/256,910, filed October 30, 2009, entitled "Systems and Methods for Sanitizing or Treating the Skin or Devices Applied to the Skin," by Bernstein, *et al.;* U.S. Provisional Patent Application Serial No. 61/294,543, filed January 13, 2010, entitled "Blood Sampling Device and Method," by Chickering, *et al.;* U.S. Provisional Patent Application Serial No. 61/334,533, filed May 13, 2010, entitled "Rapid Delivery and/or Withdrawal of Fluids," by *Chickering, et al.;* U.S. Provisional Patent Application Serial No. 61/334,529, filed May 13, 2010, entitled "Sampling Device Interfaces," by Chickering, *et al.;* U.S. Provisional Patent Application Serial No. 61/357,582, filed June 23, 2010, entitled "Sampling Devices and Methods Involving Relatively Little Pain," by Chickering, *et al.;* U.S. Provisional Patent Application Serial No. 61/367,607, filed July 26, 2010, entitled "Rapid Delivery and/or Withdrawal of Fluids," by Davis, *et al.;* and U.S. Provisional Patent Application Serial No. 61/373,764, filed August 13, 2010, entitled "Clinical and/or Consumer Techniques and Devices," by Chickering, *et al.*

The following examples are intended to illustrate certain embodiments of the present invention, but do not exemplify the full scope of the invention.

### EXAMPLE 1

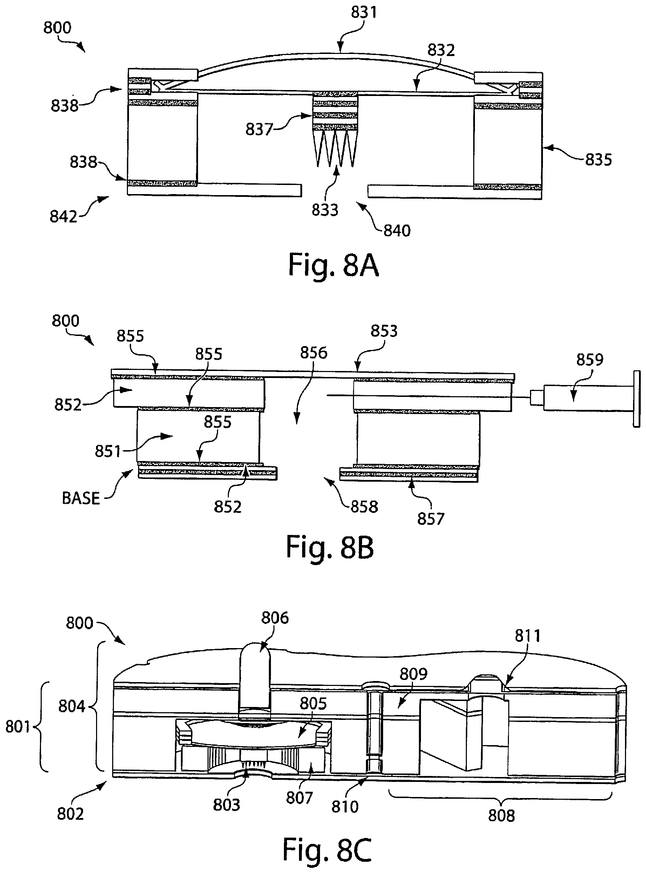

This example illustrates a device for inserting a microneedle array into the skin of a subject. Fig. 8A shows a device 800 including a fluid transporter (e.g., microneedle array 833), a reversibly deformable structure (e.g., snap dome 832), an activator (e.g., activation button 831), a retraction mechanism (e.g., silicone foam 835), and structural components constructed from multiple layers of polycarbonate bonded together using a double-sided adhesive, such as 3M 1509 or 3M 1513 tape. The microneedle arrays can be bonded to laminated post 837 on the underside of a snap dome. Structural components 838, as well as post 837, are formed from polycarbonate and 3M 1509 or 3M 1513 adhesive. The arrays may range in needle number (4 to 28 needles), needle length (350 to 1000 micrometers), and/or arrangement (square, rectangular, and circular arrays), with array footprints of less than 3 mm in diameter, where the "footprint" is the area of the base to which the needles are attached.

In use, the device may be charged by setting the snap dome in a high energy position, placing the base of the device against the skin of a subject (with the needle tips pointing towards the skin), and pushing button 831 on the top of the device. As the button is pressed, silicone foam 835 compresses, positioning the needle tips in close proximity to the skin through opening 840. When the foam is fully compressed, the force causes the button to collapse, which then translates to the back of the snap dome to cause it to move to a stable low energy state. The release of energy from the snap dome changing states accelerates the microneedle array forward through the opening in the base and inserts the needles into the skin. When the force on the button is released, the silicone foam expands to its original height and retracts the needles from the skin in the process.

### EXAMPLE 2

This example illustrates a device for withdrawing blood using vacuum. This device is shown in Fig. 8B. This device includes a vacuum chamber comprising layers of polycarbonate, polyethylene terephthalate glycol (PETG), and silicone bonded together using a double-sided adhesive, such as 3M 1509 or 3M 1513 tape. The chamber is approximately 2.7 cm in diameter and 0.6 cm high, with cup opening 858 in the base that ranges from 3 to 7 mm in diameter. The vacuum chamber may be attached to the skin of a subject over the microneedle insertion site using adhesive 857, such as 3M 1509 or Katecho 10G hydrogel. A vacuum source (i.e., vacuum pump, syringe, vacuum reservoir, etc.) can be connected to the chamber using hypodermic needle 859 inserted through silicone layer 852, and vacuum (i.e., 30 to 70 kPa) may be applied to the site for a fixed period of time (i.e., 10 s to 10 min). The application of vacuum causes blood to flow from the skin punctures into the vacuum chamber.

### EXAMPLE 3

In this example, five human subjects were tested using devices similar to those described in Examples 1 and 2. The interior forearms of the subjects were prepared for treatment by first shaving and then sanitizing with commercial alcohol swabs. Using the snap dome actuator described in Example 1, three different microneedle arrays were inserted into the skin: a microneedle array with eight 750 micrometer long needles, a microneedle array with sixteen 750 micrometer long needles, and a microneedle array with twelve 1000 micrometer long needles. Each microneedle array was tested in triplicate on each subject.

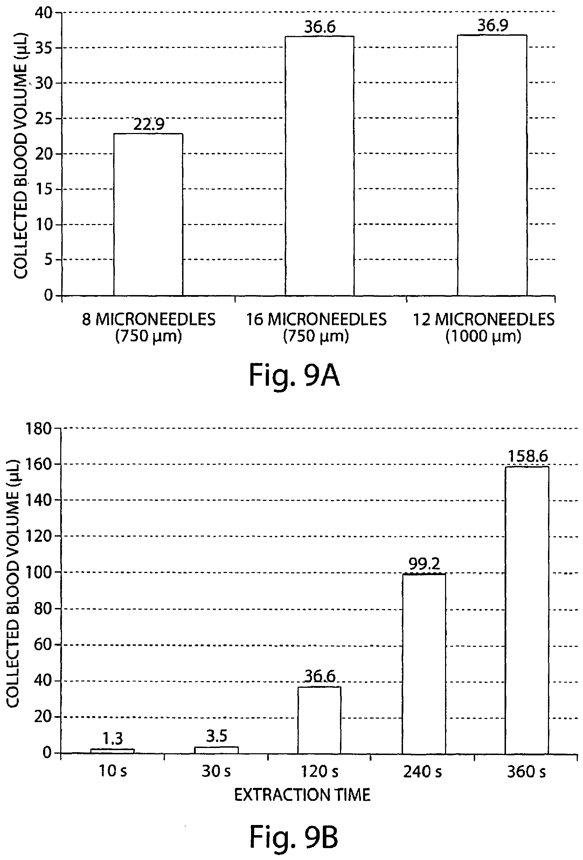

A vacuum chamber with a 5 mm diameter cup (similar to the vacuum chamber described in Example 2) was used to encompass the insertion site and expose it to an absolute pressure of 33.5 kPa for two minutes. The fluid, blood in this case, flowed from the skin into the vacuum chamber over the two minute treatment time. At the end of the treatment, the chamber was returned to atmospheric pressure, and the blood in the vacuum chamber was collected for gravimetric measurement. Fig. 9A summarizes the average volume of blood collected (n = 15 for each condition), illustrating that various configurations may be used to collect clinically relevant volumes of blood for analysis.

### EXAMPLE 4

In this example, five human subjects were used, along with snap dome insertion devices and vacuum chambers similar to those described in Examples 1 and 2. The interior forearms of the subjects were prepared for treatment by first shaving and then sanitizing with commercial alcohol swabs. A microneedle array with sixteen 750 micrometers long needles was then inserted into the prepared area of skin. A vacuum chamber treating a 5 mm diameter area of skin was used to encompass the insertion site and expose it to an absolute pressure of 33.5 kPa for varying durations including: 10 s, 30 s, 120 s, 240 s, and 360 s. Each condition was tested in triplicate on each subject. The fluid, blood in this case, flowed from the skin into the vacuum chamber over the prescribed treatment time. At the end of the treatment, the chamber was returned to atmospheric pressure and the blood in the vacuum chamber was collected for gravimetric measurement. Fig. 9B summarizes the average volume of blood collected (n = 15 for each condition).

As can be seen from this figure, microneedle arrays were successfully used to withdraw a fluid, blood in this case, over durations ranging from seconds to minutes. This example demonstrates that clinically relevant volumes of blood can be withdrawn in as little as 10 seconds.

### EXAMPLE 5

In this example, five human subjects were used to show that under various conditions, at least 5 microliters of blood could be collected, which is the maximum volume of blood required to perform a glucose test. The snap dome actuator and vacuum chamber used in this example were similar to those described in Examples 1 and 2. The interior forearms of subjects were prepared for treatment by first shaving and then sanitizing with commercial alcohol swabs. A microneedle array with sixteen 750 micrometer long needles was then inserted into the prepared area of skin with a snap dome actuator. A vacuum chamber treating a 5 mm diameter area of skin was used to encompass the insertion site and expose it to an absolute pressure of 33.5 kPa for two minutes. The fluid, blood in this case, flowed from the skin into the vacuum chamber over the prescribed treatment time. At the end of the treatment, the chamber was returned to atmospheric pressure and the blood in the vacuum chamber was collected for gravimetric measurement.

The volume of blood collected from the skin of the subjects under these conditions was 36.6 ± 12.4 microliters. Assuming the subjects are representative of the population as a whole, and that the distribution of the population is normal, the cumulative probability of collecting a blood volume greater than or equal to a desired volume may be calculated from the probability distribution function. Given the average value of 36.6 microliters and the standard deviation of 12.4 microliters, the probability of achieving 5 microliters of blood was determined to be greater than 99.5%. This example demonstrates that the use of microneedle arrays offers a higher degree of success as compared to standard lancets used on the forearm, which typically have success rates of less than 95%. For example, a value of 93.4% has been recently reported in the literature (D. Bina, et al., Diabetes Care, Vol. 26(4), p. 981-985, 2003.).

### EXAMPLE 6

In this example, a fully integrated device was constructed for the withdrawal of a fluid from human subjects. A diagram of the device is shown in Fig. 8C. In this example, the integrated device 800 includes a support structure 801 for application to the skin of the subject. The structure is constructed from multiple layers of polyethylene terephthalate glycol (PETG). These layers may be formed into the requisite geometry by machining sheet stock or injection molding. The individual layers are bonded together using double-sided adhesive, such as 3M 1513 tape, but may also be bonded using non-adhesive methods such as ultrasonic welding or laser welding. The support structure is attached to the skin of a subject using an adhesive 802, such as Katecho 10G hydrogel.

The left side of the support structure in Fig. 8C houses the components necessary to insert a microneedle array into the skin. These components include a circular microneedle array of sixteen 750 micrometers long needles 803 actuated by the extraction activator 804 comprising a reversibly deformable structure (e.g., a snap dome 805), a button 806, and a foam return mechanism 807. Pressing the button initially compresses the foam, bringing the microneedles into close proximity with the skin, and then fires the snap dome, moving it from the first stable configuration to the second stable configuration. The movement of the snap dome accelerates and inserts the microneedles into the skin. Releasing the pressure on the button allows the foam to expand and retract the microneedles from the skin.

The right side of the support structure shown in Fig. 8C comprises a self contained vacuum chamber 808 fluidically connected to a storage chamber 809. The storage chamber is fluidically connected to the extraction activator by a microfluidic channel 810. Pressing the button 811 breaks a seal and causes the fluidically connected components to be evacuated as well as reduces the pressure on the skin below the microneedle array. This reduced pressure urges blood from the skin into the microfluidic channel and into the storage chamber.

The fully integrated device was used to withdraw blood from 10 human subjects. The device collected 12 microliters of blood in an average time of 108 s (n = 17).

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

**Embodiments of the invention will now be described in the following numbered paragraphs:**
1. A device for analysis of an extractable fluid from the skin and/or from beneath the skin of a subject, the device comprising:
   a support structure for application to the skin of the subject;
   a fluid transporter associated with the support structure; and
   a signal structure for generating a signal indicative of the determination of at least one analyte contained within the fluid,
   wherein the support structure and the signal structure are constructed and arranged to be readily connectable and/or detachable from each other.
2. The device of paragraph 1, wherein the support structure further comprises an adhesive for adhesion of the support structure to the skin.
3. The device of any one of paragraphs 1 or 2, wherein the support structure is able to be placed against the skin of the subject.
4. The device of any one of paragraphs 1-3, wherein the device is able to determine a force created on the device when the device is placed against the skin.
5. The device of any one of paragraphs 1-4, wherein the device is able to determine body heat from the subject.
6. The device of any one of paragraphs 1-5, wherein the fluid transporter is able to be activated using an actuator.
7. The device of paragraph 6, wherein the actuator comprises a button.
8. The device of any one of paragraphs 1-7, wherein the fluid transporter is able to be activated remotely.
9. The device of any one of paragraphs 1-8, wherein the device comprises a timing mechanism for timed activation of the fluid transporter after removal from packaging containing the device.
10. The device of any one of paragraphs 1-9, wherein the support structure further comprises a mechanical element for affixing the device to the skin of the subject.
11. The device of any one of paragraphs 1-10, wherein the support structure contains a recess containing the fluid transporter.
12. The device of any one of paragraphs 1-11, wherein the support structure further comprises a microfluidic channel in fluidic communication with the fluid transporter.
13. The device of any one of paragraphs 1-12, wherein the fluid comprises blood.
14. The device of any one of paragraphs 1-13, wherein the fluid comprises interstitial fluid.
15. The device of any one of paragraphs 1-14, wherein the device further comprises a storage chamber in fluidic communication with the fluid transporter.
16. The device of any one of paragraphs 1-15, wherein the device is able to determine an analyte contained within the fluid withdrawn from the skin and/or from beneath the skin of the subject.
17. The device of any one of paragraphs 1-16, wherein the device is able to determine the analyte without subsequent removal of the device from the skin of the subject.
18. The device of any one of paragraphs 1-17, wherein the device is able to automatically determine the analyte.
19. The device of any one of paragraphs 1-18, wherein the device is able to display a result of the determination of the analyte.
20. The device of any one of paragraphs 1-19, wherein the fluid transporter comprises a needle.
21. The device of paragraph 20, wherein the needle is a microneedle.
22. The device of any one of paragraphs 1-21, wherein the fluid transporter comprises a solid microneedle.
23. The device of any one of paragraphs 1-22, wherein the fluid transporter comprises a hollow microneedle.
24. The device of any one of paragraphs 1-23, wherein the fluid transporter is immobilized relative to the support structure.
25. The device of any one of paragraphs 1-24, wherein the device is able to withdraw the fluid transporter from the skin of the subject after exposure of the fluid transporter to the skin of the subject.
26. The device of any one of paragraphs 1-25, wherein the support structure is able to draw the skin of the subject towards the fluid transporter.
27. The device of any one of paragraphs 1-26, wherein the support structure is able to apply a pressure less than atmospheric pressure to the skin of the subject.
28. The device of any one of paragraphs 1-27, wherein the support structure comprises a self-contained vacuum chamber having a pressure less than atmospheric pressure.
29. The device of any one of paragraphs 1-28, wherein the fluid transporter is able to move relative to the support structure.
30. The device of any one of paragraphs 1-29, wherein the fluid transporter is able to be activated by the subject.
31. The device of any one of paragraphs 1-30, wherein the fluid transporter is able to be activated upon applying the device to the skin of the subject.
32. The device of any one of paragraphs 1-31, wherein the device further comprisies a heater for applying heat to the skin of the subject.
33. The device of any one of paragraphs 1-32, wherein the device further comprises a cooler for cooling the skin of the subject.
34. The device of any one of paragraphs 1-33, wherein the at least one analyte comprises glucose.
35. A device for delivering to and/or withdrawing a fluid from the skin and/or beneath the skin of a subject, the device comprising:
   a support structure for application to the skin of the subject;
   a fluid transporter associated with the support structure; and
   a signal structure for generating a signal indicative of the fluid delivered to and/or withdrawn from the skin and/or beneath the skin of the subject;
   wherein the support structure and the signal structure are constructed and arranged to be connectable and/or detachable from each other.
36. The device of paragraph 35, wherein the device has a weight of less than about 1 kg.
37. The device of any one of paragraphs 35 or 36, wherein the device has a weight of less than about 300 g.
38. The device of any one of paragraphs 35-37, wherein the device has a maximum linear dimension less than about 25 cm.
39. The device of any one of paragraphs 35-38, wherein the device has a maximum linear dimension less than about 10 cm.
40. The device of any one of paragraphs 35-39, wherein the device has a maximum linear dimension less than about 5 cm.
41. The device of any one of paragraphs 35-40, wherein the device is constructed and arranged to be affixed on the arm of a subject.
42. The device of any one of paragraphs 35-41, wherein the device is constructed and arranged to be affixed on the leg of a subject.
43. The device of any one of paragraphs 35-42, wherein the device is constructed and arranged to be affixed to a subject such that the subject is able to walk after the device has been affixed thereto.
44. The device of any one of paragraphs 35-43, wherein the support structure defines at least a portion of a one-use, disposable module, and the signal structure defines at least a portion of a module constructed and arranged for repeated connection and disconnection to at least the support structure.
45. A device for delivering to and/or withdrawing a fluid from the skin and/or beneath the skin of a subject, the device comprising:
   a support structure for application to the skin of the subject;
   a fluid transporter associated with the support structure; and
   a signal structure for generating a signal indicative of the fluid delivered to and/or withdrawn from the skin and/or beneath the skin of the subject;
   wherein the device is repeatedly applicable to the skin of the subject to deliver to and/or withdraw the fluid from the skin and/or beneath the skin of a subject.
46. A method, comprising:
   applying a device to the skin of a subject, the device able to deliver to and/or withdraw a fluid from the skin and/or beneath the skin of a subject, the device comprising: (1) a support structure for application to the skin of the subject, (2) a fluid transporter associated with the support structure; and (3) a signal structure for generating a signal indicative of the fluid delivered to and/or withdrawn from the skin and/or beneath the skin of the subject;
   delivering to and/or withdrawing the fluid from the skin and/or beneath the skin of the subject using the device; and
   removing only one of the support structure and the signal structure from the skin of the subject.
47. The method of paragraph 46, further comprising transporting the removed structure to a secondary site for analysis.
48. The method of any one of paragraphs 46 or 47, wherein the removed structure is the support structure.
49. The method of any one of paragraphs 46-48, wherein the support structure contains a fluid withdrawn from the skin and/or beneath the skin of the subject using the device.
50. A device for withdrawing a fluid from the skin and/or from beneath the skin of a subject, the device comprising:
   a support structure for application to the skin of the subject;
   a fluid transporter associated with the support structure; and
   a stabilizing agent for stabilizing the fluid withdrawn from the skin in fluid communication with the fluid transporter.
51. A device for withdrawing a fluid from the skin and/or from beneath the skin of a subject, the device comprising:
   a support structure for application to the skin of the subject;
   a fluid transporter associated with the support structure; and
   an anticoagulant for stabilizing the fluid withdrawn from the skin in fluid communication with the fluid transporter.
52. The device of paragraph 51, wherein the anticoagulant comprises heparin.
53. A method, comprising:
   applying a device to the skin of a subject, the device able to withdraw a fluid from the skin and/or from beneath the skin of the subject, the device comprising: (1) a support structure for application to the skin of the subject, and (2) a fluid transporter associated with the support structure;
   withdrawing the fluid from the skin and/or beneath the skin of the subject using the fluid transporter; and
   transporting at least a portion of the device containing the withdrawn fluid to a secondary site for analysis.
54. The method of paragraph 53, comprising transporting by mail.
55. The method of any one of paragraphs 53 or 54, comprising transporting only a portion of the device.
56. The method of any one of paragraphs 53-55, comprising transporting the entire device.
57. A device for analysis of an extractable fluid from the skin and/or from beneath the skin of a subject, the device comprising:
   a support structure for application to the skin of the subject;
   means for extracting a fluid from the skin and/or from beneath the skin of the subject; and
   a signal structure for generating a signal indicative of the determination of at least one analyte contained within the fluid,
   wherein the support structure and the signal structure are constructed and arranged to be readily connectable and/or detachable from each other.
58. A device for delivering to and/or withdrawing a fluid from the skin and/or beneath the skin of a subject, the device comprising:
   a support structure for application to the skin of the subject;
   transport means for delivery to and/or withdrawal of a fluid from the skin and/or beneath the skin of the subject; and
   a signal structure for generating a signal indicative of the fluid delivered to and/or withdrawn from the skin and/or beneath the skin of the subject;
   wherein the support structure and the signal structure are constructed and arranged to be connectable and/or detachable from each other.
59. A device for delivering to and/or withdrawing a fluid from the skin and/or beneath the skin of a subject, the device comprising:
   a support structure for application to the skin of the subject;
   transport means for delivery to and/or withdrawal of a fluid from the skin and/or beneath the skin of the subject; and
   a signal structure for generating a signal indicative of the fluid delivered to and/or withdrawn from the skin and/or beneath the skin of the subject;
   wherein the device is repeatedly applicable to the skin of the subject to deliver to and/or withdraw the fluid from the skin and/or beneath the skin of a subject.
60. A method, comprising:
   applying a device to the skin of a subject, the device able to deliver to and/or withdraw a fluid from the skin and/or beneath the skin of the subject, the device comprising: (1) a support structure for application to the skin of the subject, (2) transport means for delivery to and/or withdrawal of the fluid from the skin and/or beneath the skin of the subject, and (3) a signal structure for generating a signal indicative of the fluid delivered to and/or withdrawn from the skin and/or beneath the skin of the subject;
   delivering to and/or withdrawing the fluid from the skin and/or beneath the skin of the subject using the device; and
   removing only one of the support structure and the signal structure from the skin of the subject.
61. A device for withdrawing a fluid from the skin and/or from beneath the skin of a subject, the device comprising:
   a support structure for application to the skin of the subject;
   transport means associated with the support structure; and
   a stabilizing agent for stabilizing the fluid withdrawn from the skin in fluid communication with the transport means.
62. A device for withdrawing a fluid from the skin and/or from beneath the skin of a subject, the device comprising:
   a support structure for application to the skin of the subject;
   transport means associated with the support structure; and
   an anticoagulant for stabilizing the fluid withdrawn from the skin in fluid communication with the transport means.
63. A method, comprising:
   applying a device to the skin of a subject, the device able to withdraw a fluid from the skin and/or from beneath the skin of the subject, the device comprising: (1) a support structure for application to the skin of the subject, and (2) transport means for withdrawal of a fluid from the skin and/or from beneath the skin of a subject associated with the support structure;
   withdrawing the fluid from the skin and/or beneath the skin of the subject using the transport means; and
   transporting at least a portion of the device containing the withdrawn fluid to a secondary site for analysis.
64. A device for extraction of a fluid from the skin and/or from beneath the skin of a subject, the device comprising:
   a support structure for application to the skin of the subject; and
   a fluid transporter associated with the support structure;
   wherein the device comprises at least one of:
      a largest lateral dimension, when the device is positioned for extraction of the fluid, parallel to the extraction area, of no more than about 5 cm,
      a largest vertical dimension, extending from the skin of the subject when the device is positioned for extraction of the fluid, of no more than about 1 cm, or
      a mass of no more than about 25 g, absent the fluid.
65. The device of paragraph 64, wherein the support structure further comprises an adhesive for adhesion of the support structure to the skin.
66. The device of any one of paragraphs 64 or 65, wherein the support structure is able to be placed against the skin of the subject.
67. The device of any one of paragraphs 64-66, wherein the device is able to determine a force created on the device when the device is placed against the skin.
68. The device of any one of paragraphs 64-67, wherein the device is able to determine body heat from the subject.
69. The device of any one of paragraphs 64-68, wherein the fluid transporter is able to be activated using an actuator.
70. The device of paragraph 69, wherein the actuator comprises a button.
71. The device of any one of paragraphs 64-70, wherein the fluid transporter is able to be activated remotely.
72. The device of any one of paragraphs 64-71, wherein the device comprises a timing mechanism for timed activation after removal of the fluid transporter from packaging containing the device.
73. The device of any one of paragraphs 64-72, wherein the support structure further comprises a mechanical element for affixing the device to the skin of the subject.
74. The device of any one of paragraphs 64-73, wherein the support structure contains a recess containing the fluid transporter.
75. The device of any one of paragraphs 64-74, wherein the support structure further comprises a microfluidic channel in fluidic communication with the fluid transporter.
76. The device of any one of paragraphs 64-75, wherein the device is able to withdraw the fluid from the skin and/or from beneath the skin of the subject via the fluid transporter.
77. The device of any one of paragraphs 64-76, wherein the medium comprises blood.
78. The device of any one of paragraphs 64-77, wherein the medium comprises interstitial fluid.
79. The device of any one of paragraphs 64-78, wherein the device further comprises a storage chamber in fluidic communication with the fluid transporter.
80. The device of any one of paragraphs 64-79, wherein the device is able to determine an analyte contained within the fluid withdrawn from the skin and/or from beneath the skin of the subject.
81. The device of paragraph 80, wherein the device is able to determine the analyte without subsequent removal of the device from the skin of the subject.
82. The device of any one of paragraphs 80 or 81, wherein the device is able to automatically determine the analyte.
83. The device of any one of paragraphs 80-82, wherein the device is able to display a result of the determination of the analyte.
84. The device of any one of paragraphs 80-83, wherein the analyte is glucose.
85. The device of any one of paragraphs 64-84, wherein the fluid transporter comprises a needle.
86. The device of any one of paragraphs 64-85, wherein the needle is a microneedle.
87. The device of any one of paragraphs 64-86, wherein the fluid transporter comprises a solid microneedle.
88. The device of any one of paragraphs 64-87, wherein the fluid transporter comprises a hollow microneedle.
89. The device of any one of paragraphs 64-88, wherein the fluid transporter is immobilized relative to the support structure.
90. The device of any one of paragraphs 64-89, wherein the device is able to withdraw the fluid transporter from the skin of the subject after exposure of the fluid transporter to the skin of the subject.
91. The device of any one of paragraphs 64-90, wherein the support structure is able to draw the skin of the subject towards the fluid transporter.
92. The device of any one of paragraphs 64-91, wherein the support structure is able to apply a pressure less than atmospheric pressure to the skin of the subject.
93. The device of any one of paragraphs 64-92, wherein the support structure comprises a self-contained vacuum chamber having a pressure less than atmospheric pressure.
94. The device of any one of paragraphs 64-93, wherein the fluid transporter is able to move relative to the support structure.
95. The device of any one of paragraphs 64-94, wherein the fluid transporter is able to be activated by the subject.
96. The device of any one of paragraphs 64-95, wherein the fluid transporter is able to be activated upon applying the device to the skin of the subject.
97. The device of any one of paragraphs 64-96, wherein the device further comprises a heater for applying heat to the skin of the subject.
98. The device of any one of paragraphs 64-97, wherein the device further comprises a cooler for cooling the skin of the subject.
99. The device of any one of paragraphs 64-98, wherein the largest lateral dimension is between about 2.5 cm and about 5 cm.
100. The device of any one of paragraphs 64-99, wherein the largest vertical dimension is between about 0.5 cm and about 1 cm.
101. The device of any one of paragraphs 64-100, wherein the mass is between about 2 g and about 25 g.
102. A device for extraction of a fluid from the skin and/or from beneath the skin of a subject, the device comprising:
   a support structure for application to the skin of the subject;
   a fluid transporter associated with the support structure;
   a sensor for determining at least one condition of a fluid extracted from the skin and/or from beneath the skin of the subject; and
   a signal and/or display generator for generating a signal and/or display relating to the at least one condition of the fluid determined by the sensor,
   wherein the device comprises at least one of:
      a largest lateral dimension, when the device is positioned for extraction of the fluid, parallel to the extraction area, of no more than about 7 cm,
      a largest vertical dimension, extending from the skin of the subject when the device is positioned for extraction of the fluid, of no more than about 3 cm, or
      a mass of no more than about 150 g, absent the fluid.
103. The device of paragraph 102, wherein the support structure further comprises an adhesive for adhesion of the support structure to the skin.
104. The device of any one of paragraphs 102 or 103, wherein the largest lateral dimension is between about 2 cm and about 7 cm.
105. The device of any one of paragraphs 102-104, wherein the largest vertical dimension is between about 2 and about 3 cm.
106. The device of any one of paragraphs 102-105, wherein the mass is between about 30 g and about 150 g.
107. The device of any one of paragraphs 102-106, wherein the sensor is a glucose sensor.
108. The device of any one of paragraphs 102-107, wherein the sensor is a test strip.
109. A device for extraction of a fluid from the skin and/or beneath the skin of a subject, the device comprising:
   a support structure for application to the skin of the subject; and
   a fluid transporter associated with the support structure,
   wherein the device is constructed and arranged to be wearable by the subject for a period of at least 15 minutes.
110. The device of paragraph 109, wherein the support structure further comprises an adhesive for adhesion of the support structure to the skin.
111. A self-contained device for delivering to and/or withdrawing a fluid from the skin and/or beneath the skin of a subject, the device comprising:
   a support structure for application to the skin of the subject; and
   a fluid transporter for delivery to and/or withdrawal of a fluid from the skin and/or beneath the skin of the subject,
   wherein the self-contained device, when affixed to the skin of the subject, comprises at least one of:
      (1) a largest lateral dimension, when the device is positioned for extraction of the fluid, parallel to the extraction area, of no more than about 5 cm,
      (2) a largest vertical dimension, extending from the skin of the subject when the device is applied to the subject, of no more than about 1 cm, or
      (3) a mass of no more than about 25 g, absent the fluid.
112. The device of paragraphs 111, wherein the largest lateral dimension is between about 2.5 cm and about 5 cm.
113. The device of any one of paragraphs 111 or 112, wherein the largest vertical dimension is between about 0.5 cm and about 1 cm.
114. The device of any one of paragraphs 111-113, wherein the mass is between about 2 g and about 25 g.
115. A self-contained device for delivering to and/or withdrawing a fluid from the skin and/or beneath the skin of a subject, the device comprising:
   a support structure for application to the skin of the subject;
   a fluid transporter for delivery to and/or withdrawal of a fluid from the skin and/or beneath the skin of the subject; and
   a signal structure for generating a signal indicative of the fluid delivered to and/or withdrawn from the skin and/or beneath the skin of the subject,
   wherein the self-contained device, when affixed to the skin of the subject, comprises at least one of:
      (1) a largest lateral dimension, when the device is positioned for extraction of the fluid, parallel to the extraction area, of no more than about 7 cm,
      (2) a largest vertical dimension, extending from the skin of the subject when the device is applied to the subject, of no more than about 3 cm, or
      (3) a mass of no more than about 150 g, absent the fluid.
116. The device of paragraph 115, wherein the device further comprises a sensor for determining at least one condition of a fluid extracted from the skin of the subject via the fluid transporter.
117. The device of paragraph 116, wherein the device comprises a signal generator for generating a signal relating to the at least one condition of the fluid determined by the sensor.
118. The device of paragraph 117, wherein the signal generator comprises a display.
119. The device of any one of paragraphs 116-118, wherein the sensor is a glucose sensor.
120. The device of any one of paragraphs 116-119, wherein the sensor is a test strip.
121. The device of any one of paragraphs 115-120, further comprising an activator constructed and arranged to cause exposure of the fluid transporter to the skin of the subject upon activation of the activator.
122. The device of any one of paragraphs 115-121, further comprising a signal structure for generating a signal indicative of the fluid delivered to and/or withdrawn from the skin and/or beneath the skin of the subject.
123. The device of any one of paragraphs 115-122, wherein the largest lateral dimension is between about 2 cm and about 7 cm.
124. The device of any one of paragraphs 115-123, wherein the largest vertical dimension is between about 2 and about 3 cm.
125. The device of any one of paragraphs 115-124, wherein the mass is between about 30 g and about 150 g.
126. A self-contained device for delivering to and/or withdrawing a fluid from the skin and/or beneath the skin of a subject, the device comprising:
   a support structure for application to the skin of the subject; and
   a fluid transporter for delivery to and/or withdrawal of the fluid from the skin and/or beneath the skin of the subject,
   wherein the device is constructed and arranged to be wearable by the subject for at least about 15 minutes.
127. The device of paragraph 126, further comprising an activator constructed and arranged to cause exposure of the fluid transporter to the skin of the subject upon activation of the activator.
128. The device of any one of paragraphs 126 or 127, further comprising a signal structure for generating a signal indicative of the fluid delivered to and/or withdrawn from the skin and/or beneath the skin of the subject.
129. The device of any one of paragraphs 126-128, wherein the device is constructed and arranged to be wearable by the subject for at least about 1 hour.
130. The device of any one of paragraphs 126-129, wherein the device is constructed and arranged to be wearable by the subject for at least about 1 day.
131. The device of any one of paragraphs 126-130, wherein the device is constructed and arranged to be wearable by the subject for at least about 1 week.
132. A method, comprising:
   applying a self-contained device to the skin of a subject able to deliver to and/or withdraw a fluid from the skin and/or beneath the skin of the subject, wherein the self-contained device, when affixed to the skin of the subject, comprises at least one of:
      (1) a largest lateral dimension, when the device is positioned for extraction of the fluid, parallel to the extraction area, of no more than about 5 cm,
      (2) a largest vertical dimension, extending from the skin of the subject when the device is applied to the subject, of no more than about 1 cm, or
      (3) a mass of no more than about 25 g, absent the fluid; and activating the device.
133. A method, comprising:
   applying a self-contained device to the skin of a subject, the device comprising: (1) a support structure for application to the skin of the subject, (2) a fluid transporter associated with the support structure, and (3) a signal structure for generating a signal indicative of the fluid delivered to and/or withdrawn from the skin and/or beneath the skin of the subject, wherein the self-contained device, when affixed to the skin of the subject, comprises at least one of:
      (1) a largest lateral dimension, when the device is positioned for extraction of the fluid, parallel to the extraction area, of no more than about 5 cm,
      (2) a largest vertical dimension, extending from the skin of the subject when the device is applied to the subject, of no more than about 1 cm, or
      (3) a mass of no more than about 25 g, absent the fluid; and activating the device.
134. A method, comprising:
   applying a self-contained device to the skin of a subject able to deliver to and/or withdraw a fluid from the skin and/or beneath the skin of a subject;
   allowing the subject to wear the device for at least about 15 minutes; and
   delivering to and/or withdrawing the fluid from the skin and/or beneath the skin of the subject.
135. A device for delivering to and/or withdrawing a fluid from the skin and/or beneath the skin of a subject, the device comprising:
   a support structure for application to the skin of the subject;
   a fluid transporter associated with the support structure; and
   a sensor for determining a condition of the fluid delivered to and/or withdrawn from the skin and/or beneath the skin of the subject,
   wherein the device is constructed and arranged to be wearable by the subject for at least about 15 minutes.
136. The device of paragraph 135, wherein the sensor is a glucose sensor.
137. The device of any one of paragraphs 135 or 136, wherein the sensor is a test strip.
138. A device for withdrawing interstitial fluid from the skin and/or from beneath the skin of a subject, the device comprising:
   a support structure for application to the skin of the subject; and
   a fluid transporter associated with the support structure,
   wherein the device is constructed and arranged to be wearable by the subject for at least about 15 minutes.
139. A device for delivering to and/or withdrawing a fluid from the skin and/or beneath the skin of a subject, the device comprising:
   a support structure for application to the skin of the subject; and
   a fluid transporter associated with the support structure,
   wherein the device is constructed and arranged to be wearable by the subject for at least about 15 minutes.
140. A method, comprising:
   applying a self-contained device to the skin of a subject able to withdraw a fluid from the skin and/or from beneath the skin of the subject;
   allowing the subject to wear the device for at least about 15 minutes;
   withdrawing the fluid from the skin and/or from beneath the skin of the subject using the device; and
   determining an analyte suspected of being contained within the fluid withdrawn from the skin of the subject.
141. The method of paragraph 140, wherein the analyte comprises glucose.
142. A method, comprising:
   applying a self-contained device to the skin of a subject able to withdraw a fluid from the skin of the subject;
   allowing the subject to wear the device for at least about 15 minutes; and
   withdrawing the fluid directly from one or more capillaries in the skin of the subject using the device.
143. A device for extraction of a fluid from the skin and/or from beneath the skin of a subject, the device comprising:
   a support structure for application to the skin of the subject; and
   a fluid transporter associated with the support structure;
   wherein the device comprises at least one of:
      a largest lateral dimension, when the device is positioned for extraction of the fluid, parallel to the extraction area, of no more than about 6 cm,
      a largest vertical dimension, extending from the skin of the subject when the device is positioned for extraction of the fluid, of no more than about 1.5 cm, or
      a mass of no more than about 25 g, absent the fluid.
144. A self-contained device for delivering to and/or withdrawing a fluid from the skin and/or beneath the skin of a subject, the device comprising:
   a support structure for application to the skin of the subject; and
   a fluid transporter associated with the support structure,
   wherein the self-contained device, when affixed to the skin of the subject, comprises at least one of:
      (1) a largest lateral dimension, when the device is positioned for extraction of the fluid, parallel to the extraction area, of no more than about 6 cm,
      (2) a largest vertical dimension, extending from the skin of the subject when the device is applied to the subject, of no more than about 1.5 cm, or
      (3) a mass of no more than about 25 g, absent the fluid.
145. A method, comprising:
   applying a self-contained device to the skin of a subject able to deliver to and/or withdraw a fluid from the skin and/or beneath the skin of the subject, wherein the self-contained device, when affixed to the skin of the subject, comprises at least one of:
      (1) a largest lateral dimension, when the device is positioned for extraction of the fluid, parallel to the extraction area, of no more than about 6 cm,
      (2) a largest vertical dimension, extending from the skin of the subject when the device is applied to the subject, of no more than about 1.5 cm, or
      (3) a mass of no more than about 25 g, absent the fluid; and
   activating the device.
146. A device for extraction of a fluid from the skin and/or from beneath the skin of a subject, the device comprising:
   a support structure for application to the skin of the subject;
   a fluid transporter associated with the support structure; and
   an adhesive for adhesion of the support structure to the skin of the subject.
147. The method of paragraph 146, comprising applying the device to the skin of the subject for no more than about 1 hour.
148. The method of paragraph 146, comprising applying the device to the skin of the subject for no more than about 30 min.
149. The method of paragraph 146, comprising applying the device to the skin of the subject for no more than about 15 min.
150. The method of paragraph 146, comprising applying the device to the skin of the subject for no more than about 10 min.
151. The method of paragraph 146, comprising applying the device to the skin of the subject for no more than about 5 min.
152. A device for extraction of a fluid from the skin and/or from beneath the skin of a subject, the device comprising:
   a support structure for application to the skin of the subject; and
   means for extracting a fluid from the skin and/or from beneath the skin of the subject at an extraction area;
   wherein the device comprises at least one of:
      a largest lateral dimension, when the device is positioned for extraction of the fluid, parallel to the extraction area, of no more than about 5 cm,
      a largest vertical dimension, extending from the skin of the subject when the device is positioned for extraction of the fluid, of no more than about 1 cm, or
      a mass of no more than about 25 g, absent the fluid.
153. A device for extraction of a fluid from the skin and/or from beneath the skin of a subject, the device comprising:
   a support structure for application to the skin of the subject;
   means for extracting a fluid from the skin and/or from beneath the skin of the subject at an extraction area;
   a sensor for determining at least one condition of a fluid extracted from the skin of the subject; and
   a signal and/or display generator for generating a signal and/or display relating to the at least one condition of the fluid determined by the sensor,
   wherein the device comprises at least one of:
      a largest lateral dimension, when the device is positioned for extraction of the fluid, parallel to the extraction area, of no more than about 7 cm,
      a largest vertical dimension, extending from the skin of the subject when the device is positioned for extraction of the fluid, of no more than about 3 cm, or
      a mass of no more than about 150 g, absent the fluid.
154. A device for extraction of a fluid from the skin and/or from beneath the skin of a subject, the device comprising:
   a support structure for application to the skin of the subject; and
   means for extracting a fluid from the skin and/or from beneath the skin of the subject at an extraction area,
   wherein the device is constructed and arranged to be wearable by the subject for a period of at least 15 minutes.
155. A self-contained device for delivering to and/or withdrawing a fluid from the skin and/or beneath the skin of a subject, the device comprising:
   a support structure for application to the skin of the subject; and
   transport means for delivery to and/or withdrawal of a fluid from the skin and/or beneath the skin of the subject,
   wherein the self-contained device, when affixed to the skin of the subject, comprises at least one of:
      (1) a largest lateral dimension, when the device is positioned for extraction of the fluid, parallel to the extraction area, of no more than about 5 cm,
      (2) a largest vertical dimension, extending from the skin of the subject when the device is applied to the subject, of no more than about 1 cm, or
      (3) a mass of no more than about 25 g, absent the fluid.
156. A self-contained device for delivering to and/or withdrawing a fluid from the skin and/or beneath the skin of a subject, the device comprising:
   a support structure for application to the skin of the subject;
   transport means for delivery to and/or withdrawal of a fluid from the skin and/or beneath the skin of the subject; and
   a signal structure for generating a signal indicative of the fluid delivered to and/or withdrawn from the skin and/or beneath the skin of the subject,
   wherein the self-contained device, when affixed to the skin of the subject, comprises at least one of:
      (1) a largest lateral dimension, when the device is positioned for extraction of the fluid, parallel to the extraction area, of no more than about 7 cm,
      (2) a largest vertical dimension, extending from the skin of the subject when the device is applied to the subject, of no more than about 3 cm, or
      (3) a mass of no more than about 150 g, absent the fluid.
157. A self-contained device for delivering to and/or withdrawing a fluid from the skin and/or beneath the skin of a subject, the device comprising:
   a support structure for application to the skin of the subject; and
   transport means for delivery to and/or withdrawal of the fluid from the skin and/or beneath the skin of the subject,
   wherein the device is constructed and arranged to be wearable by the subject for at least about 15 minutes.
158. A method, comprising:
   applying a self-contained device to the skin of a subject, the device comprising: (1) a support structure for application to the skin of the subject, (2) transport means for delivery to and/or withdrawal of a fluid from the skin and/or beneath the skin of the subject, and (3) a signal structure for generating a signal indicative of the fluid delivered to and/or withdrawn from the skin and/or beneath the skin of the subject, wherein the self-contained device, when affixed to the skin of the subject, comprises at least one of:
      (1) a largest lateral dimension, when the device is positioned for extraction of the fluid, parallel to the extraction area, of no more than about 5 cm,
      (2) a largest vertical dimension, extending from the skin of the subject when the device is applied to the subject, of no more than about 1 cm, or
      (3) a mass of no more than about 25 g, absent the fluid; and
   activating the device.
159. A device for delivering to and/or withdrawing a fluid from the skin and/or beneath the skin of a subject, the device comprising:
   a support structure for application to the skin of the subject;
   transport means for delivery to and/or withdrawal of the fluid from the skin and/or beneath the skin of the subject; and
   a sensor for determining a condition of the fluid delivered to and/or withdrawn from the skin and/or beneath the skin of the subject,
   wherein the device is constructed and arranged to be wearable by the subject for at least about 15 minutes.
160. A device for withdrawing interstitial fluid from the skin and/or from beneath the skin of a subject, the device comprising:
   a support structure for application to the skin of the subject; and
   transport means for withdrawal of interstitial fluid from the skin and/or from beneath the skin of the subject,
   wherein the device is constructed and arranged to be wearable by the subject for at least about 15 minutes.
161. A device for withdrawing a fluid from the skin and/or beneath the skin of a subject, the device comprising:
   a support structure for application to the skin of the subject; and
   transport means for withdrawal of the fluid from one or more capillaries in the skin of the subject,
   wherein the device is constructed and arranged to be wearable by the subject for at least about 15 minutes.
162. A device for extraction of a fluid from the skin and/or from beneath the skin of a subject, the device comprising:
   a support structure for application to the skin of the subject; and
   means for extracting a fluid from the skin and/or from beneath the skin of the subject at an extraction area;
   wherein the device comprises at least one of:
      a largest lateral dimension, when the device is positioned for extraction of the fluid, parallel to the extraction area, of no more than about 6 cm,
      a largest vertical dimension, extending from the skin of the subject when the device is positioned for extraction of the fluid, of no more than about 1.5 cm, or
      a mass of no more than about 25 g, absent the fluid.
163. A self-contained device for delivering to and/or withdrawing a fluid from the skin and/or beneath the skin of a subject, the device comprising:
   a support structure for application to the skin of the subject; and
   transport means for delivery to and/or withdrawal of the fluid from the skin and/or beneath the skin of the subject,
   wherein the self-contained device, when affixed to the skin of the subject, comprises at least one of:
      (1) a largest lateral dimension, when the device is positioned for extraction of the fluid, parallel to the extraction area, of no more than about 6 cm,
      (2) a largest vertical dimension, extending from the skin of the subject when the device is applied to the subject, of no more than about 1.5 cm, or
      (3) a mass of no more than about 25 g, absent the fluid.
164. A device for extraction of a fluid from the skin and/or from beneath the skin of a subject, the device comprising:
   a support structure for application to the skin of the subject;
   means for extracting a fluid from the skin and/or from beneath the skin of the subject at an extraction area; and
   an adhesive for adhesion of the support structure to the skin of the subject.
165. A device for delivering to and/or withdrawing a fluid from the skin and/or beneath the skin of a subject, the device comprising:
   a support structure for application to the skin of the subject; and
   a fluid transporter associated with the support structure;
   wherein a portion of the device is constructed and arranged to be replaceable without disassembling the device, wherein the device cannot function to deliver and/or withdraw fluid without the replaceable portion being present within the device, and wherein the replaceable portion does not comprise a power source.
166. The device of paragraph 165, wherein the replaceable portion comprises a pressure controller.
167. The device of paragraph 165, wherein the replaceable portion comprises a vacuum chamber having a pressure less than atmospheric pressure.
168. The device of paragraph 165, wherein the replaceable portion comprises an actuator for the fluid transporter.
169. The device of paragraph 165, wherein the replaceable portion comprises an activator for the fluid transporter.
170. The device of paragraph 165, wherein the replaceable portion comprises the fluid transporter.
171. The device of paragraph 165, wherein the replaceable portion comprises a sensor.
172. The device of paragraph 165, wherein the replaceable portion comprises a collection chamber.
173. The device of paragraph 165, wherein the replaceable portion comprises a fluid assay.
174. The device of paragraph 165, wherein the replaceable portion comprises a processor.
175. The device of paragraph 165, wherein the replaceable portion comprises a memory component.
176. The device of paragraph 165, wherein the replaceable portion comprises a transmitter.
177. The device of paragraph 165, wherein the replaceable portion comprises a display.
178. A device for delivering to and/or withdrawing a fluid from the skin and/or beneath the skin of a subject, the device comprising:
   a support structure for application to the skin of the subject;
   a fluid transporter associated with the support structure; and
   a removable module constructed and arranged to be reversibly removable from the support structure, wherein the removable module comprises one or more of a pressure controller, an actuator for the fluid transporter, a sensor, a fluid assay, a collection chamber, a processor, a memory component, a transmitter, or a display.

It should be understood that various changes and modifications to the presently preferred embodiments described herein will be apparent to those skilled in the art. Such changes and modifications can be made without departing from the spirit and scope of the present invention and without diminishing its intended advantages. It is therefore intended that such changes and modifications be covered by the following claims.

## Claims

1. A device for extraction of a fluid, preferably blood or interstitial fluid, from the skin and/or from beneath the skin of a subject, the device comprising:
a support structure for application to the skin of the subject, wherein the support structure comprises a pre-packaged vacuum chamber having a pressure less than atmospheric pressure prior to the point at which the device is used, and a flexible concave member movable between a first configuration and a second configuration; and
a fluid transporter mechanically coupled to the flexible concave member;
wherein a portion of the device is constructed and arranged to be replaceable without disassembling the device, wherein the device cannot function to withdraw fluid without the replaceable portion being present within the device, and wherein the replaceable portion does not comprise a power source,
preferably wherein the replaceable portion comprises one or more of a pressure controller, an actuator for the fluid transporter, a sensor, a fluid assay, a collection chamber, a processor, a memory component, a transmitter, or a display.

2. The device of any preceding claim, wherein the fluid comprises blood.

3. The device of any preceding claim, wherein the replaceable portion comprises a collection chamber, preferably wherein the collection chamber is fluidly connectable to the fluid transporter, preferably by a channel, preferably wherein the channel is a microfluidic channel.

4. The device of any preceding claim, wherein the fluid transporter comprises a needle, preferably wherein the needle is a microneedle, preferably wherein the microneedle is solid or hollow; and/or wherein the fluid transporter is immobilized relative to the support structure; and/or the device further comprising:
a stabilizing agent or an anticoagulant for stabilizing the fluid withdrawn from the skin in fluid communication with the fluid transporter, preferably wherein the anticoagulant comprises heparin.

5. The device of any preceding claim, wherein the support structure is able to apply a pressure less than atmospheric pressure to the skin of the subject; and/or wherein the fluid transporter is able to move relative to the support structure, and/or wherein the fluid transporter is able to be activated by the subject, and/or wherein the fluid transporter is able to be activated upon applying the device to the skin of the subject.

6. The device of any preceding claim, wherein the fluid transporter is able to be activated using an actuator, preferably wherein the actuator comprises a button, and/or wherein the fluid transporter is able to be activated remotely, and/or wherein the device comprises a timing mechanism for timed activation after removal of the fluid transporter from packaging containing the device; and/or wherein the device is able to withdraw the fluid from the skin and/or from beneath the skin of the subject via the fluid transporter, preferably wherein the support structure is able to draw the skin of the subject towards the fluid transporter.

7. The device of any preceding claim, wherein the device has a largest lateral dimension, when the device is positioned for extraction of the fluid, parallel to the extraction area, of no more than 7 cm, preferably wherein the largest lateral dimension is between 2.5 cm and 5 cm, and/or wherein the device has a largest vertical dimension, extending from the skin of the subject when the device is positioned for extraction of the fluid, of no more than 3 cm.

8. The device of any preceding claim, wherein the support structure further comprises a mechanical element for affixing the device to the skin of the subject, and/or a recess containing the fluid transporter.

9. The device of any preceding claim, wherein the device further comprises a sensor for determining at least one condition of a fluid extracted from the skin of the subject via the fluid transporter, preferably wherein the device comprises a signal generator for generating a signal relating to the at least one condition of the fluid determined by the sensor, preferably wherein the signal generator comprises a display, preferably wherein the sensor is a glucose sensor or a test strip; and/or wherein the device further comprises an activator constructed and arranged to cause exposure of the fluid transporter to the skin of the subject upon activation of the activator, and/or a signal structure for generating a signal indicative of the fluid withdrawn from the skin and/or beneath the skin of the subject.

10. The device of any preceding claim, wherein the device further comprises a signal structure for generating a signal indicative of the extracted fluid; wherein the support structure and the signal structure are constructed and arranged to be connectable and/or detachable to each other.

11. The device of any preceding claim, wherein the device has a weight of less than 1 kg, preferably wherein the device has a weight of less than about 300 g.

12. The device of any preceding claim, wherein the device is constructed and arranged to be affixed on the arm or leg of a subject, preferably wherein the device is constructed and arranged to be affixed to a subject such that the subject is able to walk after the device has been affixed thereto.

13. The device of any preceding claim, wherein the device is able to determine an analyte contained within the fluid withdrawn from the skin and/or from beneath the skin of the subject, preferably wherein the device is able to determine the analyte without subsequent removal of the device from the skin of the subject, preferably wherein the device is able to automatically determine the analyte, preferably wherein the device is able to display a result of the determination of the analyte, preferably wherein the at least one analyte comprises glucose.

14. The device of any preceding claim, wherein the device is constructed and arranged to be wearable by the subject for at least 15 minutes, preferably at least 1 hour, preferably at least 1 day, preferably 1 week.

15. The device of any preceding claim, wherein the device further comprises an adhesive for adhesion of the support structure to the skin of the subject.
